(19) Europäisches Patentamt
European Patent Office
Office européen des brevets

(11) Publication number: **0 186 977 A2**

# (12) EUROPEAN PATENT APPLICATION

(21) Application number: **85308759.1**

(22) Date of filing: **02.12.85**

(51) Int. Cl.[4]: **C 07 C 103/76**, C 07 C 149/23, C 07 D 233/64, C 07 D 295/12, C 07 D 211/58, C 07 D 277/30, A 61 K 31/165, A 61 K 31/395

(30) Priority: **30.11.84 JP 252878/84**
**19.12.84 JP 268035/84**
**29.01.85 JP 15177/85**

(43) Date of publication of application: **09.07.86**
**Bulletin 86/28**

(84) Designated Contracting States: **AT BE CH DE FR GB IT LI NL SE**

(71) Applicant: **SANKYO COMPANY LIMITED,**
**No. 1-6, 3-chome Nihonbashi Honcho Chuo-ku, Tokyo (JP)**

(72) Inventor: **Morisawa, Yasuhiro Sankyo Chemical Research Lab., 2-58,-1-chome, Hiromachi, Shinagawa-ku, Tokyo 140 (JP)**
Inventor: **Yabe, Yuichiro Sankyo Chemical Research Lab., No. 2-58, 1-chome Hiromachi, Shinagawa-ku, Tokyo 140 (JP)**
Inventor: **Kataoka, Mitsuru Sankyo Chemical Research Lab., No. 2-58, 1-chome Hiromachi, Shinagawa-ku, Tokyo 140 (JP)**
Inventor: **Iijima, Yasuteru Sankyo Biological Research Lab., No. 2-58, 1-chome Hiromachi, Shinagawa-ku, Tokyo 140 (JP)**
Inventor: **Kokubu, Tatsuo, 2310-7, Tanokubo, Shigenobu-cho, Onsen-gun, Ehime-prefecture (JP)**
Inventor: **Hiwada, Kunio, 2108-6, Tanokubo, Shigenobu-cho, Onsen-gun, Ehime-prefecture (JP)**

(74) Representative: **Gibson, Christian John Robert et al, Marks & Clerk 57/60 Lincoln's Inn Fields, London WC2A 3LS (GB)**

## (54) Renin-inhibitory dipeptides, their preparation and use.

(57) Compounds of formula (I):

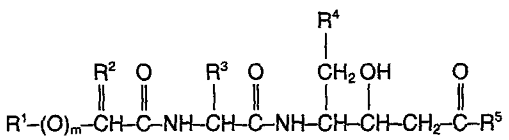

(I)

(wherein m is 0 or 1 and $R^1$–$R^5$ are a variety of organic groups) have the ability to inhibit the activity of renin and may thus be used to treat hypertension induced by failures in the renin-angiotensin system. They are prepared by conventional peptide synthesis routes.

EP 0 186 977 A2

ACTORUM AG

# RENIN-INHIBITORY DIPEPTIDES, THEIR PREPARATION AND USE

The present invention relates to a series of new dipeptides which have renin-inhibitory and, hence, hypotensive activities and thus are of particular value in the treatment of hypertension induced by failures in the renin-angiotensin system. The invention also relates to the preparation of such compounds and to compositions for use in such treatment.

There is considerable evidence that reduction of elevated blood pressure reduces the risks of morbidity and mortality. Elevated blood pressure (hypertension) can be caused by a variety of factors and a large number of drugs is available for the treatment of hypertension, the drug of choice being dictated in large measure by the cause of the hypertension. Angiotensin I is a polypeptide formed by the action of renin upon a plasma protein and is converted to angiotensin II by the action of ACE (angiotensin converting enzyme). Angiotensin II causes constriction of the arterioles and can produce hypertension. Hypertension of this type can be reduced by reducing the plasma concentration of angiotensin

which, in turn, can be achieved by inhibiting the activity of renin. The number of available drugs having this type of inhibitory activity is very limited.

Certain peptide derivatives having this type of activity are disclosed in Japanese Patent Application Kokai (i.e. as laid open to public inspection) No. 151166/77 and may be represented by the formula $R^aCO-X-His-NH-CH(CH_2R^b)-CHO$, in which $R^a$ and $R^b$ represent various organic groups and His represents the L-histidyl group.

Other polypeptides which have been proposed for use as renin inhibitors are the angiotensinogen fragments described by Szelke et al. [Nature, 299, 555 (1982)] and the statine derivatives described by Boger et al. [Nature, 303, 81 (1983) and European Patent Publication No. 114,993].

Certain hypotensive peptides closely related to the compounds of the present invention are disclosed in US Patent No. 4,548,926 and in EP-A 0152255.

We have now discovered a series of peptide derivatives having a very marked ability to inhibit the activity of renin, which ability is believed to be significantly better than that of the prior art compounds.

The compounds of the invention are peptides having the general formula (I):

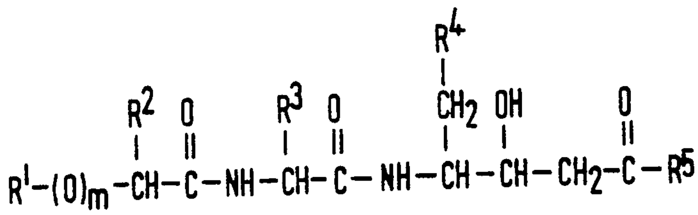

(I)

wherein:

<u>m</u> is the cypher 0 or the integer 1;

$R^1$ and $R^2$ are the same or different and each represents a $C_1$-$C_{10}$ alkyl group, a group of formula $-(A)_p-R^6$, wherein:

<u>p</u> is 0 or 1;

A represents a $C_1$-$C_4$ alkylene group or a $C_2$-$C_4$ alkenylene group; and

$R^6$ represents an aryl group or a heterocyclic group;

or a group of formula $-B-R^7$, wherein:

B represents a $C_1$-$C_4$ alkylene group or an alkoxyalkyl group where the alkoxy and alkyl parts are both $C_1$-$C_3$; and

$R^7$ represents a $C_1$-$C_4$ alkoxy group, an aryloxy group, an aralkyloxy group where the alkyl part is $C_1$-$C_4$ or a heterocyclic group;

$R^3$ represents a $C_1$-$C_{10}$ alkyl group, a substituted $C_1$-$C_{10}$ alkyl group having at least one substituent preferably selected from substituents (a):

(a) hydroxy groups, $C_1$-$C_4$ alkoxy groups, aryloxy groups, aralkyloxy groups where the alkyl part is $C_1$-$C_4$, $C_1$-$C_7$ aliphatic carboxylic acyloxy groups, $C_1$-$C_4$ alkylthio groups, arylthio groups, aralkylthio groups where the alkyl part is $C_1$-$C_4$, $C_1$-$C_4$ alkylsulphinyl groups, $C_1$-$C_4$ alkylsulphonyl groups, arylsulphinyl groups, arylsulphonyl groups, $C_1$-$C_7$ aliphatic carboxylic acylamino groups, aromatic carboxylic acylamino groups, $C_2$-$C_7$ alkoxycarbonylamino groups, aralkyloxycarbonylamino groups where the

alkyl part is $C_1$-$C_4$, $C_2$-$C_7$ alkoxycarbonyl groups, aryloxycarbonyl groups, aralkyloxycarbonyl groups where the alkyl part is $C_1$-$C_4$, carbamoyl groups, alkylcarbamoyl groups where the alkyl part is $C_1$-$C_4$, dialkylcarbamoyl groups where each alkyl part is $C_1$-$C_4$, thiocarbamoyl groups, alkyl(thiocarbamoyl) groups where the alkyl part is $C_1$-$C_4$, dialkyl(thiocarbamoyl) groups where each alkyl part is $C_1$-$C_4$, ureido groups, alkylureido groups where the alkyl part is $C_1$-$C_4$, dialkylureido groups where each alkyl part is $C_1$-$C_4$, thioureido groups, alkyl(thioureido) groups where the alkyl part is $C_1$-$C_4$, dialkyl(thioureido) groups where each alkyl part is $C_1$-$C_4$, $C_3$-$C_8$ cycloalkyl groups, $C_5$-$C_8$ cycloalkenyl groups, aryl groups, heterocyclic groups, halogen atoms, mono-, di- and tri-halomethyl groups, mercapto groups, amino groups, $C_1$-$C_4$ alkylamino groups, dialkylamino groups where each alkyl part is $C_1$-$C_4$, carboxy groups and guanidino groups;

a $C_2$-$C_5$ alkenyl group, a $C_2$-$C_5$ alkenyl group having at least one halogen substituent, a $C_2$-$C_5$ alkynyl group, a hydrogen atom or a $C_3$-$C_8$ cycloalkyl group;

$R^4$ represents an isopropyl group, a $C_3$-$C_8$ cycloalkyl group or a phenyl group;

$R^5$ represents a hydroxy group, a $C_1$-$C_{10}$ alkoxy group, an aryloxy group, a group of formula $-NR^8R^9$ wherein

$R^8$ and $R^9$ are the same or different and each represents a hydrogen atom, a $C_1$-$C_{10}$ alkyl group, a substituted $C_1$-$C_{10}$ alkyl group having at least one substituent preferably selected from substituents (b):

(b) hydroxy groups, $C_1$-$C_4$ alkoxy groups, halogen atoms, aryl groups, $C_3$-$C_8$ cycloalkyl groups, $C_1$-$C_4$ alkylamino groups, dialkylamino groups where each alkyl part is $C_1$-$C_4$, ($C_1$-$C_4$ hydroxyalkyl)amino groups, di($C_1$-$C_4$ hydroxyalkyl)amino groups and heterocyclic groups;

a $C_3$ or $C_4$ alkenyl group, a $C_3$-$C_8$ cycloalkyl group, an aryl group or a heterocyclic group;

or a heterocyclic group;

said aryl groups and the aryl parts of said aryloxy, aralkyloxy, arylthio, aralkylthio, arylsulphinyl, arylsulphonyl, aromatic carboxylic acylamino, aralkyloxycarbonylamino, aryloxycarbonyl and aralkyloxycarbonyl groups are $C_6$-$C_{14}$ carbocyclic aryl groups which are unsubstituted or have at least one substituent preferably selected from substituents (a), (b) and (c):

(c) $C_1$-$C_4$ alkyl groups, nitro groups, cyano groups, $C_3$-$C_5$ alkenoyl groups, substituted $C_3$-$C_5$ alkenoyl groups having at least one substituent preferably selected from substituents (a) and substituted $C_1$-$C_4$ alkyl groups having at least one substituent preferably selected from substituents (b); and

said heterocyclic groups have from 5 to 14 ring atoms, of which from 1 to 4 are nitrogen, oxygen or sulphur hetero-atoms, and are unsubstituted or have at least one substituent preferably selected from said substituents (a), (b) and (c);

and pharmaceutically acceptable salts, e.g. acid addition salts, thereof.

These compounds may be used for the treatment or prophylaxis of angiotensin-induced hypertension in a mammal, which may be human or non-human, by the administration thereto of the compound or a pharmaceutically acceptable salt thereof.

Thus, the invention also provides a composition for the treatment of angiotensin-induced hypertension in a mammal, which may be human or non-human, which comprises a compound of formula (I) or a pharmaceutically acceptable salt thereof in admixture with a pharmaceutically acceptable carrier or diluent.

The compounds of the invention may be prepared by:

(i) reacting a compound of formula (II):

$$R^1-(O)_m-CH(R^2)-CO-(Y)_r-OH \quad (II)$$

[in which: $R^1$, _m_ and $R^2$ are as defined above; Y represents a group of formula $-NH-CH(R^3)CO-$, where $R^3$ is as defined above; and _r_ is 0 or 1] or a reactive derivative thereof with a compound of formula (III):

$$H(Y)_sNH-CH(CH_2R^4)-CH(OH)-CH_2COR^5 \quad (III)$$

(in which: Y, $R^4$ and $R^5$ are as defined above; and _s_ is 1 if _r_ is 0 or 0 if _r_ is 1) or a reactive derivative thereof;

(ii) optionally subjecting the product to an acyl exchange reaction; and

(iii) optionally converting any group represented by $R^5$ to any other group represented by $R^5$.

Where $R^1$ or $R^2$ represents a $C_1$-$C_{10}$ alkyl group, this may be a straight or branched chain group and examples include the methyl, ethyl, propyl, isopropyl, butyl, isobutyl, sec-butyl, t-butyl, pentyl, isopentyl, neopentyl, t-pentyl, 1-methylbutyl, 2-methylbutyl, hexyl, isohexyl, sec-hexyl, 1,3-dimethylbutyl, 3,3-dimethylbutyl, 2-methylpentyl, heptyl, octyl, 1,5-dimethylhexyl, 2-ethylhexyl, nonyl and decyl groups, of which the $C_2$-$C_8$ alkyl groups are preferred, particularly, the ethyl, isopropyl, isobutyl, isopentyl and octyl groups.

Where $R^1$ or $R^2$ represents a group of formula $-(A)_p-R^6$, _p_ may be 0 or 1 and, where _p_ is 1, A may represent a $C_1$-$C_4$ alkylene group or a $C_2$-$C_4$

alkenylene group, either of which may be a straight or branched chain group. These groups are both bivalent hydrocarbon groups and, in each case, the two "free" valencies may be on different carbon atoms or on the same carbon atom. An alkylene group in which the two "free" valencies are on the same carbon atom is sometimes referred to as an "alkylidene" group. Examples of such groups include the methylene, ethylene, methylmethylene, 2-ethylethylene, 3-methyltrimethylene, 2-methyltrimethylene, propenylene (which may be 1- or 2-propenylene) and butenylene (which may be 1-, 2- or 3-, preferably 2- or 3-butenylene) groups.

Where $R^6$ or substituent (a) represents an aryl group or where $R^7$, substituent (a) or $R^5$ represents an aryloxy group or where substituent (a) represents an arylthio group, the aryl group is a carbocyclic aryl group having from 6 to 14, preferably from 6 to 10, ring carbon atoms and which may be unsubstituted or have at least one of the substituents (a), (b) and (c) defined above. Examples of the unsubstituted aryl groups are the phenyl, indenyl (which may be 1-, 2-, 3-, 4-, 5-, 6- or 7-indenyl) and naphthyl (1- or 2-naphthyl) groups. Where such aryl groups are substituted, they may be any of the groups, e.g. phenyl, indenyl and naphthyl groups, exemplified as unsubstituted aryl groups, and have at least one of the substituents (a), (b) and (c) as

exemplified herein. Preferred substituents for aryl groups are: $C_1$-$C_4$ alkyl groups, e.g. the methyl, ethyl, propyl, isopropyl, butyl, isobutyl, sec-butyl and t-butyl groups; halogen atoms, e.g. the fluorine, chlorine, bromine and iodine atoms; $C_1$-$C_4$ alkoxy groups, e.g. the methoxy, ethoxy, propoxy, isopropoxy, butoxy, isobutoxy, sec-butoxy and t-butoxy groups; $C_2$-$C_5$ alkoxycarbonyl groups, i.e. groups containing a $C_1$-$C_4$ alkoxy group, such as those alkoxy groups exemplified above; the trifluoromethyl group; the amino group; the cyano group; or the nitro group. In general, there is no particular limitation on the maximum number of substituents possible except that dictated by the number of substitutable positions (e.g. 5 for phenyl, 7 for indenyl, 7 for naphthyl); however, in particular circumstances (as described more fully below in relation to substituents generally) steric constraints may dictate that less than this maximum number of substituents may be present. In practice, most substituted aryl groups employed in compounds of this type will normally have from 1 to 5, more commonly 1 to 3, substituents.

$R^5$, $R^6$, $R^7$, substituent (a) and substituent (b) may represent heterocyclic groups. Such groups may be aromatic or non-aromatic in character. $R^6$ and substituent (a) preferably represent aromatic

heterocyclic groups, whilst $R^5$ and $R^7$ preferably represent non-aromatic heterocyclic groups. Substituent (b) is preferably either.

Where any of these groups, especially $R^6$ or substituent (a), represents an aromatic heterocyclic group, this is a heterocyclic group (as defined above) having aromatic character, i.e. a group having from 5 to 14 ring atoms of which from 1 to 5 are nitrogen, oxygen or sulphur hetero-atoms, and it may be monocyclic or polycyclic (e.g. bicyclic). More preferably, it has from 5 to 10 ring atoms, of which from 1 to 3 are such hetero-atoms. Included amongst the preferred aromatic heterocyclic groups are bicyclic groups comprising a benzene ring fused to a heterocyclic ring (the heterocyclic ring preferably having 5, 6 or 7 ring atoms of which from 1 to 3 are said hetero-atoms). Examples of such aromatic heterocyclic groups include the furyl, thienyl, oxazolyl, thiazolyl, imidazolyl, pyridyl, benzofuryl, benzothienyl, indolyl, benzothiazolyl, benzimidazolyl, quinolyl and isoquinolyl groups. Such groups may be unsubstituted or may have at least one of the substituents (a), (b) and (c) defined above. Preferred such substituents are those specified above in relation to aryl groups, particularly the alkyl, halogen and alkoxy substituents. The maximum number of such substituents is generally also as described in relation

to the aryl groups.

Where $R^7$ represents a heterocyclic group, this has from 5 to 14 ring atoms, of which from 1 to 5, more preferably from 1 to 3 and most preferably 1 or 2, are nitrogen, oxygen or sulphur hetero-atoms. The most preferred heterocyclic groups contain 5 or 6 ring atoms and most preferably 1 or 2 of these atoms are nitrogen atoms and 0 or 1 of these atoms are oxygen atoms and/or sulphur atoms. The heterocyclic group represented by $R^7$ may be aromatic in character (in which case it may be any one of those groups exemplified above) or, and more preferably, it may be non-aromatic, in which case the ring may be a fully saturated ring or it may be an unsaturated ring, provided that the unsaturation is not aromatic in character.

Specific examples of preferred non-aromatic heterocyclic groups which may be represented by $R^5$, $R^6$, $R^7$, substituent (a) and substituent (b) include the piperidyl (including the 1-piperidyl group known as "piperidino"), pyrrolidinyl (e.g. 1-pyrrolidinyl), morpholinyl (including the morpholino group), oxazolidinyl (including 1-oxazolidinyl), thiazolidinyl (including 1-thiazolidinyl), imidazolidinyl (including

1-imidazolidinyl) and piperazinyl (including 1-piperazinyl). Such a ring may be unsubstituted or may have at least one of the substituents (a), (b) and (c) defined above. In the case of the heterocyclic groups represented by $R^7$, the preferred substituents are: $C_1$-$C_4$ alkyl groups, such as those exemplified above in relation to substituents on aryl groups; $C_1$-$C_4$ hydroxyalkyl groups, such as the hydroxymethyl, 1-hydroxyethyl, 2-hydroxyethyl, 2-hydroxypropyl, 3-hydroxypropyl and 4-hydroxybutyl groups; $C_1$-$C_4$ alkoxy groups, such as those exemplified below in relation to $R^7$; phenyl groups, which may be unsubstituted or may themselves have one or more of substituents (a), (b) and (c), but most preferably the substituents exemplified above in relation to aryl groups; aromatic heterocyclic groups, such as those exemplified above in relation to $R^6$ and which, of course, may be unsubstituted or substituted as defined above; aralkyloxycarbonyl groups, in which the aralkyloxy part may be any one of those aralkyloxy groups exemplified below in relation to $R^7$; $C_2$-$C_5$ alkoxycarbonyl groups, in which the alkoxy part may be any one of those exemplified below in relation to $R^7$; and arylalkenoyl groups, in which the aryl part may be any one of those exemplified above and the alkenoyl part is a $C_3$-$C_5$ alkenoyl group, preferably a propenoyl

group, for example the cinnamoyl group, which may itself be substituted. The maximum number of substituents is as described generally below and as described specifically above in relation to aryl groups.

Where $R^1$ or $R^2$ represents a group of formula $-B-R^7$, B represents a $C_1$-$C_4$ alkylene group or an alkoxyalkyl group in which both the alkoxy and the alkyl parts have from 1 to 3, preferably 1 or 2, carbon atoms. Examples of alkylene groups which may be represented by B are as given above in relation to the similar groups which may be represented by A. Examples of alkoxyalkyl groups include those groups where the alkoxy and the alkyl parts are chosen from the alkoxy and the alkyl groups hereinbefore exemplified and having from 1 to 3 carbon atoms and preferably such groups which are straight chain. Most preferably, the alkoxyalkyl group has a total of from 2 to 4 carbon atoms and specific examples of such groups include the methoxymethyl, ethoxymethyl, 2-methoxyethyl and 2-ethoxyethyl groups.

Where $R^7$ represents a $C_1$-$C_4$ alkoxy group, this may be a straight or branched chain group, for example a methoxy, ethoxy, propoxy, isopropoxy, butoxy, isobutoxy, sec-butoxy or t-butoxy group. Where $R^7$ represents an aryloxy group, the aryl part may be as exemplified above.

Where $R^7$ represents an aralkyloxy group, the aryl group is as defined above and may be any one of those aryl groups exemplified above in relation to $R^6$, but is preferably a phenyl or naphthyl (1- or 2-naphthyl) group, more preferably a phenyl group. The alkyl part of this group is a $C_1$-$C_4$, preferably $C_1$-$C_3$, group, which may be a straight or branched chain group, e.g. the methyl, ethyl, propyl or isopropyl groups. The aralkyloxy group is most preferably a benzyloxy or phenethyloxy group, and these groups may be unsubstituted or may have one or more of the above-defined substituents (a), (b) and (c). Most preferably, the substituents are as exemplified above in relation to aryl groups.

Where $R^3$ represents a $C_1$-$C_{10}$ alkyl group, it may be any one of those groups defined above in relation to $R^1$ and $R^2$ and may be unsubstituted or may have at least one of the substituents defined above as substituents (a).

Examples of such substituents include:

(i) the hydroxy group;

(ii) $C_1$-$C_4$ alkoxy groups, such as those defined above in relation to $R^7$;

(iii) aryloxy groups, where the aryl part may be any one of those aryl groups described above, and may be unsubstituted or substituted.

(iv) aralkyloxy groups, such as those defined above in relation to $R^7$;

(v) aliphatic carboxylic acyloxy groups having from 1 to 7 carbon atoms, for example the formyloxy, acetoxy, propionyloxy, butyryloxy, isobutyryloxy, valeryloxy, isovaleryloxy, pivaloyloxy, hexanoyloxy and heptanoyloxy groups;

(vi) $C_1$-$C_4$ alkylthio groups, in which the alkyl part is a $C_1$-$C_4$ alkyl group chosen from any one of the $C_1$-$C_4$ groups exemplified above in relation to $R^1$ and $R^2$;

(vii) arylthio groups, in which the aryl part may be any one of those aryl groups described above, and may be unsubstituted or substituted.

(viii) aralkylthio groups, in which the aralkyl part may be any one of those exemplified above in relation to the aralkyl part of aralkyloxy groups;

(ix) $C_1$-$C_4$ alkylsulphinyl and alkylsulphonyl groups,

in which the alkyl parts may be any one of those $C_1$-$C_4$ alkyl groups exemplified above in relation to $R^1$ and $R^2$;

(x) arylsulphinyl and arylsulphonyl groups, in which the aryl part may be any of those aryl groups defined above in relation to $R^6$;

(xi) $C_1$-$C_7$ aliphatic acylamino groups, such as the formamido, acetamido, propionamido, butyramido, isobutyramido, valeramido, isovaleramido, pivaloylamino, hexanoylamino and heptanoylamino groups;

(xii) aromatic carboxylic acylamino groups, in which the aromatic part may be any one of those aryl groups exemplified above but is preferably a phenyl group which may be unsubstituted or have one or more substituents, for example the benzoylamino or p-nitrobenzoylamino groups;

(xiii) alkoxycarbonylamino groups having a total of from 2 to 5 carbon atoms, in which the alkoxycarbonyl part may be any one of those alkoxycarbonyl groups exemplified above in relation to substituents on aryl groups;

(xiv) aralkyloxycarbonylamino groups, in which the

aralkyloxy part may be any one of those aralkyloxy groups exemplified above in relation to $R^7$;

(xv) $C_2$-$C_5$ alkoxycarbonyl groups, which may be chosen from those exemplified above in relation to substituents on aryl groups;

(xvi) aryloxycarbonyl groups, in which the aryl part may be any one of those aryl groups exemplified above in relation to $R^6$;

(xvii) aralkyloxycarbonyl groups, in which the aralkyloxy part may be any one of those aralkyloxy groups exemplified above in relation to $R^7$;

(xviii) carbamoyl and thiocarbamoyl groups;

(xix) mono- and di-alkylcarbamoyl and mono- and di-alkyl(thiocarbamoyl) groups, in which the or each alkyl part is a $C_1$-$C_4$ alkyl group and may be chosen from those $C_1$-$C_4$ alkyl groups exemplified above in relation to $R^1$ and $R^2$;

(xx) ureido and thioureido groups;

(xxi) mono- and di-alkylureido and mono- and di-alkyl(thioureido) groups, in which the or each alkyl

part is a $C_1$-$C_4$ alkyl group, which may be chosen from any one of those $C_1$-$C_4$ alkyl groups exemplified above in relation to $R^1$ and $R^2$;

(xxii) $C_3$-$C_8$ cycloalkyl groups, for example the cyclopropyl, cyclobutyl, cyclopentyl, cyclohexyl, cycloheptyl or cyclooctyl groups;

(xxiii) $C_5$-$C_8$ cycloalkenyl groups, for example the 1-cyclopentenyl, 2-cyclopentenyl, 3-cyclopentenyl, 1-cyclohexenyl, 2-cyclohexenyl, 3-cyclohexenyl, 1-cycloheptenyl, 2-cycloheptenyl, 3-cycloheptenyl, 4-cycloheptenyl, 1-cyclooctenyl, 2-cyclooctenyl, 3-cyclooctenyl or 4-cyclooctenyl groups;

(xxiv) aryl groups, which may be substituted or unsubstituted, for example any one of those aryl groups defined above in relation to $R^6$, preferably a phenyl group or a phenyl group having at least one substituent selected from the group consisting of hydroxy groups, halogen atoms, $C_1$-$C_4$ alkyl groups and $C_1$-$C_4$ alkoxy groups;

(xxv) heterocyclic groups, preferably aromatic heterocyclic groups, which may be unsubstituted or substituted, for example any one of those aromatic heterocyclic groups defined above in relation to $R^6$,

preferably a furyl, thienyl, oxazolyl, thiazolyl, imidazolyl or pyridyl group, or, less preferably, any one of the non-aromatic groups described in relation to $R^7$, any of which may be unsubstituted or may have at least one of the previously defined substituents;

(xxvi) halogen atoms, for example the chlorine, fluorine, bromine or iodine atoms;

(xxvii) halomethyl groups, for example the chloromethyl, bromomethyl, iodomethyl, fluoromethyl, dichloromethyl, dibromomethyl, difluoromethyl or trifluoromethyl groups;

(xxviii) mercapto groups;

(xxix) amino groups;

(xxx) mono- and di- alkylamino groups in which the or each alkyl part is a $C_1$-$C_4$ alkyl group, for example the methylamino, ethylamino, propylamino, isopropylamino, butylamino, isobutylamino, sec-butylamino, dimethylamino, methyl(ethyl)amino, diethylamino, dipropylamino, diisopropylamino, dibutylamino, methyl(butyl)amino, ethyl(butyl)amino and diisobutylamino groups;

(xxxi) carboxy groups; and

(xxxii) guanidino groups.

Where $R^3$ represents a $C_2$-$C_5$ alkenyl group or a $C_2$-$C_5$ alkynyl group, these may be straight or branched chain groups and, in the case of the alkenyl groups, they may be unsubstituted or may have at least one halogen subtituent. Examples of such groups include the vinyl, propenyl (1-propenyl or allyl), isopropenyl, butenyl (1-, 2- or 3- butenyl), pentenyl (1-, 2-, 3- or 4- pentenyl), 3-methyl-2-butenyl, 2-methyl-3-butenyl, 1-chlorovinyl, 2-chlorovinyl, 2,2-dichlorovinyl, 2-bromovinyl, 2-chloroallyl, 2,3-dichloroallyl, 3-chloro-1-propenyl, 4-bromo-2-butenyl, ethynyl, propynyl (2-propynyl and 3-propynyl), butynyl (1-, 2- and 3- butynyl) and pentynyl (1-, 2-, 3- and 4- pentynyl) groups.

Where $R^3$ represents a $C_3$-$C_8$ cycloalkyl group, this may be any one of those described above in relation to substituents (a)-(xxii).

$R^4$ may represent an isopropyl group, a phenyl group or a $C_3$-$C_8$ cycloalkyl group. Where $R^4$ represents a $C_3$-$C_8$ cycloalkyl group, this may be a cyclopropyl, cyclobutyl, cyclopentyl, cyclohexyl,

cycloheptyl or cyclooctyl group.

Where $R^5$ represents a $C_1$-$C_{10}$ alkoxy group, this may be a straight or branched chain group and examples include the methoxy, ethoxy, propoxy, isopropoxy, butoxy, isobutoxy, sec-butoxy, t-butoxy, pentyloxy, isopentyloxy, neopentyloxy, t-pentyloxy, 1-methylbutoxy, 2-methylbutoxy, hexyloxy, isohexyloxy, sec-hexyloxy, 1,3-dimethylbutoxy, 3,3-dimethylbutoxy, 2-methylpentyloxy, heptyloxy, octyloxy, 1,5-dimethylhexyloxy, 2-ethylhexyloxy, nonyloxy and decyloxy groups, of which the $C_1$-$C_4$ groups, and particularly the methoxy and ethoxy groups, are preferred.

Where $R^5$ represents a heterocyclic group, this is preferably a non-aromatic heterocyclic group and may be any one of those exemplified above in relation to $R^7$. We particularly prefer those heterocyclic groups having a hydroxyalkyl, arylalkenoyl, aralkyl or aryl substituent. Most preferred are those heterocyclic groups containing at least one ring nitrogen atom and attached to the remainder of the molecule through said ring nitrogen atom.

Where $R^5$ represents a group of formula $-NR^8R^9$, $R^8$ and $R^9$ are the same or different and each

represents a hydrogen atom, a $C_1$-$C_{10}$ alkyl group, a substituted $C_1$-$C_{10}$ alkyl group having at least one (and preferably from 1 to 3) substituents preferably selected from substituents (b), a $C_3$ or $C_4$ alkenyl group, a $C_3$-$C_8$ cycloalkyl group, an aryl group or a heterocyclic group.

Where $R^8$ and $R^9$ both represent hydrogen atoms, then $R^5$ represents an amino group.

Where $R^8$ and/or $R^9$ represents a $C_1$-$C_{10}$ alkyl group or a substituted $C_1$-$C_{10}$ alkyl group, the alkyl group may be chosen from any of those exemplified above in relation to $R^1$ and $R^2$ and the substituents are chosen from substituents (b):

(i) hydroxy groups;

(ii) $C_1$-$C_4$ alkoxy groups, e.g. those exemplified above in relation to $R^7$;

(iii) halogen atoms, e.g. chlorine, bromine, iodine or fluorine;

(iv) aryl groups, e.g. any of those exemplified above in relation to $R^6$, but preferably phenyl groups, which may be unsubstituted or have at least one substituent,

e.g. any of those substituents exemplified above as substituents on aryl groups;

(v) aromatic heterocyclic groups, e.g. the pyridyl group, which may be unsubstituted or may have at least one substituent selected from the group consisting of substituents (a), (b) and (c);

(vi) $C_3-C_8$ cycloalkyl groups, e.g. those exemplified above in relation to $R^4$;

(vii) mono- and di-alkylamino groups, preferably dialkylamino groups, in which each alkyl part is $C_1-C_4$, e.g. those containing $C_1-C_4$ alkyl groups and exemplified above in relation to substituents (a)-(xxx);

(viii) mono- and di-($C_1-C_4$ hydroxyalkyl)amino groups, preferably the di($C_1-C_4$ hydroxyalkyl)amino groups, e.g. di(hydroxymethyl)amino, di(2-hydroxyethyl)amino, di(3-hydroxypropyl)amino and di(4-hydroxybutyl)amino groups; and

(ix) non-aromatic heterocyclic groups, e.g. those exemplified above in relation to $R^7$.

Where one or both of $R^8$ and $R^9$ represents a $C_3$

or $C_4$ alkenyl group, this may be an allyl, methallyl, 1-propenyl, 1-butenyl, 2-butenyl or 3-butenyl group.

Where $R^8$ or $R^9$ represents a $C_3$-$C_8$ cycloalkyl group, an aryl group or a heterocyclic group, these may be as exemplified above in relation to $R^4$, $R^6$ and $R^7$, respectively.

Preferably, both of $R^8$ and $R^9$ represent hydrogen atoms, alkyl groups, substituted alkyl groups or alkenyl groups or $R^8$ represents a hydrogen atom and $R^9$ represents an alkyl group, a substituted alkyl group, an alkenyl group, a cycloalkyl group, an aryl group or a heterocyclic group.

Examples of $C_1$-$C_4$ alkyl groups and substituted alkyl groups which may be represented by substituents (c) are included amongst the similar groups which may be represented by $R^3$. Examples of $C_3$-$C_5$ alkenoyl groups which may be represented by substituents (c) are the propenoyl, butenoyl and pentenoyl groups. These may be substituted or unsubstituted and, if substituted, the substituents may be chosen from any of those illustrated above as substituents (b). However, the preferred substituent is the aryl group, especially the phenyl group, which itself may be substituted or unsubstituted. If substituted, the substituents may be

selected from substituents (a), (b) and (c) defined above, but are preferably as described above as substituents on aryl groups. The most preferred substituted alkenoyl groups are the cinnamoyl and mono-, di- and tri-methoxycinnamoyl groups, e.g. the 3,4,5-trimethoxycinnamoyl group.

In general, where reference is made herein to "substituted" groups, there is no specific maximum limit to the number of possible substituents, although, in specific cases, steric constraints may impose a practical maximum limit. In principle, the maximum number of substituents is determined by the number of substitutable atoms and, where the substituent is relatively small, complete substitution may be possible. For example, where the substituent is relatively small such as a halogen (e.g. fluorine or chlorine) atom, the substituted group may range from the monohalo to perhalo groups, e.g. from monohaloalkyl to perhaloalkyl. Where the substituent is "bulkier", e.g. a t-butyl group, steric effects may prevent substitution of all substitutable positions. However, these effects are well-known to those skilled in the art and require no further discussion here.

A preferred class of compounds of the present invention are those compounds in which:

m is 0;

$R^1$ and $R^2$ are the same or different and each represents a $C_1$-$C_{10}$ alkyl group, a group of formula $-(A)_p-R^6$, wherein:

p is 0 or 1;

A represents a $C_1$-$C_4$ alkylene group or a $C_2$-$C_4$ alkenylene group; and

$R^6$ represents an aryl group or an aromatic heterocyclic group;

or a group of formula $-B-R^7$, wherein:

B represents a $C_1$-$C_4$ alkylene group or an alkoxyalkyl group where the alkoxy and alkyl parts are both $C_1$ or $C_2$; and

$R^7$ represents a $C_1$-$C_4$ alkoxy group or an aralkyloxy group where the alkyl part is $C_1$-$C_4$;

$R^3$ represents a $C_1$-$C_{10}$ alkyl group, a substituted $C_1$-$C_{10}$ alkyl group having at least one of the substituents (a'):

(a') hydroxy groups, $C_1$-$C_4$ alkoxy groups, $C_1$-$C_4$ alkylthio groups, $C_1$-$C_7$ aliphatic carboxylic acylamino groups, $C_2$-$C_5$ alkoxycarbonylamino groups, benzyloxycarbonylamino groups, $C_2$-$C_5$ alkoxycarbonyl groups, carbamoyl groups, $C_3$-$C_8$ cycloalkyl groups, phenyl groups, phenyl groups having at least one of the substituents (a'), (b') and (c), aromatic heterocyclic groups and carboxy groups;

a $C_3$ or $C_4$ alkenyl group or a propargyl group;

$R^4$ represents an isopropyl group, a $C_5$ or $C_6$ cycloalkyl group or a phenyl group;

$R^5$ represents a hydroxy group, a group of formula $-NR^8R^9$ wherein

$R^8$ and $R^9$ are the same or different and each represents a hydrogen atom, a $C_1$-$C_{10}$ alkyl group, a substituted $C_1$-$C_{10}$ alkyl group having at least one of the substituents (b'):

(b') hydroxy groups, $C_1$-$C_4$ alkoxy groups, pyridyl groups, dialkylamino groups where each alkyl part is $C_1$-$C_4$, di($C_1$-$C_4$ hydroxyalkyl)amino groups and non-aromatic heterocyclic groups;

a $C_3$ or $C_4$ alkenyl group or a heterocyclic group;

or a heterocyclic group;

said aryl groups and the aryl parts of said aralkyloxy groups are $C_6$-$C_{10}$ carbocyclic aryl groups which are unsubstituted or have at least one substituent selected from said substituents (a), (b) and (c) defined above; and

said heterocyclic groups have from 5 to 10 ring atoms of which from 1 to 3 are nitrogen, oxygen or sulphur hetero-atoms and are unsubstituted or have at least one substituent selected from said substituents (a), (b) and (c).

A more preferred class of compounds of the present invention are those compounds of formula (I) in which:

<u>m</u> is 0;

$R^1$ and $R^2$ are the same or different and each represents a group of formula $-A-R^6$ in which:

A represents a $C_1$-$C_4$ alkylene group; and
$R^6$ represents an aryl group or an aromatic heterocyclic group;

or of formula $-B-R^7$ in which:

B represents a $C_1-C_4$ alkylene group or an alkoxyalkyl group in which each of the alkoxy part and the alkyl part is $C_1$ or $C_2$; and

$R^7$ represents a $C_1-C_4$ alkoxy group or a benzyloxy group;

$R^3$ represents a $C_1-C_{10}$ alkyl group, a substituted $C_1-C_{10}$ alkyl group having at least one of the substituents (a"):

(a") hydroxy groups, $C_1-C_4$ alkylthio groups, carbamoyl groups, phenyl groups, aromatic heterocyclic groups, $C_2-C_5$ alkoxycarbonylamino groups, carboxy groups and cyclopentyl groups;

a $C_3$ or $C_4$ alkenyl group or a propargyl group;

$R^4$ represents an isopropyl group, a $C_5$ or $C_6$ cycloalkyl group or a phenyl group; and

$R^5$ represents a group of formula $-NR^{8'}R^{9'}$, in which $R^{8'}$ and $R^{9'}$ are the same or different and each represents a hydrogen atom, a 1-benzyl-4-piperidyl group, a $C_1-C_{10}$ alkyl group or a substituted $C_1-C_{10}$ alkyl group having at least one of the substituents (b"):

(b") hydroxy groups, $C_1$-$C_4$ alkoxy groups, di($C_1$-$C_4$ hydroxyalkyl)amino groups and non-aromatic heterocyclic groups.

Specific preferred examples of groups which may be represented by $R^1$ and/or $R^2$ include the phenyl, benzyl, chlorobenzyl, methylbenzyl, methoxybenzyl, 3,4-dimethoxybenzyl, phenethyl, methoxyphenethyl, 3-phenylpropyl, 4-phenylbutyl, 1-phenylbutyl, 3-phenylbutyl, 2-phenylbutyl, 3-phenylallyl, 3-(3,4,5-trimethoxyphenyl)allyl, 3-methyl-2-indenylmethyl, 1-naphthyl, 1-naphthylmethyl, 2-naphthylmethyl, furfuryl, 5-methylfurfuryl, 2-methyl-3-furylmethyl, pyridyl (preferably 4-pyridyl), 2-thenyl, 5-methyl-2-thenyl, 3-methyl-2-thenyl, 2-(3-thienyl)ethyl, 2-(2-thienyl)ethyl, 4-(2-thienyl)butyl, 2-(4-methyl-5-thiazolyl)ethyl, 4-pyridylmethyl, 3-pyridylmethyl, 2-pyridylmethyl, 2-(2-pyridyl)ethyl, 3-(3-pyridyl)allyl, 3-(3-pyridyl)propyl, 5-methoxy-2-indolylmethyl, 3-indolylmethyl, 1-methyl-2-indolylmethyl, 2-methyl-3-indolylmethyl, benzimidazol-2-ylmethyl, 4-quinolylmethyl, 3-quinolylmethyl, 2-quinolylmethyl, methyl, ethyl, propyl, butyl, isobutyl, pentyl, isopentyl, hexyl, heptyl, 2-methoxyethyl, 2-ethoxyethyl, 2-(2-methoxyethoxy)ethyl, 2-(2-ethoxyethoxy)ethyl, 2-phenoxyethyl, 2-(4-chlorophenoxy)ethyl,

2-(4-chlorophenoxy)propyl, 2-benzyloxyethyl, 2-(benzyloxyethoxy)ethyl, 1-pyrrolidinylmethyl, 2-(1-pyrrolidinyl)ethyl, piperidinomethyl, 2-piperidinoethyl, 3-piperidinopropyl, 2-(1-methyl-2-pyrrolidinyl)ethyl, morpholinomethyl, 2-morpholinoethyl, 3-morpholinopropyl, 4-methyl-1-piperazinylmethyl, 4-phenyl-1-piperazinylmethyl, 4-(4-ethoxycarbonylphenyl)-1-piperazinylmethyl, 2-(4-methyl-1-piperazinyl)-ethyl, 2-[4-(4-chlorophenyl)-1-piperazinyl]ethyl, 2-[4-(4-methoxyphenyl)-1-piperazinyl]ethyl and 2-(4-ethoxycarbonyl-1-piperazinyl)ethyl groups.

Examples of atoms and groups which may be represented by $R^3$ include the hydrogen atom and the fluoromethyl, chloromethyl, 2,2-dichloroethyl, 2,2,2-trichloroethyl, 2,2-di(trifluoromethyl)ethyl, hydroxymethyl, 2-hydroxyethyl, 1-hydroxyethyl, 1-methyl-2-hydroxyethyl, methoxymethyl, ethoxymethyl, t-butoxymethyl, 2-methoxyethyl, 2-ethoxyethyl, phenoxymethyl, benzyloxymethyl, 1-benzyloxyethyl, acetoxymethyl, mercaptomethyl, 2-mercaptoethyl, 2-methylthioethyl, 2-ethylthioethyl, 3-methylthiopropyl, 4-methylthiobutyl, phenylthiomethyl, benzylthiomethyl, 2-methylsulphinylethyl, 2-methanesulphonylethyl, 2-benzenesulphonylethyl, aminomethyl, 2-aminoethyl, 3-aminopropyl, 4-aminobutyl, 2-(diethylamino)ethyl, acetamidomethyl, 4-propionamidobutyl, 4-(t-butoxycarbonylamino)butyl, 3-(benzyloxycarbonylamino)propyl, carboxymethyl, 2-carboxyethyl, methoxycarbonylmethyl, t-butoxycarbonylmethyl, 2-(t-butoxycarbonyl)ethyl, phenoxycarbonylmethyl, benzyloxycarbonylmethyl, carbamoylmethyl, 2-carbamoylethyl, methylcarbamoylmethyl, (diethylcarbamoyl)methyl, ureidomethyl, 3-ureidopropyl, thioureidomethyl, 2-guanidinoethyl, 3-guanidinopropyl, 4-guanidinobutyl, cyclopropylmethyl, cyclopentylmethyl, cyclohexylmethyl, 2-cyclohexylethyl, 2-cyclopentenylmethyl, 2-(2-cyclopentenyl)ethyl, 1-cyclohexenylmethyl, 3-cyclohexenylmethyl, 1-cycloheptenylmethyl, benzyl, phenethyl, fluorobenzyl, methylbenzyl, chlorobenzyl, hydroxybenzyl, methoxybenzyl, nitrobenzyl, aminobenzyl, naphthylmethyl, 2,3,4,5,6-pentamethylbenzyl, furfuryl, thenyl, pyridylmethyl, 4-thiazolylmethyl, 4-methyl-5-thiazolylmethyl, 3-indolylmethyl, 3-benzothienylmethyl, pentyl, isopentyl, 2,3-dimethylpropyl, t-pentyl, 1-methylbutyl, 1-ethylpropyl, hexyl, 1-methylpentyl, isohexyl, 1,3-dimethylbutyl, heptyl, 5-methylhexyl, octyl, 1-methylheptyl, nonyl, decyl, vinyl, 1-methylvinyl, allyl, 3-butenyl, methallyl, 2-butenyl, 4-pentenyl, 2-methyl-3-butenyl, 2-methyl-2-butenyl, 3-methyl-2-butenyl, 2,2-dichlorovinyl, 3-chloro-3-butenyl, ethynyl, propargyl and 2-butynyl groups.

Examples of groups which may be represented by $R^5$ include the methoxy, ethoxy, phenoxy, 2-methylbutoxy, isopentyloxy, 2-ethylbutoxy, amino, methylamino, ethylamino, propylamino, isopropylamino, butylamino, isobutylamino, sec-butylamino, pentylamino, isopentylamino, 2-methylbutylamino, 1-methylbutylamino, hexylamino, 1,3-dimethylbutylamino, 3,3-dimethylbutylamino, heptylamino, octylamino, 1,5-dimethylhexylamino, nonylamino, dimethylamino, methyl(ethyl)amino, diethylamino, dipropylamino, diisopropylamino, dibutylamino, methyl(butyl)amino, ethyl(butyl)amino, diisobutylamino, dihexylamino, dioctylamino, dinonylamino, 2-hydroxyethylamino, 3-hydroxypropylamino, 1-(hydroxymethyl)ethylamino, 2-hydroxypropylamino, 4-hydroxybutylamino, 1-(hydroxymethyl)propylamino, 1-(hydroxymethyl)-2-methylpropylamino, 5-hydroxypentylamino, 6-hydroxyhexylamino, 1-(hydroxymethyl)-3-methylbutylamino, 1-(hydroxymethyl)-2-methylbutylamino, 1-(2-hydroxyethyl)-2-methylbutylamino, 1-(2-hydroxyethyl)-3-methylbutylamino, 1,1-di(hydroxymethyl)propylamino, 2,3-dihydroxypropylamino, 1-(hydroxymethyl)-2-hydroxypropylamino, 1-(1,2-dihydroxyethyl)-3-methylbutylamino, 1-(1,3-dihydroxypropyl)-3-methylbutylamino, β-hydroxyphenethylamino, α-(hydroxymethyl)phenethylamino,

di(2-hydroxyethyl)amino, 2-methoxyethylamino, 2-ethoxyethylamino, di(2-ethoxyethyl)amino, 2-chloroethylamino, 2-bromoethylamino, 2-fluoroethylamino, 3-chloropropylamino, 3-bromopropylamino, benzylamino, fluorobenzylamino, chlorobenzylamino, methylbenzylamino, methoxybenzylamino, dichlorobenzylamino, dimethylbenzylamino, phenethylamino, p-chlorophenethylamino, p-methoxyphenethylamino, m,p-dimethoxyphenethylamino, 2-phenylpropylamino, 3-phenylpropylamino, 4-phenylbutylamino, 2,2-diphenylethylamino, 3,3-diphenylpropylamino, ethyl(benzyl)amino, isopropyl(benzyl)amino, (2-pyridylmethyl)amino, (3-pyridylmethyl)amino, (4-pyridylmethyl)amino, 2-(2-pyridyl)ethylamino, cyclopropylmethylamino, cyclohexylmethylamino, 2-(dimethylamino)ethylamino, 2-(diethylamino)ethylamino, 3-(dimethylamino)propylamino, 3-(diethylamino)-propylamino, 3-(dibutylamino)propylamino, 2-[di(2-hydroxyethyl)amino]ethylamino, 3-[di(2-hydroxyethyl)amino]propylamino, (1-methyl-2-pyrrolidinylmethyl)amino, (1-ethyl-2-pyrrolidinylmethyl)amino, 2-(1-methyl-2-pyrrolidinyl)ethylamino, 2-(1-pyrrolidinyl)ethylamino, 2-piperidinoethylamino, 2-morpholinoethylamino, 3-morpholinopropylamino, 3-(4-methyl-1-piperazinyl)propylamino, allylamino, methallylamino, di(methallyl)amino, cyclopentylamino,

cyclohexylamino, cyclopropylamino, cyclobutylamino, cycloheptylamino, cyclooctylamino, phenylamino, tolylamino, chlorophenylamino, (methoxyphenyl)amino, 1-naphthylamino, (1-benzyl-4-piperidyl)amino, (1-p-methoxybenzyl-4-piperidyl)amino, 1-pyrrolidinyl, 2-carboxy-1-pyrrolidinyl, 2-methoxycarbonyl-1-pyrrolidinyl, 2-ethoxycarbonyl-1-pyrrolidinyl, 2-t-butoxycarbonyl-1-pyrrolidinyl, piperidino, methylpiperidino, (hydroxymethyl)piperidino, 4-phenylpiperidino, 4-benzylpiperidino, 4-methyl-1-piperazinyl, 4-(2-hydroxyethyl)-1-piperazinyl, 4-phenyl-1-piperazinyl, 4-tolyl-1-piperazinyl, 4-(chlorophenyl)-1-piperazinyl, 4-(methoxyphenyl)-1-piperazinyl, 4-benzyl-1-piperazinyl, 4-(m-methylbenzyl)-1-piperazinyl, 4-ethoxycarbonyl-1-piperazinyl, 4-cinnamoyl-1-piperazinyl, 4-(o, m or p-methylcinnamoyl)-1-piperazinyl, 4-(o, m or p-chlorocinnamoyl)-1-piperazinyl, 4-(o, m or p-methoxycinnamoyl)-1-piperazinyl, 4-(m, m, p-trimethoxycinnamoyl)-1-piperazinyl and 4-(2-pyridyl)-1-piperazinyl groups.

Of the compounds mentioned above, we particularly prefer the following classes of compound:

(A) Compounds in which:

m is 0;

$R^2$ represents a 1-naphthylmethyl group; and

$R^4$ represents an isopropyl group.

(B) Compounds as defined in (A) above, in which:

$R^1$ represents a $C_1$-$C_4$ alkyl group having a single ω-substituent selected from phenyl, 3-pyridyl, 1-naphthyl, 2-methoxyethoxy and 2-benzyloxyethoxy groups;

$R^3$ represents an allyl group, a 2-propynyl group, a $C_1$-$C_{10}$ alkyl group or a $C_1$-$C_4$ alkyl group having a single ω-substituent selected from 5-imidazolyl, methylthio, t-butoxycarbonylamino, carbamoyl, carboxy, 1,3-thiazol-4-yl, phenyl and cyclopentyl substituents; and

$R^5$ represents an amino group, a (1-benzyl-4-piperidyl)amino group or a $C_2$-$C_6$ alkylamino group having one or two substituents selected from hydroxy, $C_1$ or $C_2$ alkoxy, di($C_1$ or $C_2$ alkyl)amino and morpholino substituents.

(C) Compounds in which:

m is 0;

$R^1$ and $R^2$ both represent 1-naphthylmethyl groups; and

$R^4$ represents an isopropyl group.

(D) Compounds as defined in (C) above, in which:

$R^3$ represents an allyl group, a 2-propynyl group, a $C_1$-$C_5$ alkyl group or a $C_1$-$C_4$ alkyl group having a single ω-substituent selected from 5-imidazolyl, carbamoyl, 1,3-thiazol-4-yl and cyclopentyl substituents; and

$R^5$ represents a $C_2$-$C_6$ alkylamino group or a $C_2$-$C_6$ alkylamino group having a single substituent selected from hydroxy groups, di($C_1$ or $C_2$ alkyl)amino and morpholino groups.

The compounds of the invention contain at least one asymmetric carbon atom, that is to say the carbon atom to which the group represented by $R^4CH_2$ is attached, and can, depending upon the values of the various substituent groups $R^1$-$R^5$ defined above, also contain other asymmetric carbon atoms. Accordingly, a variety of optical isomers are possible. The present invention envisages both the individual isolated isomers as well

as mixtures (e.g. racemates) thereof. However, we particularly prefer those isomers in which the carbon atom to which the group represented by $R^4CH_2$ is attached has the S-configuration and, where $R^3$ represents a group other than hydrogen, we prefer those isomers in which the carbon atom to which the group represented by $R^3$ is attached also has the S-configuration. Where optically active starting materials are employed to produce the compounds of the invention and/or stereo-specific routes are employed, it may be possible to produce individual isomers of the compounds of the invention. In other cases, mixtures of various isomers may be produced and, in such a case, these mixtures may be used as such or the individual isomers may be isolated by well-known techniques.

The compounds of the invention contain basic nitrogen atoms and, depending upon the value of certain of the substituents represented by $R^1$-$R^5$, may also contain free carboxy groups. Accordingly, the compounds of the invention will normally form acid addition salts and may, where they contain at least one carboxy group, also form salts with cations. The nature of the acid or cation employed to form such a salt is not critical to the invention, except where the compounds of the invention are intended for therapeutic use, in which case the resulting salt must be pharmaceutically

acceptable which, as is well-known to those skilled in the art, means that the salt must not have an increased toxicity or substantially increased toxicity or a reduced activity or substantially reduced activity, as compared with the free compound of formula (I). However, where the compounds of the invention are intended for non-therapeutic use, e.g. as intermediates, even this limitation need not apply.

Examples of acids which can be employed to form acid addition salts include: mineral acids, such as hydrochloric acid, sulphuric acid and phosphoric acid; organic carboxylic acids, such as oxalic acid, maleic acid, succinic acid and citric acid; and organic sulphonic acids, such as methanesulphonic acid, benzenesulphonic acid and p-toluenesulphonic acid. The compounds may also form salts with: alkali and alkaline earth metals, such as sodium, potassium, calcium and magnesium; and organic bases, such as dicyclohexylamine.

The compounds of the invention having a carboxy group may likewise form esters and those esters which are particularly preferred in the present invention are defined as the various substituted oxycarbonyl groups. However, it will be appreciated that other esters may likewise be employed.

Specific examples of compounds of the invention are those compounds of formula (Ia):

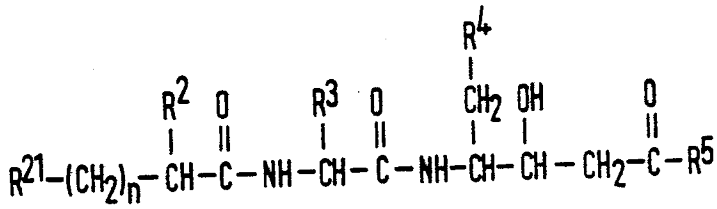

(Ia)

in which $R^{21}$, n, $R^2$, $R^3$, $R^4$ and $R^5$ are as defined in the following Table. In the Table, the following abbreviations are used:

| | |
|---|---|
| All | allyl |
| Bu | butyl |
| iBu | isobutyl |
| sBu | sec-butyl |
| tBu | t-butyl |
| Bun | 2-butenyl |
| Bz | benzyl |
| Car | carbamoyl |
| c-Pr | cyclopropyl |
| c-Pn | cyclopentyl |
| c-Hx | cyclohexyl |
| Cin | cinnamoyl |
| Dc | decyl |

| | |
|---|---|
| Et | ethyl |
| Fur | furyl |
| Hp | heptyl |
| Hx | hexyl |
| iHx | isohexyl |
| Imid | imidazolyl |
| Ind | indolyl |
| Me | methyl |
| Mor | morpholino |
| Nn | nonyl |
| Np | naphthyl |
| Oc | octyl |
| Ph | phenyl |
| Pip | piperidyl |
| Piz | piperazinyl |
| Pn | pentyl |
| iPn | isopentyl |
| Pr | propyl |
| iPr | isopropyl |
| Pyn | 2-propynyl |
| Pyr | pyridyl |
| Pyrd | pyrrolidinyl |
| Quin | quinolyl |
| Thi | thienyl |
| Thiz | 1,3-thiazolyl |

Where the free valency of a group denoted by one of

the above abbreviations needs to be specified, this is done by a number preceding the abbreviation for that group, e.g. the 3-pyridyl group is shown as "3-Pyr". Where a group denoted by one of the above abbreviations is itself substituted, the abbreviation for the substituted group is preceded by a symbol or abbreviation for the substituent and this, in turn, is, where appropriate, preceded by a number indicating the position of attachment of the substituent on the substituted group. For example, a 1-piperazinyl group having a phenyl substituent at the 4-position is shown as "4-Ph-1-Piz".

| Cpd No. | $R^{21}$ | n | $R^2$ | $R^3$ | $R^4$ | $R^5$ |
|---|---|---|---|---|---|---|
| 1 | Ph | 4 | 1-NpMe | iBu | iPr | $NH_2$ |
| 2 | Ph | 4 | 1-NpMe | iBu | iPr | EtNH |
| 3 | Ph | 4 | 1-NpMe | iBu | iPr | (2-MeBu)NH |
| 4 | Ph | 4 | 1-NpMe | iBu | iPr | (2,3-diOHPr)NH |
| 5 | Ph | 4 | 1-NpMe | iBu | iPr | (1-OHMe-2-OHPr)NH |
| 6 | Ph | 4 | 1-NpMe | iBu | iPr | (1-OHMe-2-MeBu)NH |
| 7 | Ph | 4 | 1-NpMe | iBu | iPr | (1-iBu-2,4-diOHBu)NH |
| 8 | Ph | 4 | 1-NpMe | iBu | iPr | (1-OHMe-3-MeBu)NH |
| 9 | Ph | 4 | 1-NpMe | iBu | iPr | [1,1-di(OHMe)Pr]NH |
| 10 | Ph | 4 | 1-NpMe | iBu | iPr | [3-di(2-OHEt)NPr]NH |
| 11 | Ph | 4 | 1-NpMe | iBu | iPr | (1-Bz-4-Pip)NH |
| 12 | Ph | 4 | 1-NpMe | iBu | iPr | [2-(3,4-diOMePh)Et]NH |
| 13 | Ph | 4 | 1-NpMe | iBu | iPr | 4-(2-OHEt)-1-Piz |
| 14 | Ph | 4 | 1-NpMe | iBu | iPr | [2-(2-Pyr)Et]NH |
| 15 | Ph | 4 | 1-NpMe | iBu | iPr | (2-MorEt)NH |
| 16 | Ph | 4 | 1-NpMe | iBu | iPr | c-Hx-NH |
| 17 | Ph | 4 | 1-NpMe | 5-ImidMe | iPr | (2-MeBu)NH |
| 18 | Ph | 4 | 1-NpMe | 5-ImidMe | iPr | (1-OHMe-2-MeBu)NH |
| 19 | Ph | 4 | 1-NpMe | 5-ImidMe | iPr | [3-di(2-OHEt)NPr]NH |
| 20 | Ph | 4 | 1-NpMe | iBu | Ph | (1-OHMe-2-MeBu)NH |
| 21 | Ph | 4 | 1-NpMe | iBu | c-Hx | (1-OHMe-2-MeBu)NH |
| 22 | Ph | 4 | 1-NpMe | Bu | iPr | (1-OHMe-2-MeBu)NH |

| Cpd No. | $R^{21}$ | n | $R^2$ | $R^3$ | $R^4$ | $R^5$ |
|---|---|---|---|---|---|---|
| 23 | Ph | 4 | 1-NpMe | Bu | iPr | [3-di(2-OHEt)NPr]NH |
| 24 | Ph | 4 | 1-NpMe | iPr | iPr | (1-OHMe-2-MeBu)NH |
| 25 | Ph | 4 | Bz | iBu | iPr | (1-OHMe-2-MeBu)NH |
| 26 | Ph | 4 | Bz | 5-ImidMe | iPr | (1-OHMe-2-MeBu)NH |
| 27 | Ph | 4 | Bz | 5-ImidMe | iPr | (1-OHMe-3-MeBu)NH |
| 28 | Ph | 4 | 2-PyrMe | iBu | iPr | (1-OHMe-2-MeBu)NH |
| 29 | Ph | 3 | 1-NpMe | iBu | iPr | (1-OHMe-2-MeBu)NH |
| 30 | Ph | 3 | 1-NpMe | iBu | iPr | (2-[4-(2,3-diOHPr)-<br>-1-Piz]Et)NH |
| 31 | Ph | 3 | 1-NpMe | iBu | iPr | [3-di(2-OHEt)NPr]NH |
| 32 | Ph | 3 | 1-NpMe | 5-ImidMe | iPr | (1-OHMe-2-MeBu)NH |
| 33 | Ph | 3 | 1-NpMe | Bu | iPr | (1-OHMe-2-MeBu)NH |
| 34 | Ph | 2 | 1-NpMe | iBu | iPr | (1-iBu-2,4-diOHBu)NH |
| 35 | Ph | 2 | 1-NpMe | 5-ImidMe | iPr | (1-OHMe-2-MeBu)NH |
| 36 | Ph | 1 | 1-NpMe | iBu | iPr | (1-OHMe-2-MeBu)NH |
| 37 | 4-ClPh | 1 | 1-NpMe | 5-ImidMe | iPr | (1-OHMe-2-MeBu)NH |
| 38 | 4-MeOPh | 1 | 1-NpMe | iBu | iPr | (1-OHMe-2-MeBu)NH |
| 39 | Np | 1 | 1-NpMe | iBu | iPr | (1-OHMe-2-MeBu)NH |
| 40 | Np | 1 | 1-NpMe | iBu | iPr | (2-MeBu)NH |
| 41 | Np | 1 | 1-NpMe | iBu | iPr | EtNH |
| 42 | Np | 1 | 1-NpMe | iBu | iPr | $NH_2$ |
| 43 | Np | 1 | 1-NpMe | iBu | iPr | OH |
| 44 | Np | 1 | 1-NpMe | iBu | iPr | iBuNH |
| 45 | Np | 1 | 1-NpMe | iBu | iPr | (2-EtOEt)NH |

| Cpd No. | $R^{21}$ | n | $R^2$ | $R^3$ | $R^4$ | $R^5$ |
|---|---|---|---|---|---|---|
| 46 | Np | 1 | 1-NpMe | iBu | iPr | (1-OHMe-2-MePr)NH |
| 47 | Np | 1 | 1-NpMe | iBu | iPr | [1,1-di(OHMe)Pr]NH |
| 48 | Np | 1 | 1-NpMe | iBu | iPr | (1-iBu-2,4-diOHBu)NH |
| 49 | Np | 1 | 1-NpMe | iBu | iPr | (1-OHMe-3-MeBu)NH |
| 50 | Np | 1 | 1-NpMe | iBu | iPr | 2-($NEt_2$)EtNH |
| 51 | Np | 1 | 1-NpMe | iBu | iPr | (2-PhEt)NH |
| 52 | Np | 1 | 1-NpMe | iBu | iPr | 2-(3,4-diMeOPh)EtNH |
| 53 | Np | 1 | 1-NpMe | iBu | iPr | (2-MorEt)NH |
| 54 | Np | 1 | 1-NpMe | iBu | iPr | (3-MorPr)NH |
| 55 | Np | 1 | 1-NpMe | iBu | iPr | [2-(1-Me-2-Pyrd)Et]NH |
| 56 | Np | 1 | 1-NpMe | iBu | iPr | [(1-Et-2-Pyrd)Me]NH |
| 57 | Np | 1 | 1-NpMe | iBu | iPr | [3-(4-Me-1-Piz)Pr]NH |
| 58 | Np | 1 | 1-NpMe | iBu | iPr | (1-Bz-4-Pip)NH |
| 59 | Np | 1 | 1-NpMe | iBu | iPr | [2-(2-Pyr)Et]NH |
| 60 | Np | 1 | 1-NpMe | iBu | iPr | c-Hx-NH |
| 61 | Np | 1 | 1-NpMe | iBu | iPr | 4-(2,3-diOHPr)-1-Piz |
| 62 | Np | 1 | 1-NpMe | iBu | iPr | 4-(3-OHPr)-1-Piz |
| 63 | Np | 1 | 1-NpMe | iBu | iPr | 4-(2-OHEt)-1-Piz |
| 64 | Np | 1 | 1-NpMe | iBu | iPr | [3-di(2-OHEt)NPr]NH |
| 65 | Np | 1 | 1-NpMe | iBu | iPr | 4-Ph-1-Piz |
| 66 | Np | 1 | 1-NpMe | iBu | iPr | 4-Cin-1-Piz |
| 67 | Np | 1 | 1-NpMe | iBu | iPr | 4-(3,4,5-triMeOCin)-<br>-1-Piz |
| 68 | Np | 1 | 1-NpMe | iBu | Ph | (1-OHMe-2-MeBu)NH |

| Cpd No. | $R^{21}$ | n | $R^2$ | $R^3$ | $R^4$ | $R^5$ |
|---|---|---|---|---|---|---|
| 69 | Np | 1 | 1-NpMe | iBu | c-Hx | (1-OHMe-2--MeBu)NH |
| 70 | Np | 1 | 1-NpMe | 5-ImidMe | iPr | (3-MorPr)NH |
| 71 | Np | 1 | 1-NpMe | 5-ImidMe | iPr | $NH_2$ |
| 72 | Np | 1 | 1-NpMe | 5-ImidMe | iPr | (1-OHMe-2-MeBu)NH |
| 73 | Np | 1 | 1-NpMe | 5-ImidMe | iPr | iBuNH |
| 74 | Np | 1 | 1-NpMe | 5-ImidMe | iPr | (2-MeBu)NH |
| 75 | Np | 1 | 1-NpMe | 5-ImidMe | iPr | [3-di(2-OHEt)NPr]NH |
| 76 | Np | 1 | 1-NpMe | 5-ImidMe | iPr | (2-$NMe_2$Et)NH |
| 77 | Np | 1 | 1-NpMe | 5-ImidMe | iPr | (2-MorEt)NH |
| 78 | Np | 1 | 1-NpMe | 5-ImidMe | iPr | [2-(2-Pyr)Et]NH |
| 79 | Np | 1 | 1-NpMe | 5-ImidMe | iPr | (1-Bz-4-Pip)NH |
| 80 | Np | 1 | 1-NpMe | 5-ImidMe | iPr | (1-Et-2-PyrdMe)NH |
| 81 | Np | 1 | 1-NpMe | 5-ImidMe | Ph | (1-OHMe-2-MeBu)NH |
| 82 | Np | 1 | 1-NpMe | sBu | iPr | [3-di(2-OHEt)NPr]NH |
| 83 | Np | 1 | 1-NpMe | sBu | iPr | (1-OHMe-2-MeBu)NH |
| 84 | Np | 1 | 1-NpMe | sBu | iPr | (2-MorEt)NH |
| 85 | Np | 1 | 1-NpMe | sBu | iPr | (3-MorPr)NH |
| 86 | Np | 1 | 1-NpMe | sBu | iPr | (1-Bz-4-Pip)NH |
| 87 | Np | 1 | 1-NpMe | Bu | iPr | [3-di(2-OHEt)NPr]NH |
| 88 | Np | 1 | 1-NpMe | Bu | iPr | (1-OHMe-2-MeBu)NH |
| 89 | Np | 1 | 1-NpMe | Bu | iPr | (2-MorEt)NH |
| 90 | Np | 1 | 1-NpMe | Bu | iPr | 2-($NEt_2$)EtNH |
| 91 | Np | 1 | 1-NpMe | Bu | iPr | (1-Et-2-PyrdMe)NH |

| Cpd No. | $R^{21}$ | n | $R^2$ | $R^3$ | $R^4$ | $R^5$ |
|---|---|---|---|---|---|---|
| 92 | Np | 1 | 1-NpMe | Bu | iPr | [2-(2-Pyr)Et]NH |
| 93 | Np | 1 | 1-NpMe | Bu | iPr | [3-di(2-OHEt)NPr]NH |
| 94 | Np | 1 | 1-NpMe | Bu | iPr | 4-(2-OHEt)-1-Piz |
| 95 | Np | 1 | 1-NpMe | iPr | iPr | (1-OHMe-2-MeBu)NH |
| 96 | Np | 1 | 1-NpMe | iPr | iPr | (1-OHMe-2-MePr)NH |
| 97 | Np | 1 | 1-NpMe | iPr | iPr | 2-($NEt_2$)EtNH |
| 98 | Np | 1 | 1-NpMe | iPr | iPr | (2-MorEt)NH |
| 99 | Np | 1 | 1-NpMe | iPr | iPr | (3-MorPr)NH |
| 100 | Np | 1 | 1-NpMe | iPr | iPr | [3-(4-Me-1-Piz)Pr]NH |
| 101 | Np | 1 | 1-NpMe | iPr | iPr | [2-(2-Pyr)Et]NH |
| 102 | Np | 1 | 1-NpMe | iPr | iPr | (1-Bz-4-Pip)NH |
| 103 | Np | 1 | 1-NpMe | iPr | iPr | (1-Et-2-PyrdMe)NH |
| 104 | Np | 1 | 1-NpMe | Pr | iPr | (1-OHMe-2-MeBu)NH |
| 105 | Np | 1 | 1-NpMe | Pr | iPr | (2-MorEt)NH |
| 106 | Np | 1 | 1-NpMe | Pr | iPr | [3-di(2-OHEt)NPr]NH |
| 107 | Np | 1 | 1-NpMe | Et | iPr | (1-OHMe-2-MeBu)NH |
| 108 | Np | 1 | 1-NpMe | Et | iPr | [3-di(2-OHEt)NPr]NH |
| 109 | Np | 1 | 1-NpMe | Me | iPr | (1-OHMe-2-MeBu)NH |
| 110 | Ph | 1 | Bz | iBu | iPr | (1-OHMe-2-MeBu)NH |
| 111 | 3-Pyr | 3 | 1-NpMe | iBu | iPr | (1-OHMe-2-MeBu)NH |
| 112 | 3-Pyr | 3 | 1-NpMe | 5-ImidMe | iPr | (1-OHMe-2-MeBu)NH |
| 113 | 3-Pyr | 1 | 1-NpMe | iBu | iPr | (1-OHMe-2-MeBu)NH |
| 114 | 3-Pyr | 1 | 1-NpMe | 5-ImidMe | iPr | (1-OHMe-2-MeBu)NH |
| 115 | 3-Pyr | 1 | 1-NpMe | Bu | iPr | (1-OHMe-2-MeBu)NH |

| Cpd No. | $R^{21}$ | n | $R^2$ | $R^3$ | $R^4$ | $R^5$ |
|---|---|---|---|---|---|---|
| 116 | 4-Pyr | 1 | 1-NpMe | iBu | iPr | (1-OHMe-2-MeBu)NH |
| 117 | 4-Pyr | 1 | 1-NpMe | 5-ImidMe | iPr | (1-OHMe-2-MeBu)NH |
| 118 | 4-Pyr | 1 | 1-NpMe | Bu | iPr | (1-OHMe-2-MeBu)NH |
| 119 | 2-Pyr | 1 | 1-NpMe | iBu | iPr | (1-OHMe-2-MeBu)NH |
| 120 | 2-Pyr | 1 | 1-NpMe | 5-ImidMe | iPr | (1-OHMe-2-MeBu)NH |
| 121 | 2-Pyr | 1 | 1-NpMe | Bu | iPr | (1-OHMe-2-MeBu)NH |
| 122 | 3-Ind | 1 | 1-NpMe | iBu | iPr | (1-OHMe-2-MeBu)NH |
| 123 | 1-Me-3-Ind | 1 | 1-NpMe | iBu | iPr | (1-OHMe-2-MeBu)NH |
| 124 | 4-Quin | 1 | 4-QuinMe | iBu | iPr | (1-OHMe-2-MeBu)NH |
| 125 | 2-Thi | 4 | 1-NpMe | iBu | iPr | (1-OHMe-2-MeBu)NH |
| 126 | 2-Thi | 1 | 1-NpMe | iBu | iPr | (1-OHMe-2-MeBu)NH |
| 127 | 5-Me-2-Thi | 1 | 1-NpMe | iBu | iPr | (1-OHMe-2-MeBu)NH |
| 128 | 2-Fur | 1 | 1-NpMe | iBu | iPr | (1-OHMe-2-MeBu)NH |
| 129 | 4-Me-5-Thiz | 2 | 1-NpMe | 5-ImidMe | iPr | (1-OHMe-2-MeBu)NH |
| 130 | 2-Thi | 1 | 2-ThiMe | iBu | iPr | (1-OHMe-2-MeBu)NH |
| 131 | 1-Pip | 3 | 1-NpMe | iBu | iPr | (1-OHMe-2-MeBu)NH |
| 132 | 1-Pip | 3 | 1-NpMe | Bu | iPr | (1-Bz-4-Pip)NH |
| 133 | 1-Pip | 3 | 1-NpMe | Bu | iPr | (1-OHMe-2-MeBu)NH |
| 134 | 1-Pip | 2 | 1-NpMe | iBu | iPr | (2-MorEt)NH |
| 135 | 1-Me-2-Pyrd | 2 | 1-NpMe | iBu | iPr | (1-OHMe-2-MeBu)NH |
| 136 | 4-Me-1-Piz | 2 | 1-NpMe | iBu | iPr | (2-MorEt)NH |

| Cpd No. | $R^{21}$ | n | $R^2$ | $R^3$ | $R^4$ | $R^5$ |
|---|---|---|---|---|---|---|
| 137 | 4-Ph-1-Piz | 2 | 1-NpMe | iBu | iPr | (1-OHMe-2-MeBu)NH |
| 138 | 4-(4-MeOPh)- -1-Piz | 2 | 1-NpMe | iBu | iPr | (1-OHMe-2-MeBu)NH |
| 139 | Mor | 3 | 1-NpMe | 5-ImidMe | iPr | (1-OHMe-2-MeBu)NH |
| 140 | Mor | 2 | 1-NpMe | iBu | iPr | (1-OHMe-2-MeBu)NH |
| 141 | Mor | 2 | 1-NpMe | iBu | iPr | [3-di(2-OHEt)NPr]NH |
| 142 | Mor | 2 | 1-NpMe | iBu | iPr | $NH_2$ |
| 143 | Mor | 2 | 1-NpMe | iBu | iPr | (1-OHMe-2-OHPr)NH |
| 144 | Mor | 2 | 1-NpMe | 5-ImidMe | iPr | (1-OHMe-2-MeBu)NH |
| 145 | Mor | 2 | 1-NpMe | Bu | iPr | (1-OHMe-2-MeBu)NH |
| 146 | 4-Ph-1-Piz | 2 | 1-NpMe | Bu | iPr | (1-OHMe-2-MeBu)NH |
| 147 | Mor | 2 | 1-NpMe | Bu | iPr | (2-MorEt)NH |
| 148 | Mor | 2 | 3-PyrMe | 5-ImidMe | iPr | (1-OHMe-2-MeBu)NH |
| 149 | Mor | 2 | 1-NpMe | iBu | iPr | (2-MorEt)NH |
| 150 | Oc | 0 | 1-NpMe | iBu | iPr | (1-OHMe-2-MeBu)NH |
| 151 | BzO | 2 | 1-NpMe | iBu | iPr | (1-OHMe-2-MeBu)NH |
| 152 | 1-NpO | 2 | Bz | 5-ImidMe | iPr | (1-OHMe-2-MeBu)NH |
| 153 | 2-MeOEtO | 2 | 1-NpMe | iBu | iPr | (1-OHMe-2-MeBu)NH |
| 154 | 2-MeOEtO | 2 | 1-NpMe | Bu | iPr | (1-OHMe-2-MeBu)NH |
| 155 | 2-MeOEtO | 2 | 1-NpMe | Bu | iPr | (1-OHMe-3-OHPr)NH |
| 156 | 2-MeOEtO | 2 | 1-NpMe | Bu | iPr | [3-di(2-OHEt)NPr]NH |
| 157 | 2-(EtO)EtO | 2 | 1-NpMe | iBu | iPr | (1-OHMe-2-MeBu)NH |
| 158 | 2-BzOEtO | 2 | 1-NpMe | iBu | iPr | (1-OHMe-2-MeBu)NH |
| 159 | Ph | 4 | 1-NpMe | HOMe | iPr | $NH_2$ |

| Cpd No. | $R^{21}$ | n | $R^2$ | $R^3$ | $R^4$ | $R^5$ |
|---|---|---|---|---|---|---|
| 160 | Ph | 4 | 1-NpMe | HOMe | iPr | (1-OHMe-2-MeBu)NH |
| 161 | Ph | 4 | 1-NpMe | EtOMe | iPr | (1-OHMe-2-MeBu)NH |
| 162 | Ph | 4 | 1-NpMe | 2-MeSEt | iPr | (1-OHMe-2-MeBu)NH |
| 163 | Ph | 4 | 1-NpMe | 2-CarEt | iPr | (1-OHMe-2-MeBu)NH |
| 164 | Ph | 4 | 1-NpMe | Bz | iPr | (1-OHMe-2-MeBu)NH |
| 165 | Ph | 4 | 1-NpMe | 3-PyrMe | iPr | (1-OHMe-2-MeBu)NH |
| 166 | Ph | 4 | 1-NpMe | 2-PyrMe | iPr | (1-OHMe-2-MeBu)NH |
| 167 | Ph | 3 | 1-NpMe | EtOMe | iPr | (1-OHMe-2-MeBu)NH |
| 168 | Ph | 3 | 1-NpMe | 2-MeSEt | iPr | (1-OHMe-2-MeBu)NH |
| 169 | Ph | 3 | 1-NpMe | CarMe | iPr | (1-OHMe-2-MeBu)NH |
| 170 | Ph | 3 | 1-NpMe | 2-CarEt | iPr | (1-OHMe-2-MeBu)NH |
| 171 | Ph | 3 | 1-NpMe | 4-ThizMe | iPr | (1-OHMe-2-MeBu)NH |
| 172 | Ph | 3 | 1-NpMe | 2-ThiMe | iPr | (1-OHMe-2-MeBu)NH |
| 173 | Ph | 3 | 1-NpMe | c-Pr-Me | iPr | (1-OHMe-2-MeBu)NH |
| 174 | Ph | 2 | 1-NpMe | H | iPr | (1-OHMe-2-MeBu)NH |
| 175 | Ph | 2 | 1-NpMe | EtOMe | iPr | (1-OHMe-2-MeBu)NH |
| 176 | Ph | 2 | 1-NpMe | 2-MeSEt | iPr | (1-OHMe-2-MeBu)NH |
| 177 | Ph | 2 | 1-NpMe | CarMe | iPr | (1-OHMe-2-MeBu)NH |
| 178 | Ph | 2 | 1-NpMe | 2-CarEt | iPr | (1-OHMe-2-MeBu)NH |
| 179 | Ph | 2 | 1-NpMe | 4-ThizMe | iPr | (1-OHMe-2-MeBu)NH |
| 180 | Ph | 2 | Bz | 2-MeSEt | iPr | (1-OHMe-2-MeBu)NH |
| 181 | Ph | 2 | Bz | 2-CarEt | iPr | (1-OHMe-2-MeBu)NH |
| 182 | Ph | 1 | 1-NpMe | H | iPr | (1-OHMe-2-MeBu)NH |
| 183 | Ph | 1 | 1-NpMe | HOMe | iPr | (1-OHMe-2-MeBu)NH |

| Cpd No. | $R^{21}$ | n | $R^2$ | $R^3$ | $R^4$ | $R^5$ |
|---|---|---|---|---|---|---|
| 184 | Ph | 1 | 1-NpMe | 2-MeSEt | iPr | (1-OHMe-2-MeBu)NH |
| 185 | Ph | 1 | 1-NpMe | CarMe | iPr | [3-di(2-OHEt)NPr]NH |
| 186 | Ph | 1 | 1-NpMe | 2-CarEt | iPr | [3-di(2-OHEt)NPr]NH |
| 187 | Ph | 1 | 1-NpMe | 2-CarEt | iPr | (1-OHMe-2-MeBu)NH |
| 188 | Ph | 1 | 4-QuinMe | 4-ThizMe | iPr | (1-OHMe-2-MeBu)NH |
| 189 | 1-Np | 1 | 1-NpMe | H | iPr | (1-OHMe-2-MeBu)NH |
| 190 | 1-Np | 1 | 1-NpMe | HOMe | iPr | 2-MeBuNH |
| 191 | 1-Np | 1 | 1-NpMe | HOMe | iPr | (1-OHMe-2-MeBu)NH |
| 192 | 1-Np | 1 | 1-NpMe | HOMe | iPr | (2-MorEt)NH |
| 193 | 1-Np | 1 | 1-NpMe | EtOMe | iPr | (1-OHMe-2-MeBu)NH |
| 194 | 1-Np | 1 | 1-NpMe | MeOMe | iPr | (1-OHMe-2-MeBu)NH |
| 195 | 1-Np | 1 | 1-NpMe | BzOMe | iPr | (1-OHMe-2-MeBu)NH |
| 196 | 1-Np | 1 | 1-NpMe | 2-MeSEt | iPr | (1-OHMe-2-MeBu)NH |
| 197 | 1-Np | 1 | 1-NpMe | 2-MeSEt | iPr | [3-di(2-OHEt)NPr]NH |
| 198 | 1-Np | 1 | 1-NpMe | 2-(MeS→O)Et | iPr | (1-OHMe-2-MeBu)NH |
| 199 | 1-Np | 1 | 1-NpMe | 2-($MeSO_2$)Et | iPr | (1-OHMe-2-MeBu)NH |
| 200 | 1-Np | 1 | 1-NpMe | 2-($MeSO_2$)Et | iPr | (2-MeBu)NH |
| 201 | 1-Np | 1 | 1-NpMe | 4-MeSBu | iPr | (1-OHMe-2-MeBu)NH |
| 202 | 1-Np | 1 | 1-NpMe | 4-$NH_2$Bu | iPr | (1-OHMe-2-MeBu)NH |
| 203 | 1-Np | 1 | 1-NpMe | 4-(tBuOCONH)Bu | iPr | (1-OHMe-2-MeBu)NH |
| 204 | 1-Np | 1 | 1-NpMe | CarMe | iPr | [3-di(2-OHEt)NPr]NH |
| 205 | 1-Np | 1 | 1-NpMe | CarMe | iPr | (1-OHMe-2-MeBu)NH |
| 206 | 1-Np | 1 | 1-NpMe | CarMe | iPr | $NH_2$ |
| 207 | 1-Np | 1 | 1-NpMe | 2-CarEt | iPr | [3-di(2-OHEt)NPr]NH |

| Cpd No. | $R^{21}$ | n | $R^2$ | $R^3$ | $R^4$ | $R^5$ |
|---|---|---|---|---|---|---|
| 208 | 1-Np | 1 | 1-NpMe | 2-CarEt | iPr | (1-OHMe-2-MeBu)NH |
| 209 | 1-Np | 1 | 1-NpMe | 2-CarEt | iPr | $NH_2$ |
| 210 | 1-Np | 1 | 1-NpMe | 2-(tBuOCO)Et | iPr | (1-OHMe-2-MeBu)NH |
| 211 | 1-Np | 1 | 1-NpMe | 2-(HOOC)Et | iPr | (2-MorEt)NH |
| 212 | 1-Np | 1 | 1-NpMe | 2-(HOOC)Et | iPr | (1-OHMe-2-MeBu)NH |
| 213 | 1-Np | 1 | 1-NpMe | Bz | iPr | (1-OHMe-2-MeBu)NH |
| 214 | 1-Np | 1 | 1-NpMe | Bz | iPr | [3-di(2-OHEt)NPr]NH |
| 215 | 1-Np | 1 | 1-NpMe | 4-ClBz | iPr | (1-OHMe-2-MeBu)NH |
| 216 | 1-Np | 1 | 1-NpMe | 4-MeBz | iPr | (2-MorEt)NH |
| 217 | 1-Np | 1 | 1-NpMe | 2-ThiMe | iPr | (1-OHMe-2-MeBu)NH |
| 218 | 1-Np | 1 | 1-NpMe | 2-Thi | iPr | (1-OHMe-2-MeBu)NH |
| 219 | 1-Np | 1 | 1-NpMe | 2-FurMe | iPr | (1-OHMe-2-MeBu)NH |
| 220 | 1-Np | 1 | 1-NpMe | 4-Me-5-ThizMe | iPr | (1-OHMe-2-MeBu)NH |
| 221 | 1-Np | 1 | 1-NpMe | 4-ThizMe | iPr | $NH_2$ |
| 222 | 1-Np | 1 | 1-NpMe | 4-ThizMe | iPr | EtNH |
| 223 | 1-Np | 1 | 1-NpMe | 4-ThizMe | iPr | (1-OHMe-2-MeBu)NH |
| 224 | 1-Np | 1 | 1-NpMe | 4-ThizMe | iPr | (1-OHMe-3-MeBu)NH |
| 225 | 1-Np | 1 | 1-NpMe | 4-ThizMe | iPr | (1-OHMe-2-OHPr)NH |
| 226 | 1-Np | 1 | 1-NpMe | 4-ThizMe | iPr | 2-($NEt_2$)EtNH |
| 227 | 1-Np | 1 | 1-NpMe | 4-ThizMe | iPr | 4-(2,3-diOHPr)-1-Piz |
| 228 | 1-Np | 1 | 1-NpMe | 4-ThizMe | iPr | [3-di(2-OHEt)NPr]NH |
| 229 | 1-Np | 1 | 1-NpMe | 4-ThizMe | Ph | (1-OHMe-2-MeBu)NH |
| 230 | 1-Np | 1 | 1-NpMe | 2-Pyr | iPr | (1-OHMe-2-MeBu)NH |
| 231 | 1-Np | 1 | 1-NpMe | 3-PyrMe | iPr | (1-OHMe-2-MeBu)NH |

| Cpd No. | $R^{21}$ | n | $R^2$ | $R^3$ | $R^4$ | $R^5$ |
|---|---|---|---|---|---|---|
| 232 | 1-Np | 1 | 1-NpMe | 4-PyrMe | iPr | (1-OHMe-2-MeBu)NH |
| 233 | 1-Np | 1 | 1-NpMe | 3-IndMe | iPr | (1-OHMe-2-MeBu)NH |
| 234 | 1-Np | 1 | 1-NpMe | c-Pr-Me | iPr | (1-OHMe-2-MeBu)NH |
| 235 | 1-Np | 1 | 1-NpMe | c-Pn-Me | iPr | (1-OHMe-2-MeBu)NH |
| 236 | 1-Np | 1 | 1-NpMe | c-Hx-Me | iPr | (1-OHMe-2-MeBu)NH |
| 237 | 1-Np | 1 | 1-NpMe | HSMe | iPr | (1-OHMe-2-MeBu)NH |
| 238 | 1-Np | 1 | 1-NpMe | MeSMe | iPr | (1-OHMe-2-MeBu)NH |
| 239 | 1-Np | 1 | 1-NpMe | EtSMe | iPr | (1-OHMe-2-MeBu)NH |
| 240 | 1-Np | 1 | 1-NpMe | 2-HSEt | iPr | (1-OHMe-2-MeBu)NH |
| 241 | 1-Np | 1 | 1-NpMe | c-Hx | iPr | (1-OHMe-2-MeBu)NH |
| 242 | 1-Np | 1 | 1-NpMe | c-Pn | iPr | (1-OHMe-2-MeBu)NH |
| 243 | 2-Pyr | 1 | 1-NpMe | 4-ThizMe | iPr | (1-OHMe-2-MeBu)NH |
| 244 | 3-Pyr | 1 | 1-NpMe | 2-MeSEt | iPr | (1-OHMe-2-MeBu)NH |
| 245 | 4-Quin | 1 | 1-NpMe | 2-MeSEt | iPr | (1-OHMe-2-MeBu)NH |
| 246 | Mor | 2 | 1-NpMe | 2-CarEt | iPr | $NH_2$ |
| 247 | Mor | 2 | 1-NpMe | 2-CarEt | iPr | (1-OHMe-2-MeBu)NH |
| 248 | Mor | 2 | 1-NpMe | 2-CarEt | iPr | [3-di(2-OHEt)NPr]NH |
| 249 | 4-Me-1-Piz | 2 | 1-NpMe | 2-CarEt | iPr | [3-di(2-OHEt)NPr]NH |
| 250 | 4-Me-1-Piz | 2 | 1-NpMe | 2-CarEt | iPr | $NH_2$ |
| 251 | 4-Quin | 1 | 1-NpMe | Bz | iPr | (1-OHMe-2-MeBu)NH |
| 252 | 4-Ph-1-Piz | 2 | 1-NpMe | 4-ThizMe | iPr | (1-OHMe-2-MeBu)NH |
| 253 | 4-Me-1-Piz | 2 | 1-NpMe | 4-ThizMe | iPr | (1-OHMe-2-MeBu)NH |
| 254 | 4-Me-1-Piz | 1 | 1-NpMe | 4-ThizMe | iPr | [3-di(2-OHEt)NPr]NH |
| 255 | 4-Me-1-Piz | 1 | 1-NpMe | c-Pr-Me | iPr | (1-OHMe-2-MeBu)NH |

| Cpd No. | $R^{21}$ | n | $R^2$ | $R^3$ | $R^4$ | $R^5$ |
|---|---|---|---|---|---|---|
| 256 | 2-Thi | 1 | 1-NpMe | 4-ThizMe | iPr | (1-OHMe-2-MeBu)NH |
| 257 | Mor | 2 | 1-NpMe | 4-ThizMe | iPr | (1-OHMe-2-MeBu)NH |
| 258 | Mor | 2 | 1-NpMe | 4-ThizMe | iPr | (1-OHMe-2-OHPr)NH |
| 259 | Mor | 2 | 1-NpMe | 4-ThizMe | iPr | [3-di(2-OHEt)NPr]NH |
| 260 | Mor | 2 | 1-NpMe | 4-ThizMe | iPr | $NH_2$ |
| 261 | Mor | 2 | 1-NpMe | 2-MeSEt | iPr | [2-di(2-OHEt)NEt]NH |
| 262 | Mor | 2 | 1-NpMe | 2-MeSEt | iPr | (1-OHMe-2-MeBu)NH |
| 263 | Mor | 2 | 1-NpMe | 2-MeSEt | iPr | [3-di(2-OHEt)NPr]NH |
| 264 | Ph | 1 | 1-NpMe | Pn | iPr | (1-OHMe-2-MeBu)NH |
| 265 | Ph | 1 | 1-NpMe | 1-MeBu | iPr | (1-OHMe-2-MeBu)NH |
| 266 | Ph | 1 | 1-NpMe | iHx | iPr | (1-OHMe-2-MeBu)NH |
| 267 | Ph | 1 | 1-NpMe | 5-MeHx | iPr | (1-OHMe-2-MeBu)NH |
| 268 | 4-ClPh | 1 | 1-NpMe | Pn | iPr | (1-OHMe-2-MeBu)NH |
| 269 | 4-ClPh | 1 | 1-NpMe | iPn | iPr | (1-OHMe-2-MeBu)NH |
| 270 | 4-MeOPh | 1 | 1-NpMe | Pn | iPr | (1-OHMe-2-MeBu)NH |
| 271 | 1-Np | 1 | 1-NpMe | Pn | iPr | EtO |
| 272 | 1-Np | 1 | 1-NpMe | Pn | iPr | $NH_2$ |
| 273 | 1-Np | 1 | 1-NpMe | Pn | iPr | EtNH |
| 274 | 1-Np | 1 | 1-NpMe | Pn | iPr | 2-MeBuNH |
| 275 | 1-Np | 1 | 1-NpMe | Pn | iPr | (1-OHMe-2-MeBu)NH |
| 276 | 1-Np | 1 | 1-NpMe | Pn | iPr | (2-MorEt)NH |
| 277 | 1-Np | 1 | 1-NpMe | Pn | iPr | c-Hx-NH |
| 278 | 4-Me-1-Piz | 1 | 1-NpMe | Pn | iPr | $NH_2$ |
| 279 | 4-Me-1-Piz | 1 | 1-NpMe | Pn | iPr | (1-OHMe-2-MeBu)NH |

| Cpd No. | $R^{21}$ | n | $R^2$ | $R^3$ | $R^4$ | $R^5$ |
|---|---|---|---|---|---|---|
| 280 | 1-Np | 1 | 1-NpMe | Pn | Ph | (1-OHMe-2-MeBu)NH |
| 281 | 1-Np | 1 | 1-NpMe | Pn | c-Hx | 2-MeBuNH |
| 282 | 1-Np | 1 | 1-NpMe | Pn | c-Hx | (1-OHMe-2-MeBu)NH |
| 283 | 1-Np | 1 | 1-NpMe | 1-EtPr | iPr | (1-OHMe-2-MeBu)NH |
| 284 | 1-Np | 1 | 1-NpMe | Hx | iPr | (1-OHMe-2-MeBu)NH |
| 285 | 1-Np | 1 | 1-NpMe | iHx | iPr | (1-OHMe-2-MeBu)NH |
| 286 | 1-Np | 1 | 1-NpMe | Hp | iPr | (1-OHMe-2-MeBu)NH |
| 287 | 1-Np | 1 | 1-NpMe | Oc | iPr | (1-OHMe-2-MeBu)NH |
| 288 | 1-Np | 1 | 1-NpMe | Nn | iPr | (1-OHMe-2-MeBu)NH |
| 289 | 1-Np | 1 | 1-NpMe | Dc | iPr | (1-OHMe-2-MeBu)NH |
| 290 | 1-Np | 1 | 1-NpMe | All | iPr | 2-MeBuNH |
| 291 | 1-Np | 1 | 1-NpMe | All | iPr | (1-OHMe-2-MeBu)NH |
| 292 | 1-Np | 1 | 1-NpMe | All | iPr | (2-MorEt)NH |
| 293 | 1-Np | 1 | 1-NpMe | Bun | iPr | (1-OHMe-2-MeBu)NH |
| 294 | 1-Np | 1 | 1-NpMe | 3-MeBun | iPr | (1-OHMe-2-MeBu)NH |
| 295 | 1-Np | 1 | 1-NpMe | Pyn | iPr | [3-di(2-OHEt)NPr]NH |
| 296 | 1-Np | 1 | 1-NpMe | Pyn | iPr | (1-OHMe-2-MeBu)NH |
| 297 | 1-Np | 1 | 1-NpMe | 1-Me-2-PyrdMe | iPr | (1-OHMe-2-MeBu)NH |
| 298 | Mor | 2 | 1-NpMe | Pn | iPr | $NH_2$ |
| 299 | Mor | 2 | 1-NpMe | Pn | iPr | (1-OHMe-2-MeBu)NH |
| 300 | 2-Pyr | 1 | 1-NpMe | Hx | iPr | (1-OHMe-2-MeBu)NH |
| 301 | 2-Quin | 1 | 1-NpMe | Hx | iPr | (1-OHMe-2-MeBu)NH |
| 302 | 2-Quin | 1 | 1-NpMe | Hp | iPr | (1-OHMe-2-MeBu)NH |
| 303 | 4-Ph-1-Piz | 1 | 1-NpMe | Oc | iPr | (1-OHMe-2-MeBu)NH |

| Cpd No. | $R^{21}$ | n | $R^2$ | $R^3$ | $R^4$ | $R^5$ |
|---|---|---|---|---|---|---|
| 304 | 4-Ph-1-Piz | 1 | 1-NpMe | Oc | iPr | [3-di(2-OHEt)NPr]NH |
| 305 | 4-Me-1-Piz | 1 | 1-NpMe | Hp | iPr | (1-OHMe-2-MeBu)NH |
| 306 | 4-Me-1-Piz | 1 | 1-NpMe | All | iPr | $NH_2$ |
| 307 | 4-Me-1-Piz | 1 | 1-NpMe | All | iPr | (1-OHMe-2-MeBu)NH |
| 308 | 2-MeOEtO | 2 | 1-NpMe | All | iPr | [3-di(2-OHEt)NPr]NH |
| 309 | Mor | 1 | 1-NpMe | All | iPr | (1-OHMe-2-MeBu)NH |
| 310 | Mor | 1 | 1-NpMe | All | iPr | $NH_2$ |
| 311 | Mor | 1 | 1-NpMe | All | iPr | [3-di(2-OHEt)NPr]NH |

Of the compounds listed above, the following compounds are preferred: Compounds Nos. 6, 17, 18, 22, 24, 36, 39, 40, 41, 42, 45, 47, 50, 53, 58, 59, 60, 71, 72, 74, 83, 88, 95, 116, 153, 158, 196, 203, 205, 208, 212, 213, 223, 235, 274, 275, 276, 277, 286, 289, 291 and 296. The most preferable compounds of the present invention are Compounds Nos. 39, 40, 50, 53, 58, 71, 72, 74, 83, 88, 95, 208, 223, 235, 275, 276, 291 and 296.

In particular, the following isomers are most preferred. Compound Nos. as assigned in the above list are shown in parentheses:

N-[4(S)-{N-[Bis(1-naphthylmethyl)acetyl]-L-leucylamino}-3(S)-hydroxy-6-methylheptanoyl]-L-isoleucinol (39);

4(S)-{N-[Bis(1-naphthylmethyl)acetyl]-L-leucylamino}-3(S)-hydroxy-6-methyl-N-(2-methylbutyl)-heptanamide (40);

4(S)-{N-[Bis(1-naphthylmethyl)acetyl]-L-leucylamino}-3(S)-hydroxy-6-methyl-N-(2-diethylaminoethyl)-heptanamide (50);

4(S)-{N-[Bis(1-naphthylmethyl)acetyl]-L-leucylamino}-3(S)-hydroxy-6-methyl-N-(2-morpholinoethyl)-heptanamide (53);

4-[4(S)-{N-[Bis(1-naphthylmethyl)acetyl]-L-leucylamino}-3(S)-hydroxy-6-methylheptanoyl]amino-1-benzylpiperidine (58);

4(S)-{N-[Bis(1-naphthylmethyl)acetyl]-L-histidylamino}-3(S)-hydroxy-6-methylheptanamide (71);

N-[4(S)-{N-[Bis(1-naphthylmethyl)acetyl]-L-histidylamino}-3(S)-hydroxy-6-methylheptanoyl]-L-isoleucinol (72);

4(S)-{N-[Bis(1-naphthylmethyl)acetyl]-L-histidylamino}-3(S)-hydroxy-6-methyl-N-[2(S)-(-)-methylbutyl]-heptanamide (74);

N-[4(S)-{N-[Bis(1-naphthylmethyl)acetyl]-L-isoleucylamino}-3(S)-hydroxy-6-methylheptanoyl]-L-isoleucinol (83);

N-[4(S)-{N-[Bis(1-naphthylmethyl)acetyl]-L-norleucylamino}-3(S)-hydroxy-6-methylheptanoyl]-L-isoleucinol (88);

N-[4(S)-{N-[Bis(1-naphthylmethyl)acetyl]-L-valyl-

amino}-3(S)-hydroxy-6-methylheptanoyl]-L-isoleucinol (95);

N-[4(S)-{N-[Bis(1-naphthylmethyl)acetyl]-L-glutaminylamino}-3(S)-hydroxy-6-methylheptanoyl]-L-isoleucinol (208);

N-[4(S)-{N-[Bis(1-naphthylmethyl)acetyl]-3-(4-thiazolyl)-DL-alanylamino}-3(S)-hydroxy-6-methyl-heptanoyl]-L-isoleucinol (223);

N-[4(S)-{N-[Bis(1-naphthylmethyl)acetyl]-3-(cyclopentyl)-DL-alanylamino}-3(S)-hydroxy-6-methyl-heptanoyl]-L-isoleucinol (235);

N-[4(S)-{2(RS)-[Bis(1-naphthylmethyl)acetyl]-aminoheptanoyl}amino-3(S)-hydroxy-6-methylheptanoyl]-L-isoleucinol (275);

4(S)-{2(RS)-[Bis(1-naphthylmethyl)acetyl]amino-heptanoyl}amino-3(S)-hydroxy-6-methyl-N-(2-morpholinoethyl)heptanamide (276);

N-[4(S)-{N-[Bis(1-naphthylmethyl)acetyl]-DL-allylglycylamino}-3(S)-hydroxy-6-methylheptanoyl]-L-isoleucinol (291);

N-[4(S)-{N-[Bis(1-naphthylmethyl)acetyl]-DL-propargylglycylamino}-3(S)-hydroxy-6-methylheptanoyl]-L-isoleucinol (296)

and pharmaceutically acceptable salts thereof.

The compounds of the invention may be prepared by:

(i) reacting a compound of formula (II):

$$R^1-(O)_m-CH(R^2)-CO-(Y)_r-OH \quad (II)$$

[in which: $R^1$, m and $R^2$ are as defined above; Y represents a group of formula $-NH-CH(R^3)CO-$, where $R^3$ is as defined above; and r is 0 or 1] or a reactive derivative thereof with a compound of formula (III):

$$H(Y)_sNH-CH(CH_2R^4)-CH(OH)-CH_2COR^5 \quad (III)$$

(in which: Y, $R^4$ and $R^5$ are as defined above; and s is 1 if r is 0 or 0 if r is 1) or a reactive derivative thereof;

(ii) optionally subjecting the product to an acyl exchange reaction; and

(iii) optionally converting any group represented by $R^5$ to any other group represented by $R^5$.

Specifically, in a first step of this reaction, a carboxylic acid compound of formula (IV):

$$R^1-(O)_m-CH(R^2)-CO-NH-CH(R^3)-COOH \quad (IV)$$

(in which $R^1$, $R^2$, $R^3$ and m are as defined above) or a reactive derivative thereof is reacted with an amino compound of formula (V):

$$H_2N-CH(CH_2R^4)-CH(OH)-CH_2-COR^5 \quad (V)$$

(in which $R^4$ and $R^5$ are as defined above) or with a reactive derivative thereof.

Alternatively, in the first step, a carboxylic acid compound of formula (VI):

$$R^1-(O)_m-CH(R^2)-COOH \quad (VI)$$

(in which $R^1$, $R^2$ and m are as defined above) or a reactive derivative thereof is reacted with an amino compound of formula (VII):

$$H_2N-CH(R^3)-CO-NH-CH(CH_2R^4)-CH(OH)-CH_2-COR^5 \quad (VII)$$

(in which $R^3$, $R^4$ and $R^5$ are as defined above) or with a reactive derivative thereof.

These two reactions are standard condensation reactions of the type conventionally used in peptide synthesis and they may be carried out according to any of the well-known techniques employed in peptide synthesis, for example by the azide method, the active ester method, the mixed acid anhydride method, the carbodiimide method or the condensation method. The reactive derivatives employed in these reactions are those reactive derivatives conventionally employed in such methods. Certain of these methods are described in more detail below.

Azide Method

First, the carboxylic acid of formula (IV) or (VI), usually in the form of its corresponding alkyl ester, is treated with hydrazine in an inert solvent (e.g. dimethylformamide) generally at about ambient temperature, to give the corresponding acid hydrazide. This hydrazide is then reacted with a nitrite, to convert it into an azide, after which the azide is reacted with the amine of formula (V) or (VII).

Examples of nitrites which may be employed include: alkali metal nitrites, such as sodium nitrite, and alkyl nitrites, such as isopentyl nitrite.

The reaction of the acid hydrazide with the nitrite and the subsequent reaction of the resulting azide with the amine of formula (V) or (VII) are commonly carried out in the same reaction solution, without intermediate isolation of the azide. Both reactions are preferably carried out in the presence of an inert solvent. The nature of the solvent is not critical, provided that it does not interfere with the reaction. Suitable solvents include, for example: amides, such as dimethylformamide or dimethylacetamide; sulphoxides, such as dimethyl sulphoxide; and pyrrolidones, such as N-methylpyrrolidone. The reaction with the nitrite is preferably effected at a relatively low temperature, e.g. from -50°C to 0°C, whilst the reaction of the azide with the amine is preferably effected at a temperature of from -10°C to +10°C. The time required for each of these reactions will vary, depending upon the nature of the reagents and the reaction temperature, but a period of from 5 minutes to 1 hour and a period of from 10 hours to 15 days will normally suffice for the reaction with the nitrite and the reaction of the azide with the amine, respectively.

Active Ester Method

In this method, the carboxylic acid of formula (IV) or (VI) is first converted to an active ester, after which this active ester is reacted with the amine of formula (V) or (VII).

Formation of the active ester is preferably effected by reacting the carboxylic acid of formula (IV) or (VI) with, for example, an N-hydroxyimide compound, such as N-hydroxysuccinimide, 1-hydroxybenzotriazole or N-hydroxy-5-norbornene-2,3-dicarboximide. The reaction to form the active ester is preferably effected in the presence of a condensing agent, such as dicyclohexylcarbodiimide or carbonyldiimidazole.

The reaction is preferably effected in the presence of an inert solvent, the nature of which is not critical, provided that it has no adverse effect upon the reaction. Suitable solvents include, for example: halogenated hydrocarbons, such as methylene chloride or chloroform; ethers, such as tetrahydrofuran; and amides, such as dimethylformamide or dimethylacetamide.

The reaction temperature may vary over a wide range, for example from -10°C to +10°C. The time required for the reaction will vary widely, depending upon the nature of the reagents and upon the reaction temperature, but a period of from 30 minutes to 10 hours will normally suffice.

Reaction of this active ester with the amine of formula (V) or (VII) may be carried out with or without intermediate isolation of the active ester. Reaction of

the active ester with the amine is preferably effected in the presence of an inert solvent, examples of which are as given for the preparation of the active ester itself. The temperature required for the reaction is not particularly critical and, for this reason, we normally prefer to carry out the reaction at about ambient temperature. The time required for the reaction will vary widely, but a period of from 30 minutes to 10 hours will normally suffice.

Mixed Acid Anhydride Method

In this method, the carboxylic acid of formula (IV) or (VI) is first converted to a mixed acid anhydride, and this is then reacted with the amine of formula (V) or (VII).

Preparation of the mixed acid anhydride is effected by reacting the acid of formula (IV) or (VI) with a suitable reagent, preferably in the presence of an inert solvent. Suitable reagents include: lower alkyl haloformates, such as ethyl chloroformate or isobutyl chloroformate; and di(lower alkyl) phosphorocyanidates, such as diethyl phosphorocyanidate. Examples of suitable inert solvents include the amides and ethers referred to in relation to the active ester method.

This reaction is preferably effected in the presence of an organic amine, such as triethylamine or N-methylmorpholine. The reaction temperature may vary over a wide range, for example from -10°C to +10°C. The period required for the reaction will also vary widely, depending upon such factors as the nature of the reagents and the reaction temperature, but a period of from 30 minutes to 5 hours will normally suffice.

Reaction of the resulting mixed acid anhydride with the amine of formula (V) or (VII) is preferably effected in the presence of an inert solvent, the nature of which is not critical, provided that it does not interfere with the reaction. Suitable solvents include the amides and ethers hereinbefore exemplified in relation to the active ester method. The reaction will take place over a wide range of temperatures, but we generally find it convenient to carry out the reaction at a temperature of from 0°C to about ambient temperature. The time required for the reaction will vary, depending upon many factors, such as the nature of the reagents and the reaction temperature, but a period of from 1 hour to 24 hours will normally suffice.

Condensation Method

In this method, the carboxylic acid of formula (IV)

or (VI) is directly reacted with the amine of formula (V) or (VII). Such a reaction is preferably effected in the presence of a condensing agent, such as dicyclohexylcarbodiimide or carbonyldiimidazole. Otherwise, the reaction conditions and solvents are similar to those already described in relation to the active ester method.

Protecting Reactive Groups

Where the reagents employed in any of the above reactions, that is to say the carboxylic acids of formula (IV) and (VI) or the amines of formula (V) or (VII) or their reactive derivatives, contain amino groups or carboxy groups which might interfere with the above reactions or undesirably participate in them, it is desirable that these groups should be protected before the reaction to form the peptide linkage and then, after that reaction, that the protected groups should be deprotected.

There is no particular limitation on the nature of the protecting group employed and such groups are well-known in peptide chemistry. For example, suitable amino-protecting groups include: carbonate residues, such as the benzyloxycarbonyl, p-methoxybenzyloxycarbonyl, t-butoxycarbonyl and 9-fluorenylmethyloxycarbonyl groups. Suitable carboxy-protecting groups include the t-butyl group and aralkyl groups, such as the benzyl

group. The protecting groups may be inserted and then removed by conventional methods. For example, where the amino-protecting group is a t-butoxycarbonyl group or the carboxy-protecting group is a t-butyl group, these groups may be removed by treatment with an acid (e.g. hydrochloric acid or trifluoroacetic acid) whilst other amino-protecting groups (for example the benzyloxycarbonyl group) and such carboxy-protecting groups as the aralkyl groups can be removed by catalytic reduction (for example in a hydrogen atmosphere at a pressure of from atmospheric to 10 atmospheres and in the presence of a suitable catalyst, such as palladium-on-carbon).

Conversion Reactions

If desired, a group represented by $R^5$ in the compound of formula (I) prepared as described above may be converted to any other group represented by $R^5$ by appropriate reactions well-known in the field of peptide synthesis.

If desired, any acyl group within the resulting compound of formula (I) may be converted to any other acyl group; the reactions and reaction conditions involved in such conversions are well known in the art.

After completion of any of the above reactions or of

the final such reaction, the desired compound may be isolated from the reaction mixture by conventional means. For example, one suitable recovery procedure comprises: if necessary, neutralizing the reaction mixture; removing any insoluble residue by filtration; and then distilling off the solvent to give the desired compound. If necessary, this compound may be further purified by such conventional means as recrystallization, reprecipitation or the various chromatography techniques, such as column chromatography or preparative thin layer chromatography.

INHIBITION OF RENIN ACTIVITY

The ability of various compounds of the invention to inhibit the activity of renin was determined according to the following method, which follows essentially the procedure of Kokubu et al. [Hypertension, 5, 191-197 (1983)].

Specifically, each test compound was dissolved in 60% v/v aqueous ethanol. Human renin activity in the presence and absence of each compound was measured using sheep angiotensinogen. The total volume of 1 ml of assay mixture contained 0.1 mole/litre phosphate buffer (pH 7.3), human renin (equivalent to 0.5 ng angiotensin I per ml per minute), sheep angiotensinogen (equivalent to 200 ng angiotensin I), 1 x $10^{-6}$M of the test

compound, 6% v/v ethanol and angiotensinase inhibitors (10 mmole/litre sodium ethylenediaminetetraacetate and 3.4 mmole/litre 8-hydroxyquinoline). The mixture was allowed to react for 10 minutes at 37°C, and then the reaction was stopped by placing the reaction tube in a boiling water bath for 5 minutes. The mixture was then centrifuged and the supernatant (0.05-0.1 ml) was used to assay remaining angiotensin I.

An identical experiment was carried out, as a control, except that the test compound was omitted. From the values obtained were calculated the % inhibition of renin activity achieved by each test compound. The results are shown in the following Table, in which the compounds of the invention are identified by the numbers assigned to them in the foregoing list. The values given are the mean of 3 or 4 experiments.

The isomers of these test compounds employed in these experiments are those isomers prepared as described in the subsequent Examples.

Table

| Compound No | % inhibition (human renin) |
|---|---|
| 15 | 93.5% |
| 45 | 99.0% |
| 72 | 99.0% |
| 74 | 98.2% |
| 88 | 99.0% |
| 196 | 93.5% |
| 208 | 93.0% |
| 212 | 92.0% |
| 223 | 95.0% |
| 274 | 99.0% |
| 286 | 99.0% |
| 291 | 99.0% |

As can be seen from the above Table, the compounds of the invention have a substantial inhibitory effect on the activity of human renin and are thus useful for the diagnosis and therapy of renin/angiotensin - induced hypertension in humans and other animals.

The route of administration may be oral or parenteral and the compound of the invention may be

formulated accordingly, normally with a pharmaceutically acceptable carrier or diluent, as, for example, a tablet, capsule, granule, powder or syrup for oral administration or as an injection or suppository for parenteral administration. The dosage will vary depending upon the age, symptoms and body weight of the patient as well as upon the desired end result, but normally we would anticipate a dose of from 0.01 mg. to 100 mg. per Kg. body weight per day, which may be administered in a single dose or in divided doses.

The invention is further illustrated by the following non-limiting Examples. In these Examples, all values of optical rotation were measured using the sodium D-line, i.e. all such values are $[\alpha]_D$. The preparation of certain intermediates is illustrated in the subsequent Preparations.

## EXAMPLE 1

### 4(S)-{N-[Bis(1-naphthylmethyl)acetyl]-L-histidylamino}-3(S)-hydroxy-6-methyl-N-[2(S)-(-)-methylbutyl]-heptanamide (Compound No. 74)

### 1(a) 4(S)-(N-Benzyloxycarbonyl-L-histidylamino)-3(S)-hydroxy-6-methyl-N-[2(S)-(-)-methylbutyl]heptanamide

10 ml of a 6N solution of hydrochloric acid in dioxane were added to 0.9 g (2.6 mmole) of (3S,4S)-4-t-butoxycarbonylamino-3-hydroxy-6-methyl-N-[2(S)-(-)-methylbutyl]heptanamide. The reaction mixture was then stirred for 20 minutes at room temperature in a nitrogen atmosphere, after which it was evaporated to dryness under reduced pressure. The residue was dissolved in 10 ml of dimethylformamide, and then 0.26 g (2.6 mmole) of N-methylmorpholine was added, with ice-cooling, to prepare a solution of (3S,4S)-4-amino-3-hydroxy-6-methyl-N-[2(S)-(-)-methylbutyl]heptanamide. This solution was added to 10 ml of a cold dimethylformamide solution of N-benzyloxycarbonyl-L- histidine azide [synthesized from 0.91 g (3 mmole) of N-benzyloxycarbonyl-L-histidine hydrazide by treatment with isopentyl nitrite according to the same procedure as the corresponding reaction described hereafter in Example 2(c)], and the mixture was stirred for 5 days at 4°C. The solvent was distilled off under reduced pressure, and then a saturated aqueous solution of potassium carbonate was added to the residue and the mixture was extracted with ethyl acetate. The ethyl acetate extract was washed with a saturated aqueous solution of sodium chloride, dried over anhydrous sodium sulphate and concentrated by evaporation under reduced pressure. The residue obtained was purified by silica gel column chromatography, eluted with a 20:1 by volume mixture of chloroform and methanol. The desired fraction was concentrated by evaporation under reduced pressure to give a syrupy residue. This syrup was solidified by the addition of diethyl ether and the precipitate was collected by filtration to give 0.96 g of the title compound as a white powder, melting at 158-168°C.

Elemental Analysis:

Calculated for $C_{27}H_{41}N_5O_5$:

C, 62.89%; H, 8.01%; N, 13.58%.

Found: C, 62.73%; H, 7.94%; N, 13.67%.

1(b) 4(S)-{N-[Bis(1-naphthylmethyl)acetyl]-L-histidylamino}-3(S)-hydroxy-6-methyl-N-[2(S)-(-)-methylbutyl]-heptanamide

The benzyloxycarbonyl group was removed from the compound produced as described in Example 1(a) by treating it with hydrogen at room temperature in the presence of 5% w/w palladium-on-activated carbon. 91 mg (0.2 mmole) of the resulting compound, 66.5 mg (0.2 mmole) of bis(1-naphthylmethyl)acetic acid and 39.4 mg (0.22 mmole) of N-hydroxy-5-norbornen-2,3-dicarboximide were dissolved in a mixed solvent consisting of 1 ml of dimethylformamide and 6 ml of methylene chloride, and the solution was ice-cooled. To the resulting solution were added 44 mg (0.4 mmole) of N-methylmorpholine and 45.4 mg (0.22 mmole) of dicyclohexylcarbodiimide, in that order, and the mixture was stirred for 1 hour whilst ice-cooling and for a further 10 days at room temperature. The dicyclohexylurea which separated was filtered off and the filtrate was condensed by evaporation under reduced pressure. A 5% w/v aqueous solution of sodium bicarbonate was added to the resulting residue. The oily substance which separated was extracted with ethyl acetate. The organic extract was washed with a saturated aqueous solution of sodium chloride, dried over anhydrous sodium sulphate and

concentrated by evaporation under reduced pressure. The residue obtained was purified by silica gel column chromatography, using a 10:1 by volume mixture of chloroform and methanol as eluent. The desired fractions were collected and concentrated by evaporation under reduced pressure. The residue was solidified by the addition of diethyl ether and the resulting solid was collected by filtration, to give 35 mg of the desired Compound No. 74 as a pale yellow powder, melting at 130-135°C.

$[\alpha]^{23}$ = -66° (C=0.1, methanol).

Elemental Analysis:

Calculated for $C_{43}H_{53}N_5O_4.H_2O$:
C, 71.54%; H, 7.68%; N, 9.70%.

Found: C, 71.78%; H, 7.61%; N, 10.00%.

EXAMPLE 2

4(S)-{N-[Bis(1-naphthylmethyl)acetyl]-L-leucylamino}-3(S)-hydroxy-6-methyl-N-ethylheptanamide (Compound No. 41)

2(a) Ethyl 4(S)-(N-t-Butoxycarbonyl-L-leucylamino)-3(S)-hydroxy-6-methylheptanoate

The t-butoxycarbonyl group was removed from 3.06 g (10.1 mmole) of ethyl (3S,4S)-4-t-butoxycarbonylamino-3-hydroxy-6-methylheptanoate by conventional means [the same as the first step in Example 1(a)]. The product was dissolved in methylene chloride and neutralized with triethylamine, whilst ice cooling. To the resulting solution were added 3.48 g (10.6 mmole) of N-t-butoxycarbonyl-L-leucine N-hydroxysuccinimide, and the mixture was stirred for 3 hours at room temperature; 1.1 ml of 3-(N,N-dimethylamino)propylamine was then added and the mixture was stirred for a further 1 hour. The reaction solution was concentrated by evaporation under reduced pressure. Ethyl acetate was added to dissolve the residue, and the ethyl acetate solution was washed with a 5% w/v aqueous solution of sodium bicarbonate, a 10% w/v aqueous solution of citric acid and a saturated aqueous solution of sodium chloride, in that order. It was then dried over anhydrous sodium sulphate. The solvent was distilled off under reduced pressure, and the resulting residue was recrystallized from a mixture of diethyl ether and hexane, to give 3.02 g of the title compound as a pale red powder, melting at 113-114°C.

Elemental Analysis:

Calculated for $C_{21}H_{40}N_2O_6$:

C, 60.55%; H, 9.68%; N, 6.73%.

Found: C, 60.36%; H, 9.70%; N, 6.66%.

2(b) 4(S)-(N-t-Butoxycarbonyl-L-leucylamino)-3(S)-hydroxy-6-methylheptanoic acid hydrazide

2.65 g (6.36 mmole) of the compound synthesized as described in Example 2(a) and 3.19 g (63.7 mmole) of hydrazine monohydrate were dissolved in 80 ml of dimethylformamide, and the mixture was stirred for 19 hours at room temperature and for a further 12.5 hours at 50°C. The reaction solution was then concentrated by evaporation under reduced pressure. Water and ethyl acetate were added to the residue. The insoluble substance which separated out was filtered off. The filtrate was washed with water and then dried over anhydrous magnesium sulphate. The solvent was distilled off under reduced pressure. The residue and the insoluble substance which had been separated by filtration were combined and suspended in boiling diethyl ether. The compound which separated was collected by filtration and dried to give 1.73 g of the title compound as colourless prisms, melting at 140-147°C.

2(c) 4(S)-(N-t-Butoxycarbonyl-L-leucylamino)-3(S)-hydroxy-6-methyl-N-ethylheptanamide

402 mg (1.00 mmole) of the compound synthesized as described in Example 2(b) were dissolved in 8 ml of dimethylformamide, and the solution was cooled to -60°C. To the cooled solution were added 0.62 ml of a 6N solution of hydrochloric acid in dioxane and 0.18 ml (1.35 mmole) of isopentyl nitrite, and the resulting solution was stirred for 10 minutes at -20°C and then again cooled to -60°C. The solution was then neutralized by adding 0.61 g of N-methylmorpholine, after which 108 mg (1.32 mmole) of ethylamine hydrochloride were added, and the reaction mixture was stirred for 20.5 hours at 5°C. The reaction mixture was then poured into water and the mixture was extracted with ethyl acetate. The ethyl acetate phase was washed with a 5% w/v aqueous solution of sodium bicarbonate, a 10% w/v aqueous solution of citric acid and a saturated aqueous solution of sodium chloride, in that order, and then dried over anhydrous magnesium sulphate. The solvent was distilled off under reduced pressure. The residue was washed with hexane, collected by filtration and dried to give 345 mg of the title compound as a white powder, melting at 193-197°C.

Elemental Analysis:

Calculated for $C_{21}H_{41}N_3O_5$:

C, 60.69%; H, 9.94%; N, 10.11%.

Found: C, 60.46%; H, 10.04%; N, 10.13%.

2(d) 4(S)-{N-[Bis(1-naphthylmethyl)acetyl]-L-leucylamino}-3(S)-hydroxy-6-methyl-N-ethylheptanamide

The t-butoxycarbonyl group was removed from 99 mg (0.24 mmole) of the compound synthesized as described in Example 2(c) by conventional means. The resulting product was dissolved in dimethylformamide. To the resulting solution were added 81 mg (0.24 mmole) of bis(1-naphthylmethyl)acetic acid, 50 mg (0.28 mmole) of 90% diethyl phosphorocyanidate and 64 mg (0.63 mmole) of triethylamine, whilst ice cooling, and the mixture was stirred for 14.5 hours at room temperature. The reaction solution was subsequently treated as described in Example 1(b), and the product obtained was recrystallized from a mixed solvent consisting of methylene chloride and hexane, to afford 59 mg of the title compound as colourless needles, melting at 93-96°C.

$[\alpha]^{23}$ = -157° (C=0.1, methanol).

Elemental Analysis:

Calculated for $C_{40}H_{51}N_3O_4.1/2H_2O$:

C, 75.32%; H, 8.06%; N, 6.59%.

Found: C, 75.06%; H, 8.08%; N, 6.60%.

EXAMPLE 3

4(S)-{N-[Bis(1-naphthylmethyl)acetyl]-L-leucylamino}-3(S)-hydroxy-6-methyl-N-(2-methylbutyl)heptanamide (Compound No. 40)

3(a) 4(S)-(N-t-Butoxycarbonyl-L-leucylamino)-3(S)-hydroxy-6-methyl-N-(2-methylbutyl)heptanamide

344 mg (1.23 mmole) of (3S,4S)-4-amino-3-hydroxy-6-methyl-N-(2-methylbutyl)heptanamide monohydrochloride were dissolved in methylene chloride. The solution was neutralized with triethylamine, whilst ice-cooling, and then 420 mg (1.28 mmole) of N-t-butoxycarbonyl-L-leucine N-hydroxysuccinimide were added thereto. The mixture was stirred for 22.5 hours at room temperature, and then 0.2 ml of 3-(N,N-dimethylamino)propylamine were added and the mixture was stirred for a further 1 hour. The reaction solution was then concentrated by evaporation under reduced pressure. Methylene chloride was added to the residue, and the solution was washed with a 5% w/v aqueous solution of sodium bicarbonate, a 10% w/v

aqueous solution of citric acid and a saturated aqueous solution of sodium chloride, in that order, and then dried over anhydrous magnesium sulphate. The solvent was distilled off under reduced pressure. The residue obtained was washed with ethyl acetate, collected by filtration and dried to give 328 mg of the title compound as a white powder, melting at 166-168°C.

Elemental Analysis:

Calculated for $C_{24}H_{47}N_3O_5$:

C, 62.99%; H, 10.35%; N, 9.18%.

Found: C, 62.92%; H, 10.16%; N, 9.14%.

<u>3(b) 4(S)-{N-[Bis(1-naphthylmethyl)acetyl]-L-leucyl-amino}-3(S)-hydroxy-6-methyl-N-(2-methylbutyl)heptan-amide</u>

The t-butoxycarbonyl group was removed from 100 mg (0.22 mmole) of 4(<u>S</u>)-(<u>N</u>-t-butoxycarbonyl-<u>L</u>-leucyl-amino)-3(<u>S</u>)-hydroxy-6-methyl-<u>N</u>-(2-methylbutyl)heptanamide [prepared as described in Example 3(a)] by conventional means, and then the product obtained was dissolved in dimethylformamide. To this solution were added 77 mg (0.23 mmole) of bis(1-naphthylmethyl)acetic acid, 42 mg (0.23 mmole) of 90% diethyl phosphorocyanidate and 52 mg (0.54 mmole) of triethylamine, whilst ice-cooling, and the mixture was stirred for 4 hours at room

temperature. The reaction solution was then concentrated by evaporation under reduced pressure. Ethyl acetate was added to the residue and the solution was washed with a 5% w/v aqueous solution of sodium bicarbonate, 1N hydrochloric acid and a saturated aqueous solution of sodium chloride, in that order, after which it was dried over anhydrous magnesium sulphate. The solvent was distilled off under reduced pressure, and the residue obtained was recrystallized from a mixed solvent consisting of methylene chloride and hexane, to afford 40 mg of the title compound as a white powder, melting at 98-100°C.

$[\alpha]^{23}$ = -143° (C=0.1, methanol).

Elemental Analysis:

Calculated for $C_{43}H_{57}N_3O_4.1/2H_2O$:
C, 74.96%; H, 8.49%; N, 6.10%.

Found: C, 75.10%; H, 8.56%; N, 6.10%.

## EXAMPLE 4

4(S)-{N-[Bis(1-naphthylmethyl)acetyl]-L-leucylamino}-3(S)-hydroxy-6-methyl-N-(2-ethoxyethyl)heptanamide (Compound No 45)

4(a) 4(S)-(N-t-Butoxycarbonyl-L-leucylamino)-3(S)-hydroxy-6-methyl-N-(2-ethoxyethyl)heptanamide

The t-butoxycarbonyl group was removed from 539 mg (1.56 mmole) of (3S,4S)-4-t-butoxycarbonylamino-3-hydroxy-6-methyl-N-(2-ethoxyethyl)heptanamide by conventional means, and then the product obtained was dissolved in methylene chloride, whilst ice-cooling, and the resulting solution was neutralized with triethylamine. To this solution were added 534 mg (1.63 mmole) of N-t-butoxycarbonyl-L- leucine N-hydroxysuccinimide, and the reaction mixture was stirred for 22 hours at room temperature. 0.2 ml of 3-(N,N-dimethylamino)propylamine was then added, and the mixture was stirred for a further 1 hour. The reaction solution was then concentrated by evaporation under reduced pressure. Methylene chloride was added to the residue, and the solution obtained was washed with a 5% w/v aqueous solution of sodium bicarbonate, a 10% w/v aqueous solution of citric acid and a saturated aqueous solution of sodium chloride, in that order, and was then dried over anhydrous magnesium sulphate. The solvent was distilled off under reduced pressure. The residue obtained was recrystallized from a mixed solvent consisting of methylene chloride and ethyl acetate, to afford 294 mg of the title compound as colourless needles, melting at 172-173°C.

Elemental Analysis:

Calculated for $C_{23}H_{45}N_3O_6$:

C, 60.10%; H, 9.87%; N, 9.14%.

Found: C, 59.99%; H, 9.96%; N, 9.09%.

4(b) 4(S)-{N-[Bis(1-naphthylmethyl)acetyl]-L-leucylamino}-3(S)-hydroxy-6-methyl-N-(2-ethoxyethyl)heptanamide

The t-butoxycarbonyl group was removed from 105 mg (0.23 mmole) of 4(S)-(N-t-butoxycarbonyl-L-leucylamino)-3(S)-hydroxy-6-methyl-N-(2-ethoxyethyl)heptanamide by conventional means, and then the product obtained was dissolved in dimethylformamide. To this solution were added 78 mg (0.23 mmole) of bis(1-naphthylmethyl)acetic acid, 43 mg (0.24 mmole) of 90% diethyl phosphorocyanidate and 50 mg (0.49 mmole) of triethylamine, whilst ice-cooling, and the mixture was stirred for 5.5 hours at room temperature. The reaction solution was subsequently treated as described in Example 3(b), and the product obtained was recrystallized from a mixed solvent consisting of methylene chloride and diethyl ether, to afford 58 mg of the title compound as colourless needles, melting at 168-169°C.

$[\alpha]^{23}$ = -168° (C=0.1, methanol).

Elemental Analysis:

Calculated for $C_{42}H_{55}N_3O_5$:

C, 73.98%; H, 8.13%; N, 6.16%.

Found: C, 73.85%; H, 8.24%; N, 6.14%.

## EXAMPLE 5

4(S)-{N-[Bis(1-naphthylmethyl)acetyl]-L-leucylamino}-3(S)-hydroxy-6-methyl-N-(3,4-dimethoxyphenethyl)heptanamide (Compound No. 52)

5(a) 4(S)-(N-t-Butoxycarbonyl-L-leucylamino)-3(S)-hydroxy-6-methyl-N-(3,4-dimethoxyphenethyl)heptanamide

0.74 g (1.7 mmole) of (3S,4S)-4-t-butoxycarbonylamino-3-hydroxy-6-methyl-N-(3,4-dimethoxyphenethyl)-heptanamide was dissolved in 8 ml of a 6N solution of hydrochloric acid in dioxane, and the reaction mixture was stirred for 20 minutes at room temperature and then evaporated to dryness under reduced pressure. The residue was dissolved in 20 ml of methylene chloride and, whilst ice-cooling, the resulting solution was neutralized by the addition of 0.24 ml of triethylamine. 0.58 g (1.8 mmole) of N-t-butoxycarbonyl-L-leucine N-hydroxysuccinimide dissolved in 5 ml of methylene chloride was then added. The reaction mixture was stirred for 20 hours at room temperature, after

which 0.2 ml of 3-(N,N-dimethylamino)propylamine was added to the mixture, and the reaction mixture was stirred for a further 1 hour. The reaction solution was concentrated by evaporation under reduced pressure, and then water and ethyl acetate were added to the resulting residue. The precipitate which separated was filtered off and the organic layer was collected. The organic layer was washed with a 10% w/v aqueous solution of citric acid, a 5% w/v aqueous solution of sodium bicarbonate and a saturated aqueous solution of sodium chloride, in that order, and then dried over anhydrous magnesium sulphate. The solvent was then distilled off under reduced pressure. The residue and the precipitate filtered off as described above were combined and recrystallized from a mixed solvent consisting of methylene chloride and ethyl acetate, to afford 0.69 g of the title compound as colourless needles, melting at 192-193°.

Elemental Analysis:

Calculated for $C_{29}H_{49}N_3O_7$:
C, 63.13%; H, 8.95%; N, 7.62%.

Found: C, 63.06%; H, 8.83%; N, 7.56%.

5(b) 4(S)-{N-[Bis(1-naphthylmethyl)acetyl]-L-leucyl-amino}-3(S)-hydroxy-6-methyl-N-(3,4-dimethoxyphenethyl)-heptanamide

153 mg (0.28 mmole) of 4(S)-(N-t-butoxycarbonyl-L-leucylamino)-3(S)-hydroxy-6-methyl-N-(3,4-dimethoxyphenethyl)heptanamide were dissolved in 1.4 ml of a 6N solution of hydrochloric acid in dioxane, and the reaction solution was stirred for 20 minutes at room temperature and then evaporated to dryness under reduced pressure. The residue was dissolved in 3 ml of dimethylformamide and stirred whilst ice-cooling. To this solution were added 93 mg (0.27 mmole) of bis(1-naphthylmethyl)acetic acid, 49 mg (0.27 mmole) of 90% diethyl phosphorocyanidate and 59 mg (0.58 mmole) of triethylamine, and the reaction mixture was stirred for 1 hour whilst ice-cooling and then stirred for a further 3 hours at room temperature. The reaction solution was then concentrated by evaporation under reduced pressure. Ethyl acetate was added to the residue, and the resulting solution was washed with a 5% w/v aqueous solution of sodium bicarbonate, 1N hydrochloric acid and a saturated aqueous solution of sodium chloride, in that order, and dried over anhydrous magnesium sulphate. The solvent was distilled off under reduced pressure. The residue was washed with hexane and was then recrystallized from a mixed solvent consisting of methylene chloride and diethyl ether, to afford 128 mg of the title compound as a white powder, melting at 164-166°C.

$[\alpha]^{23}$ = -104° (C=0.1, methanol).

Elemental Analysis:

Calculated for $C_{48}H_{59}N_3O_6$:

C, 74.49%; H, 7.68%; N, 5.43%.

Found: C, 74.32%; H, 7.63%; N, 5.44%.

EXAMPLE 6

4(S)-{N-[Bis(1-naphthylmethyl)acetyl]-L-leucylamino}-3(S)-hydroxy-6-methyl-N-(2-diethylaminoethyl)heptanamide (Compound No. 50)

6(a) 4(S)-(N-t-Butoxycarbonyl-L-leucylamino)-3(S)-hydroxy-6-methyl-N-(2-diethylaminoethyl)heptanamide

The t-butoxycarbonyl group was removed from 275 mg (0.74 mmole) of (3S,4S)-4-t-butoxycarbonylamino-3-hydroxy-6-methyl-N-(2-diethylaminoethyl)heptanamide by conventional means, and then the product was dissolved in a mixed solvent consisting of dimethylformamide and methylene chloride and neutralized with triethylamine, whilst ice-cooling. To this solution were added 253 mg (0.77 mmole) of N-t-butoxycarbonyl-L-leucine N-hydroxysuccinimide, and the mixture was stirred for 20 hours at room temperature, after which 0.2 ml of 3-(N,N-dimethylamino)propylamine was added and the

mixture was stirred for a further 1 hour. The reaction solution was then concentrated by evaporation under reduced pressure. Methylene chloride was added to the residue, and the solution obtained was washed with a 5% w/v aqueous solution of sodium bicarbonate and a saturated aqueous solution of sodium chloride, in that order, and dried over anhydrous magnesium sulphate. The solvent was distilled off under reduced pressure. The residue was washed with ethyl acetate, collected by filtration and dried to give 231 mg of the title compound as a white powder, melting at 207-211°C.

Elemental Analysis:

Calculated for $C_{25}H_{50}N_4O_5$:
C, 61.70%; H, 10.36%; N, 11.51%.

Found: C, 61.67%; H, 10.25%; N, 11.55%.

<u>6(b) 4(S)-{N-[Bis(1-naphthylmethyl)acetyl]-L-leucyl-amino}-3(S)-hydroxy-6-methyl-N-(2-diethylaminoethyl)-heptanamide</u>

The t-butoxycarbonyl group was removed from 100 mg (0.21 mmole) of 4(<u>S</u>)-(<u>N</u>-t-butoxycarbonyl-<u>L</u>-leucyl-amino)-3(<u>S</u>)-hydroxy-6-methyl-<u>N</u>-(2-diethylaminoethyl)-heptanamide by conventional means, and then the product was dissolved in dimethylformamide. 70 mg (0.21 mmole) of bis(1-naphthylmethyl)acetic acid, 37 mg (0.20 mmole)

of 90% diethyl phosphorocyanidate and 69 mg (0.68 mmole) of triethylamine were added to this solution, and the mixture was stirred for 3 hours at room temperature. The reaction solution was then concentrated by evaporation under reduced pressure. Ethyl acetate was added to the residue, and the resulting solution was washed with a 5% w/v aqueous solution of sodium bicarbonate and a saturated aqueous solution of sodium chloride, in that order, and dried over anhydrous magnesium sulphate. The solvent was distilled off under reduced pressure and the residue obtained was purified by preparative thin layer chromatography (developing solvent, a 5:1 by volume mixture of chloroform and methanol), to give 23 mg of the title compound as a pale orange powder, melting at 122-128°C.

$[\alpha]^{23}$ = -162° (C=0.1, methanol).

Elemental Analysis:

Calculated for $C_{44}H_{60}N_4O_4.2H_2O$:
C, 70.94%; H, 8.66%; N, 7.52%.

Found: C, 70.95%; H, 8.29%; N, 7.47%.

## EXAMPLE 7

4-[4(S)-{N-[Bis(1-naphthylmethyl)acetyl]-L-leucyl-amino}-3(S)-hydroxy-6-methylheptanoyl]amino-1-benzyl-piperidine (Compound No. 58)

7(a) 4-[(3S,4S)-4-t-Butoxycarbonylamino-3-hydroxy-6-methylheptanoyl]amino-1-benzylpiperidine

348 mg (1.83 mmole) of 4-amino-1-benzylpiperidine, 510 mg (1.85 mmole) of (3S,4S)-4-t-butoxycarbonylamino-3-hydroxy-6-methylheptanoic acid and 358 mg (2.2 mmole) of diethyl phosphorocyanidate were dissolved in 2 ml of dimethylformamide. To this solution were added dropwise 223 mg (2.2 mmole) of triethylamine, whilst ice-cooling, and the mixture was stirred for 30 minutes at 0°C, and then overnight at room temperature. Ethyl acetate was added to the reaction solution, which was then washed with 10% v/v aqueous hydrochloric acid, a saturated aqueous solution of sodium bicarbonate and a saturated aqueous solution of sodium chloride, in that order, dried over anhydrous sodium sulphate and condensed by evaporation under reduced pressure. The residue obtained was purified by silica gel column chromatography (eluted with a 20:1 by volume mixture of chloroform and methanol) to give 663 mg of the title compound as a slightly yellow, glassy substance.

7(b) 4-[4(S)-(N-t-Butoxycarbonyl-L-leucylamino)-3(S)-hydroxy-6-methylheptanoyl]amino-1-benzylpiperidine

370 mg (1.60 mmole) of N-t-butoxycarbonyl-L-leucine, 620 mg (1.47 mmole) of 4-[(3S,4S)-4-amino-3-hydroxy-6-

methylheptanoyl]amino-1-benzylpiperidine dihydrochloride [prepared by removing, by conventional means, the t-butoxycarbonyl group from the compound prepared as described in Example 7(a)] and 313 mg (1.92 mmole) of diethyl phosphorocyanidate were dissolved in 2 ml of dimethylformamide. To this solution were added dropwise 388 mg (3.83 mmole) of triethylamine, whilst ice-cooling, and the mixture was stirred for 30 minutes at 0°C, and then overnight at room temperature. Ethyl acetate was added to the reaction solution, which was then washed with 10% v/v aqueous hydrochloric acid, a saturated aqueous solution of sodium bicarbonate and a saturated aqueous solution of sodium chloride, in that order, dried over anhydrous sodium sulphate and condensed by evaporation under reduced pressure. The residue obtained was mixed with a solvent (a 1:1 by volume mixture of ethyl acetate and diethyl ether) and pulverized to give 680 mg of the title compound as colourless crystals, melting at 186-187°C.

Elemental Analysis:

Calculated for $C_{31}H_{52}N_4O_5$:

C, 66.40%; H, 9.35%; N, 9.99%.

Found: C, 66.33%; H, 9.38%; N, 10.04%.

7(c) 4-[4(S)-{N-[Bis(1-naphthylmethyl)acetyl]-L-leucylamino}-3(S)-hydroxy-6-methylheptanoyl]amino-1-benzylpiperidine

213 mg (0.4 mmole) of 4-[4(S)-L-leucylamino-3(S)-hydroxy-6-methylheptanoyl]amino-1-benzylpiperidine dihydrochloride [prepared by removing, by conventional means, the t-butoxycarbonyl group from the compound prepared as described in Example 7(b)], 136 mg (0.4 mmole) of bis(1-naphthylmethyl)acetic acid and 78 mg (0.48 mmole) of diethyl phosphorocyanidate were dissolved in 2 ml of dimethylformamide. To this solution were added dropwise 146 mg (1.44 mmole) of triethylamine, whilst ice-cooling, and the mixture was stirred for 30 minutes at 0°C, and then overnight at room temperature. The reaction solution was mixed with ethyl acetate, washed with 10% v/v aqueous hydrochloric acid and a saturated aqueous solution of sodium chloride, in that order, dried over anhydrous sodium sulphate and condensed by evaporation under reduced pressure. The residue obtained was mixed with ethyl acetate and pulverized to give 288 mg of the title compound as colourless crystals, melting at 183-184°C.

$[\alpha]^{23}$ = -105° (C=0.1, methanol).

## EXAMPLE 8

N-[4(S)-{N-[Bis(1-naphthylmethyl)acetyl-L-norleucyl-amino}-3(S)-hydroxy-6-methylheptanoyl]-L-isoleucinol (Compound No. 88)

8(a) N-[4(S)-(N-t-Butoxycarbonyl-L-norleucylamino)-3(S)-hydroxy-6-methylheptanoyl]-L-isoleucinol

375 mg (1 mmole) of N-[4(S)-(N-t-butoxycarbonylamino)-3(S)-hydroxy-6-methylheptanoyl]-L-isoleucinol were treated with a 6N solution of hydrochloric acid in dioxane to remove the t-butoxycarbonyl group and give (3S,4S)-4-amino-3-hydroxy-6-methylheptanoyl-L-isoleucinol hydrochloride. 231 mg (1 mmole) of N-t-butoxycarbonyl-L-norleucine and 135 mg (1 mmole) of 1-hydroxybenzotriazole were added to this compound and the mixture was dissolved in 5 ml of dimethylformamide. Whilst ice-cooling, 101 mg (1 mmole) of triethylamine and 206 mg (1 mmole) of dicyclohexylcarbodiimide were added, and the reaction mixture was stirred for 1 hour at 0°C and then overnight at room temperature. Water was added, the reaction mixture was extracted with diethyl ether and the insoluble residue was filtered off. The organic extract was washed with a 10% w/v aqueous solution of citric acid, a 5% w/v aqueous solution of sodium bicarbonate and a saturated aqueous

solution of sodium chloride, in that order, and then dried over anhydrous sodium sulphate. The solution was concentrated by evaporation under reduced pressure and then reprecipitated by the addition of a mixture of diethyl ether and hexane, to afford 330 mg of the title compound as a white powder, melting at 139-144°C.

8(b) N-[4(S)-{N-[Bis(1-naphthylmethyl)acetyl]-L-norleucylamino}-3(S)-hydroxy-6-methylheptanoyl]-L-isoleucinol

163 mg (0.33 mmole) of N-[4(S)-(N-t-butoxycarbonyl-L-norleucylamino)-3(S)-hydroxy-6-methylheptanoyl]-L-isoleucinol were treated in a similar manner to that described in Example 3(b), to obtain 110 mg of the title compound as a white powder, melting at 135-138°C.

$[\alpha]^{23} = -124°$ (C=0.1, methanol).

Elemental Analysis:

Calculated for $C_{44}H_{59}N_3O_5.1/3H_2O$:

C, 73.81%; H, 8.40%; N, 5.87%.

Found: C, 73.92%; H, 8.36%; N, 5.94%.

## EXAMPLE 9

N-[4(S)-{N-[Bis(1-naphthylmethyl)acetyl]-L-histidyl-amino}-3(S)-hydroxy-6-methylheptanoyl]-L-isoleucinol (Compound No. 72)

749 mg (2 mmole) of N-[4(S)-(N-t-butoxycarbonylamino)-3(S)-hydroxy-6-methylheptanoyl]-L-isoleucinol were treated with a 6N solution of hydrochloric acid in dioxane, as is conventional, to remove the t-butoxycarbonyl group and obtain N-[(3S,4S)-4-amino-3-hydroxy-6-methylheptanoyl]-L-isoleucinol hydrochloride. 819 mg (2 mmole) of N-t-butoxycarbonyl-$N^{im}$-tosyl-L-histidine and 359 mg (2.2 mmole) of diethyl phosphorocyanidate were added to this compound, and the whole mixture was dissolved in 10 ml of dimethylformamide. Whilst ice-cooling, 425 mg (4.2 mmole) of triethylamine were added dropwise to the reaction mixture, which was then stirred for 1 hour at 0°C and then overnight at room temperature. This reaction mixture was then concentrated by evaporation under reduced pressure and the residue was mixed with ethyl acetate. The ethyl acetate solution was washed with a 10% w/v aqueous solution of citric acid, a 5% w/v aqueous solution of sodium bicarbonate and a saturated aqueous solution of sodium chloride, in that order, dried over anhydrous sodium sulphate and concentrated by

evaporation under reduced pressure. The oily residue was treated with trifluoracetic acid in the presence of anisole, to remove the t-butoxycarbonyl group and obtain 4(S)-($N^{im}$-tosyl-L-histidylamino)-3(S)-hydroxy-6-methyl-heptanoyl-L-isoleucinol trifluoracetate.

120 mg (0.35 mmole) of bis(1-naphthylmethyl)acetic acid and 60 mg (0.35 mmole) of diethyl phosphorocyanidate were added to this compound, and the whole mixture was dissolved in 2.5 ml of dimethylformamide. 70 mg (0.67 mmole) of triethylamine were added dropwise, whilst ice-cooling, to the reaction mixture, which was then stirred for 1 hour at 0°C. The reaction mixture was then concentrated by evaporation under reduced pressure and the residue was mixed with ethyl acetate. The ethyl acetate solution was washed with a 10% w/v aqueous solution of citric acid, a 5% w/v aqueous solution of sodium bicarbonate and a saturated aqueous solution of sodium chloride, in that order, dried over anhydrous sodium sulphate and concentrated by evaporation under reduced pressure. To the residue were added 170 mg (1.28 mmole) of 1-hydroxybenzotriazole, and the mixture was dissolved in 5 ml of tetrahydrofuran. This reaction mixture was then stirred at room temperature overnight, after which it was concentrated by evaporation under reduced pressure. The residue was mixed with ethyl acetate. The ethyl acetate solution

was washed with a 5% w/v aqueous solution of sodium bicarbonate and a saturated aqueous solution of sodium chloride, in that order, dried over anhydrous sodium sulphate and concentrated by evaporation under reduced pressure. The residue was purified by silica gel thin layer chromatography (using an 8:1 by volume mixture of chloroform and methanol as developing solvent), to give 38.8 mg of the title compound as white crystals, melting at 175-179°C.

$[\alpha]^{23}$ = -70° (C=0.1, methanol).

Elemental Analysis:

Calculated for $C_{44}H_{55}N_5O_5 \cdot 1.5H_2O$:

C, 69.45%; H, 7.68%; N, 9.20%.

Found: C, 69.34%; H, 7.38%; N, 8.78%.

## EXAMPLE 10

N-[4(S)-{N-[2-(1-Naphthyl)methyl-6-phenylhexanoyl]-L-norleucylamino}-3(S)-hydroxy-6-methylheptanoyl]-L-isoleucinol (Compound No. 22)

174 mg (0.41 mmole) of N-[4(S)-(L-norleucylamino)-3(S)-hydroxy-6-methylheptanoyl]-L-isoleucinol hydrochloride, 133 mg (0.4 mmole) of 2-(1-naphthyl)-

methyl-6-phenylhexanoic acid and 80 mg (0.49 mmole) of diethyl phosphorocyanidate were dissolved in 2 ml of dimethylformamide. To this solution were added dropwise 100 mg (0.99 mmole) of triethylamine, whilst ice-cooling, and the mixture was stirred at 0°C for 30 minutes, and then at room temperature overnight. Ethyl acetate was added to this reaction mixture, which was then washed with a 10% v/v aqueous hydrochloric acid solution, a saturated aqueous solution of sodium bicarbonate and a saturated aqueous solution of sodium chloride, in that order, dried over anhydrous sodium sulphate and concentrated by evaporation under reduced pressure. The residue was purified by silica gel thin layer chromatography (developing solvent, chloroform:methanol = 10:1 by volume) to give 134 mg of the title compound as colourless crystals, melting at 154-159°C.

$[\alpha]^{23}$ = -53.7° (C=0.3, methanol).

Elemental Analysis:

Calculated for $C_{43}H_{63}N_3O_5 . 1/2H_2O$:

C, 72.64%; H, 9.07%; N, 5.91%.

Found: C, 72.86%; H, 8.95%; N, 6.05%.

## EXAMPLE 11

### N-[4(S)-{N-[Bis(1-naphthylmethyl)acetyl]-L-valyl-amino}-3(S)-hydroxy-6-methylheptanoyl]-L-isoleucinol (Compound No. 95)

### 11(a) N-[4(S)-(N-t-Butoxycarbonyl-L-valylamino)-3(S)-hydroxy-6-methylheptanoyl]-L-isoleucinol

622 mg (2 mmole) of N-[(3S,4S)-4-amino-3-hydroxy-6-methylheptanoyl]-L-isoleucinol hydrochloride, 521 mg (2.4 mmole) of t-butoxycarbonyl-L-valine and 391 mg (2.4 mmole) of diethyl phosphorocyanidate were dissolved in 10 ml of dimethylformamide. Whilst ice-cooling, 486 mg (4.8 mmole) of triethylamine were added dropwise, and then the reaction mixture was stirred for 30 minutes at 0°C and then for two days at room temperature. The reaction mixture was then mixed with ethyl acetate, and the ethyl acetate solution was washed with 10% v/v aqueous hydrochloric acid, a saturated aqueous solution of sodium bicarbonate and a saturated aqueous solution of sodium chloride, in that order. The ethyl acetate solution was then dried over anhydrous sodium sulphate and concentrated by evaporation under reduced pressure. The residue was mixed with ethyl acetate and pulverized, yielding 470 mg of the title compound as colourless crystals.

11(b) N-[4(S)-{N-[Bis(1-naphthylmethyl)acetyl]-L-valylamino}-3(S)-hydroxy-6-methylheptanoyl]-L-isoleucinol

173 mg (0.42 mmole) of N-[4(S)-L-valylamino-3(S)-hydroxy-6-methylheptanoyl]-L-isoleucinol hydrochloride, 144 mg (0.42 mmole) of bis(1-naphthylmethyl)acetic acid and 82 mg (0.5 mmole) of diethyl phosphorocyanidate were dissolved in 2 ml of dimethylformamide. To this solution were added dropwise 102 mg (1.0 mmole) of triethylamine, whilst ice-cooling, and the mixture was stirred at 0°C for 30 minutes, and then at room temperature overnight. Ethyl acetate was then added to the mixture, after which it was washed with 10% v/v aqueous hydrochloric acid, a saturated aqueous solution of sodium bicarbonate and a saturated aqueous solution of sodium chloride, in that order, dried over anhydrous sodium sulphate and concentrated by evaporation under reduced pressure. The residue was mixed with ethyl acetate and pulverized, yielding 285 mg of the title compound as colourless crystals, melting at 194-196°C.

$[\alpha]^{23}$ = -96.7° (C=0.3, methanol).

Elemental Analysis:

Calculated for $C_{43}H_{57}N_3O_5.1/2H_2O$:

C, 73.26%; H, 8.29%; N, 5.96%.

Found: C, 73.53%; H, 8.22%; N, 5.93%.

EXAMPLE 12

N-[4(S)-{N-[Bis(1-naphthylmethyl)acetyl]-L-methionyl-amino}-3(S)-hydroxy-6-methylheptanoyl]-L-isoleucinol (Compound No. 196)

12(a) N-{4(S)-[N-t-Butoxycarbonyl-L-methionylamino]-3(S)-hydroxy-6-methylheptanoyl}-L-isoleucinol

2 ml of a 6N solution of hydrochloric acid in dioxane were added to 749 mg (2 mmole) of N-{4(S)-[$N^{\alpha}$-t-butoxycarbonylamino]-3(S)-hydroxy-6-methyl-heptanoyl}-L-isoleucinol. After the reaction solution had been stirred for 2 hours at room temperature, it was concentrated by evaporation under reduced pressure. 500 mg (2 mmole) of t-butoxycarbonylmethionine and 270 mg (2 mmole) of 1-hydroxybenzotriazole were then added to the residue. The resulting reaction mixture was dissolved in 30 ml of dimethylformamide, and then 412 mg (2 mmole) of dicyclohexylcarbodiimide were added, and the mixture was stirred for 3 hours at 40°C. At the end of this time, the reaction mixture was poured into ice-water and

extracted with ethyl acetate. The insoluble residue was filtered off. The ethyl acetate extract was washed with a 10% w/v aqueous solution of citric acid, a 10% w/v aqueous solution of sodium bicarbonate and a saturated aqueous solution of sodium chloride, in that order, and then dried over anhydrous sodium sulphate. The solution was concentrated by evaporation under reduced pressure, and then the residue was reprecipitated by the addition of a mixture of chloroform and hexane, to afford 600 mg of the title compound as a white powder.

12(b) N-[4(S)-{N-[Bis(1-naphthylmethyl)acetyl]-L-methionylamino}-3(S)-hydroxy-6-methylheptanoyl]-L-isoleucinol

2 ml of a 6N solution of hydrochloric acid in dioxane were added to 600 mg (1.19 mmole) of the isoleucinol compound obtained as described in Example 12(a). The mixture was stirred for 2 hours at room temperature, and then the resulting solution was concentrated by evaporation under reduced pressure. 403 mg (1.19 mmole) of bis(1-naphthylmethyl)acetic acid were added to the residue, and the resulting mixture was dissolved in 20 ml of dimethylformamide. To this were added, whilst ice-cooling, 300 mg (1.84 mmole) of diethyl phosphorocyanidate and 2 ml of triethylamine, with stirring. The mixture was allowed to react for 1

hour at 40°C, and then poured into ice-water and extracted with ethyl acetate. The ethyl acetate solution was washed with a 10% w/v aqueous solution of citric acid, a 10% w/v aqueous solution of sodium bicarbonate and a saturated aqueous solution of sodium chloride, in that order. The extract was then purified by preparative thin layer chromatography (using a 10:1 by volume mixture of chloroform and methanol as developing solvent), and reprecipitation of the active fraction by the addition of a mixture of chloroform and hexane gave 520 mg of the title compound as a white powder, melting at 76-80°C.

$[\alpha]^{25}$ = -133.0° (C=0.1, methanol).

EXAMPLE 13

N-{4(S)-[2(S)-{N-[Bis(1-naphthylmethyl)acetyl]-amino}-4-(methylsulphinyl)butyrylamino]-3(S)-hydroxy-6-methylheptanoyl}-L-isoleucinol (Compound No. 198)

500 mg of a 35% v/v aqueous solution of hydrogen peroxide were added to 100 mg (0.14 mmole) of the thioether compound (Compound No. 196), obtained as described in Example 12, dissolved in 5 ml of methanol. The reaction mixture was then stirred for 4 hours at room temperature. At the end of this time, the reaction mixture was poured into ice-water and extracted with

ethyl acetate. The ethyl acetate solution was washed with a 10% w/v aqueous solution of sodium bicarbonate and a saturated aqueous solution of sodium chloride, in that order. The extract was then purified by preparative thin layer chromatography (using a 10:1 by volume mixture of chloroform and methanol as the developing solvent), and reprecipitation of the active fraction by the addition of a mixture of chloroform and hexane gave 88 mg of the title compound as a white powder, melting at 85-95°C.

$[\alpha]^{25}$ = -87.0° (C=0.1 methanol).

EXAMPLE 14

N-[4(S)-{$N^{\alpha}$-[Bis(1-naphthylmethyl)acetyl]-$N^{\varepsilon}$-t-butoxycarbonyl-L-lysylamino}-3(S)-hydroxy-6-methylheptanoyl]-L-isoleucinol (Compound No. 203)

14(a) N-[4(S)-($N^{\alpha}$-Benzyloxycarbonyl-$N^{\varepsilon}$-t-butoxycarbonyl-L-lysylamino)-3(S)-hydroxy-6-methylheptanoyl]-L-isoleucinol

486 mg (4.8 mmole) of triethylamine were added dropwise to a solution of 761 mg (2 mmole) of $\underline{N}^{\alpha}$-benzyloxycarbonyl-$\underline{N}^{\varepsilon}$-t-butoxycarbonyl-$\underline{L}$-lysine, 622 mg (2 mmole) of $\underline{N}$-[(3$\underline{S}$,4$\underline{S}$)-4-amino-3-

hydroxy-6-methylheptanoyl]-L-isoleucinol and 391 mg (2.4 mmole) of diethyl phosphorocyanidate in 4 ml of dimethylformamide, whilst ice-cooling. The reaction mixture was stirred for 30 minutes at 0°C and then overnight at room temperature, after which it was mixed with ethyl acetate. The ethyl acetate solution was washed with a 10% v/v aqueous solution of hydrochloric acid, a saturated aqueous solution of sodium bicarbonate and a saturated aqueous solution of sodium chloride, in that order, dried over anhydrous sodium sulphate and concentrated by evaporation under reduced pressure. The residue obtained was purified by silica gel column chromatography (eluted with a 20:1 by volume mixture of chloroform and methanol), to give 880 mg of the title compound as a colourless caramel-like substance. This substance was subjected immediately to the next reaction, without further purification.

14(b) N-[4(S)-($N^{\varepsilon}$-t-Butoxycarbonyl-L-lysylamino)-3(S)-hydroxy-6-methylheptanoyl]-L-isoleucinol

837 mg (1.31 mmole) of N-[4(S)-($N^{\alpha}$-benzyloxy-$N^{\varepsilon}$-t-butoxycarbonyl-L-lysylamino)-3(S)-hydroxy-6-methylheptanoyl]-L-isoleucinol [prepared as described in Example 14(a)] were dissolved in 10 ml of methanol. To this solution were added 1.4 ml of 1N aqueous hydrochloric acid and a catalytic amount of 10% w/w

palladium-on-carbon and, whilst bubbling hydrogen through it, it was stirred at room temperature for 1.5 hours. The reaction solution was then filtered, and the filtrate was mixed with 1.4 ml of a 1N aqueous solution of sodium hydroxide and then concentrated by evaporation under reduced pressure. A saturated aqueous solution of sodium bicarbonate was added to the residue, and the mixture was extracted with ethyl acetate. The ethyl acetate solution was washed with a saturated aqueous solution of sodium chloride, dried over anhydrous sodium sulphate and concentrated by evaporation under reduced pressure. The residue was mixed with a 1:1 by volume mixture of hexane and ethyl acetate and pulverized, to give 482 mg of the title compound as colourless crystals, melting at 80-82°C.

$[\alpha]^{23}$ = -45.0° (C=0.2, methanol).

<u>14(c) N-[4(S)-{$N^{\alpha}$-[Bis(1-naphthylmethyl)acetyl]-$N^{\varepsilon}$-t-butoxycarbonyl-L-lysylamino}-3(S)-hydroxy-6-methylheptanoyl]-L-isoleucinol</u>

200 mg (0.4 mmole) of <u>N</u>-[4(<u>S</u>)-(<u>N</u>$^{\varepsilon}$-t-butoxycarbonyl-<u>L</u>-lysylamino)-3(<u>S</u>)-hydroxy-6-methylheptanoyl]-<u>L</u>-isoleucinol [prepared as described in Example 14(b)], 134 mg (0.39 mmole) of bis(1-naphthylmethyl)acetic acid and 78 mg (0.48 mmole) of diethyl phosphorocyanidate were dissolved in 2 ml of dimethylformamide. To this

solution were added 49 mg (0.48 mmole) of triethylamine, whilst ice-cooling, and the mixture was stirred at 0°C for 30 minutes, and then at room temperature for 4 hours. Ethyl acetate was added to this reaction mixture, which was then washed with a 10% v/v aqueous solution of hydrochloric acid, a saturated aqueous solution of sodium bicarbonate and a saturated aqueous solution of sodium chloride, in that order, dried over anhydrous sodium sulphate and concentrated by evaporation under reduced pressure. The residue obtained was purified by thin layer silica gel column chromatography to give 247 mg of the title compound as colourless crystals, melting at 81-89°C.

$[\alpha]^{23}$ = -93.5° (C=0.2, methanol).

EXAMPLE 15

N-[4(S)-{$N^{\alpha}$-[Bis(1-naphthylmethyl)acetyl]-L-lysylamino}-3(S)-hydroxy-6-methylheptanoyl]-L-isoleucinol hydrochloride (Compound No. 202)

4 ml of a 6N solution of hydrochloric acid in dioxane were added to 163 mg (0.02 mmole) of N-[4(S)-{$N^{\alpha}$-[bis(1-naphthylmethyl)acetyl]-$N^{\varepsilon}$-t-butoxycarbonyl-L-lysylamino}-3(S)-hydroxy-6-methyl-heptanoyl]-L-isoleucinol (prepared as described in Example 14). The solution was stirred at room

temperature for 20 minutes and then concentrated by evaporation under reduced pressure. 2 ml of methanol were added to the residue, and the solution was further concentrated by evaporation under reduced pressure. The residue was mixed with hexane and the resulting solution was evaporated to dryness under reduced pressure, to give 148 mg of the title compound as a glassy substance, melting at 82-87°C.

$[\alpha]^{23}$ = -72.0°C (C=0.2, methanol).

## EXAMPLE 16

N-[4(S)-{N-[Bis(1-naphthylmethyl)acetyl]-3-(4-thiazolyl)-DL-alanylamino}-3(S)-hydroxy-6-methylheptanoyl]-L-isoleucinol (Compound No. 223)

16(a) Methyl ester of N-[bis(1-naphthylmethyl)acetyl]-3-(4-thiazolyl)-DL-alanine

511 mg (1.50 mmole) of bis(1-naphthylmethyl)acetic acid and 389 mg (1.50 mmole) of the methyl ester of 3-(4-thiazolyl)-DL-alanine dihydrochloride were suspended in 20 ml of anhydrous tetrahydrofuran. 0.25 ml (1.65 mmole) of diethyl phosphorocyanidate and 0.69 ml (4.95 mmole) of triethylamine were added, whilst ice-cooling, to this suspension, and the resulting

mixture was stirred overnight at room temperature under a nitrogen atmosphere. At the end of this time, the solvent was distilled off under reduced pressure, and the residue was purified by silica gel column chromatography (eluted with a 2:1 by volume mixture of ethyl acetate and hexane) to give 630 mg of the title compound, melting at 110-112°C.

16(b) N-[Bis(1-naphthylmethyl)acetyl]-3-(4-thiazolyl)-DL-alanine hydrazide

620 mg (1.22 mmole) of the methyl ester of N-[bis(1-naphthylmethyl)acetyl]-3-(4-thiazolyl)-DL-alanine [prepared as described in Example 16(a)] were dissolved in 8 ml of anhydrous dimethylformamide. This solution was mixed with 310 mg (6.19 mmole) of hydrazine hydrate and stirred for 5 hours at 40°C. The solvent was then distilled off under reduced pressure, and water was added to the residue. The crystals which separated were collected by filtration, washed with a mixed solvent consisting of hexane and diethyl ether and dried to give 408 mg of the title compound, melting at 118-121°C.

16(c) N-[4(S)-{N-[Bis(1-naphthylmethyl)acetyl]-3-(4-thiazolyl)-DL-alanylamino}-3(S)-hydroxy-6-methylheptanoyl]-L-isoleucinol

393 mg (0.77 mmole) of N-[bis(1-naphthylmethyl)-acetyl]-3-(4-thiazolyl)-DL-alanine hydrazide [prepared as described in Example 16(b)] were suspended in 10 ml of anhydrous dimethylformamide. At -60°C, 0.66 ml of a 4N solution of hydrochloric acid in dioxane was added to this suspension, and then the reaction temperature was raised to -20°C. The suspension was mixed with 0.13 ml (0.97 mmole) of isopentyl nitrite and stirred for 10 minutes. After the disappearance of the hydrazide had been confirmed, the reaction temperature was reduced again to -60°C. 0.33 ml (3.00 mmole) of N-methylmorpholine were added, for neutralization, followed by 328 mg (1.02 mmole) of N-[(3S,4S)-4-amino-3-hydroxy-6-methylheptanoyl]-L-isoleucinol hydrochloride and 0.12 ml (1.09 mmole) of N-methylmorpholine, and the reaction mixture was stirred overnight at 4°C. The solvent was then distilled off under reduced pressure, and the residue was purified by silica gel thin layer chromatography (using a 10:1 by volume mixture of chloroform and methanol as developing solvent), to give 310 mg of the title compound monohydrate as white crystals, melting at 94-98°C.

EXAMPLE 17

N-[4(S)-{2(RS)-[Bis(1-naphthylmethyl)acetyl]amino-nonanoyl}amino-3(S)-hydroxy-6-methylheptanoyl]-L-isoleucinol (Compound No. 286)

3 ml of a 6N solution of hydrochloric acid in dioxane were added to 58.6 mg (0.156 mmole) of N-[4(S)-t-butoxycarbonylamino-3(S)-hydroxy-6-methyl-heptanoyl]-L-isoleucinol. The reaction solution was then stirred for 30 minutes at room temperature and then evaporated to dryness under reduced pressure. The residue was dissolved in 2 ml of dimethylformamide, and then 77.5 mg (0.156 mmole) of 2(RS)-[bis(1-naphthyl-methyl)acetyl]aminononanoic acid and 27.9 mg (0.17 mmole) of diethyl phosphorocyanidate were added, with ice-cooling. 33 mg (0.327 mmole) of triethylamine were then added dropwise to the reaction mixture, and the mixture was stirred for 3.5 hours at room temperature and then concentrated by evaporation under reduced pressure. The residue was mixed with 2 ml of water and extracted with ethyl acetate. The organic layer was dried over anhydrous magnesium sulphate and concentrated by evaporation under reduced pressure. The residue was purified by preparative silica gel thin layer chromatography (developing solvent, a 10:1 by volume mixture of chloroform and methanol), to give 21.0 mg of

the title compound as a colourless powder, melting at 75-78°C.

EXAMPLE 18

N-[4(S)-{N-[Bis(1-naphthylmethyl)acetyl]-DL-propargylglycylamino}-3(S)-hydroxy-6-methylheptanoyl]-L-isoleucinol (Compound No. 296)

10 ml of a 6N solution of hydrochloric acid in dioxane was added to 749 mg (2 mmole) of N-[4(S)-t-butoxycarbonylamino-3(S)-hydroxy-6-methylheptanoyl]-L-isoleucinol. The solution was stirred for 30 minutes at room temperature, and then evaporated to dryness under reduced pressure. The residue was dissolved in 20 ml of dimethylformamide, and then 426 mg (2 mmole) of N-t-butoxycarbonyl-DL-propargylglycine and 270 mg (2 mmole) of 1-hydroxybenzotriazole were added. 412 mg (2 mmole) of dicyclohexylcarbodiimide and 1 ml of triethylamine were added to this solution, with ice-cooling. The mixture was stirred for 2 hours at 40-50°C, after which the separated dicyclohexylurea was filtered off. The filtrate was mixed with ethyl acetate, washed with water, a 10% w/v aqueous solution of citric acid, a 5% w/v aqueous solution of sodium bicarbonate and a saturated aqueous solution of sodium chloride, in that order, and the organic layer was dried

over anhydrous sodium sulphate. The solvent was then distilled off under reduced pressure. The residue was mixed with diethyl ether and the resulting precipitate was filtered off. The filtrate was evaporated to dryness under reduced pressure. 10 ml of a 6N solution of hydrochloric acid in dioxane were added to the residue, and, after stirring for 30 minutes at room temperature, the solution was evaporated to dryness under reduced pressure. The residue was dissolved in 20 ml of dimethylformamide to which 680 mg (2 mmole) of bis(1-naphthylmethyl)acetic acid and 543 mg (3 mmole) of diethyl phosphorocyanidate were added. To this solution was added 1 ml of triethylamine, whilst stirring. The solution was stirred for 2 hours at 40-50°C, poured into ice-water and extracted with ethyl acetate. The ethyl acetate layer was washed with a 10% w/v aqueous solution of citric acid, a 5% w/v aqueous solution of sodium bicarbonate and a saturated aqueous solution of sodium chloride in that order, and then the organic layer was dried over anhydrous sodium sulphate. The solvent was distilled off under reduced pressure, and then purification by preparative thin layer chromatography (developing solvent: ethyl acetate) afforded two isomers of the title compound (the isomers being ascribed to the asymmetric carbon atom in the propargylglycyl moiety); these melted at 182-188°C (Isomer 1) and 92-94°C (Isomer 2).

The compounds of the invention listed in the following Tables 1 and 2 were also prepared.

In Table 1, the configurations of the compounds are identified as (1), (2), (3), (4), where:

(1) is the configuration of the amino acid of which $R^3$ forms part;

(2) is the configuration of the carbon atom bearing the hydroxy group shown in formula (I);

(3) is the configuration of the carbon atom to which is attached the group of formula $-CH_2-R^4$; and

(4) is, where appropriate, the configuration of any asymmetric carbon atom within the group represented by $R^5$.

Also identified in Table 1 is the number of the Example (of those given above) which most closely represents the techniques employed to prepare the relevant compounds.

Table 1

| Cpd No | Configuration | as in Ex. No. | m.p. (°C) | $[\alpha]^{23}$ | Note |
|---|---|---|---|---|---|
| 2 | S,S,S,- | 2(d) | 114-118 | -69° | - |
| 3 | S,S,S,RS | 2 | - | -48.5° | 1 |
| 6 | S,S,S,S | 2(d) | 100-105 | -50° | - |
| 11 | S,S,S,- | 2(d) | - | -36° | 2 |
| 12 | S,S,S,- | 2 | 122-124 | -53° | - |
| 14 | S,S,S,- | 2 | 125-128 | -82° | - |
| 15 | S,S,S,- | 2 | 65-67 | -65° | - |
| 16 | S,S,S,- | 2 | 135-138 | -61° | 1 |
| 17 | S,S,S,S | 9 | 115-120 | -54° | - |
| 18 | S,S,S,S | 9 | 184-189 | -51° | - |

Table 1 (cont)

| Cpd No | Configuration | as in Ex. No. | m.p. (°C) | $[\alpha]^{23}$ | Note |
|---|---|---|---|---|---|
| 24 | S,S,S,S | 10 | 168-182 | -57° | 3 |
| 36 | S,S,S,S | 1 | 91-99 | - | - |
| 39 | S,S,S,S | 1 | 88-90 | -119° | - |
| 43 | S,S,S,- | 1 | 97-100 | - | - |
| 47 | S,S,S,- | (i)-4(a) | 75-77 | - | 4 |
| | | (ii)-3(b) | 82-86 | -147° | 4 |
| 53 | S,S,S,- | (i)-6(a) | 199-200 | - | 4 |
| | | (ii)-6(b) | 82-85 | -122° | 4 |
| 59 | S,S,S,- | (i)-6(a) | 180-182 | - | 4 |
| | | (ii)-6(b) | 85-90 | -163° | 4 |
| 60 | S,S,S,- | (i)-4(a) | 183-185 | - | 4 |
| | | (ii)-3(b) | 115-120 | -140° | 4 |
| 71 | S,S,S,- | 9 | 165-167 | - | - |

Table 1 (cont)

| Cpd No | Configuration | as in Ex. No. | m.p. (°C) | $[\alpha]^{23}$ | Note |
|---|---|---|---|---|---|
| 83 | S,S,S,S | 9 | 186-189 | - | - |
| 110 | S,S,S,S | 9 | 78-80 | - | - |
| 116 | S,S,S,S | 9 | 67-69 | - | 5 |
| 124 | S,S,S,S | 9 | 60-68 | - | - |
| 153 | S,S,S,S | 10 | - | -72° | 6 |
| 158 | S,S,S,S | 10 | - | -75° | 6 |
| 165 | S,S,S,S | 12 | 114-118 | - | - |
| 189 | S,S,S,S | 16 | 79-84 | - | - |
| 191 | S,S,S,S | 16 | 89-91 | -67° | 7 |
| 195 | S,S,S,S | 16 | 119-122 | -78° | 7 |
| 205 | S,S,S,S | 16 | 200-203 | -69° | 7 |

Table 1 (cont)

| Cpd No | Configuration | as in Ex. No. | m.p. (°C) | $[\alpha]^{23}$ | Note |
|---|---|---|---|---|---|
| 208 | S,S,S,S | 16 | 174-177 | -129° | 7 |
| 210 | S,S,S,S | 16 | 65-67 | -100° | - |
| 212 | S,S,S,S | 16 | 92-98 | -121° | - |
| 213 | S,S,S,S | 16 | 145-147 | -49° | 7 |
| 220 | RS,S,S,S | 16 | 110-113 | - | - |
| 231 | RS,S,S,S | 16 | 101-103 | - | - |
| 232 | RS,S,S,S | 16 | 92-94 | - | - |
| 235 | RS,S,S,S | 12 | 78-83 | - | - |
| 274 | RS,S,S,RS | 17 | 71-72 | - | - |
| 275 | RS,S,S,S | 17 | 95-97 | - | - |
| 276 | RS,S,S,- | 17 | 85-88 | - | - |

Table 1 (cont)

| Cpd No | Configuration | as in Ex. No. | m.p. (°C) | $[\alpha]^{23}$ | Note |
|---|---|---|---|---|---|
| 277 | RS,S,S,- | 17 | 78-80 | - | - |
| 289(1) | R/S,S,S,S | 18 | 55-58 | - | 8 |
| 289(2) | R/S,S,S,S | 18 | 52-55 | - | 8 |
| 291 | RS,S,S,S | 17 | 93-95 | - | - |

Table 2

| Cpd No | Elemental Analysis (Calculated) Found [formula] | Appearance | Note |
|---|---|---|---|
| 2 | (C, 72.30%; H, 8.87%; N, 6.49%)<br>C, 72.45%; H, 8.66%; N, 6.32%.<br>[$C_{39}H_{55}N_3O_4.H_2O$] | colorless prisms | 9 |
| 6 | (C, 71.74%; H, 9.10%; N, 5.84%)<br>C, 71.84%; H, 8.80%; N, 5.92%.<br>[$C_{43}H_{63}N_3O_5.H_2O$] | white crystals | - |
| 17 | (C, 69.78%; H, 8.37%; N, 9.69%)<br>C, 70.075; H, 8.01%; N, 9.72%.<br>[$C_{42}H_{57}N_5O_4$. $1.5H_2O$ | colorless powdery crystals | - |
| 18 | (C, 69.41%; H, 8.26%; N, 9.41%)<br>C, 69.47%; H, 7.95%; N, 9.45%<br>[$C_{43}H_{59}N_5O_5.H_2O$] | - | - |
| 39 | (C, 73.50%; H, 8.41%; N, 5.84%)<br>C, 73.37%; H, 8.21%; N, 5.95%<br>[$C_{44}H_{59}N_3O_5$. $0.5H_2O$] | colorless powder | - |

Table 2 (cont)

| Cpd No | | Elemental Analysis (Calculated) Found [formula] | Appearance | Note |
|---|---|---|---|---|
| 47 | (i) | (C, 57.60%; H, 9.71%; N, 8.40%)<br>C, 57.74%; H, 9.69%; N, 8.31%<br>[$C_{24}H_{47}N_3O_5 \cdot 3/5H_2O$] | - | 4 |
| | (ii) | (C, 71.64%; H, 8.11%; N, 5.83%)<br>C, 71.61%; H, 8.25%; N, 5.53%<br>[$C_{43}H_{57}N_3O_6 \cdot H_2O$] | pale brown powder | 3,4 |
| 53 | (i) | (C, 59.97%; H, 9.66%; N, 11.19%)<br>C, 59.80%; H, 9.70%; N, 11.17%<br>[$C_{25}H_{48}N_4O_6$] | | 4 |
| | (ii) | (C, 72.20%; H, 8.12%; N, 7.65%)<br>C, 72.28%; H, 8.03%; N, 7.61%<br>[$C_{44}H_{58}N_4O_5 \cdot 0.5H_2O$] | white powder | 4 |
| 59 | (i) | (C, 63.39%; H, 9.00%; N, 11.37%)<br>C, 63.10%; H, 8.95%; N, 11.31%<br>[$C_{26}H_{44}N_4O_5$] | - | 4 |
| | (ii) | (C, 71.97%; H, 7.78%; N, 7.46%)<br>C, 71.68%; H, 7.91%; N, 7.85%<br>[$C_{45}H_{54}N_4O_4 \cdot 2H_2O$] | white powder | 4 |

Table 2 (cont)

| Cpd No | | Elemental Analysis (Calculated) Found [formula] | Appearance | Note |
|---|---|---|---|---|
| 60 | (i) | (C, 63.93%; H, 10.09%; N, 8.95%) C, 63.80%; H, 10.00%; N, 8.89% [$C_{25}H_{47}N_3O_5$] | - | 4 |
| | (ii) | (C, 76.37%; H, 8.30%; N, 6.07%) C, 76.16%; H, 8.31%; N, 6.11% [$C_{44}H_{57}N_3O_4$] | white powder | 4 |

Notes:

1. Optical rotation - C=0.2, methanol.

2. Purified by thin layer chromatography [Merck, Art 5715], developing solvent, chloroform:methanol = 10:1 by volume, Rf = 0.50. A colorless, glassy substance.

3. Product purified by thin layer chromatography, developing solvent a 5:1 by volume mixture of chloroform and methanol.

4. A two-stage process. In each case, (i) represents the intermediate and (ii) the final product.

5. Melting with decomposition.

6. Developing solvent for purification, ethyl acetate:benzene:methanol = 5:1:0.2 by volume. Rf values (silica gel [Merck, Art 5715] developing solvent, chloroform:methanol = 10:1 by volume) are 0.59 and 0.65, respectively, for Compounds Nos. 153 and 158.

7. Optical rotation at 25°C, i.e. $[\alpha]^{25}$.

8. 289(1) and 289(2) are Isomers 1 and 2, respectively of Compound 289. Accordingly, "R/S" means one is R and the other is S, but it is not presently known which is which.

9. Product purified as in Example 1(b).

PREPARATION 1

Bis(1-naphthylmethyl)acetic acid

2.86 g (0.12 mmole) of sodium were dissolved in 250 ml of anhydrous ethanol, and, whilst ice-cooling, 19.95 g (0.12 mmole) of diethyl malonate were added to the resulting solution. The reaction solution was then stirred for 1 hour, after which 22.00 g (0.12 mmole) of 1-(chloromethyl)naphthalene were added dropwise, and the solution was stirred for 3 hours at room temperature, and then stirred for 3 hours at 50°C. The salt which separated was filtered off, and the filtrate was concentrated by evaporation under reduced pressure. The residue was purified by medium pressure liquid silica gel column chromatography to give 15.2 g (41.3%) of diethyl (1-naphthylmethyl)malonate and 9.8 g (17.8%) of diethyl di(1-naphthylmethyl)malonate.

9.8 g (22.2 mmole) of the diethyl di(1-naphthylmethyl)malonate were dissolved in 100 ml of a 1:1 by volume mixture of butanol and water. 10 g of potassium hydroxide were added to this solution, and the mixture was heated under reflux at 150°C for 8 hours. The reaction mixture was then concentrated by evaporation under reduced pressure. The residue was mixed with water and then sufficient concentrated hydrochloric acid

was added to adjust the pH to a value of 1. The separated crystals were filtered off, to give 6.2 g (82%) of the title compound as white crystals, melting at 172-174°C.

## PREPARATION 2

2-(1-Naphthylmethyl)-6-phenylhexanoic acid

1.3 g (57 mmole) of sodium was dissolved in 200 ml of anhydrous anhydrous ethanol. This solution was mixed with 14.8 g (50 mmole) of diethyl (1-naphthylmethyl)malonate under ice-cooling and stirred for 1 hour. 11.7 g (55 mmole) of 4-phenylbutyl bromide were then added dropwise, and the mixture was stirred for 6 hours at 50°C. The salt which separated was filtered off, and the filtrate was concentrated by evaporation under reduced pressure. The residue was purified by silica gel column chromatography (eluted with a 1:4 by volume mixture of ethyl acetate and hexane) to give 15.4 g (71%) of diethyl 2-(1-naphthylmethyl)-2-(4-phenylbutyl)malonate.

This compound was treated, in a similar manner to that described in Preparation 1, with potassium hydroxide in aqueous butanol, to give 6.8 g (58%) of the title compound as a colourless oily substance.

PREPARATION 3

Diethyl (1-naphthylmethyl)malonate

Sodium ethoxide [which had been prepared from 2.3 g (0.1 gram atom) of sodium] was suspended in 250 ml of dimethylformamide. 16.0 g of diethyl malonate were added, whilst ice-cooling, to the suspension, and the mixture was stirred at 0°C for 30 minutes and then at room temperature for 1 hour. 100 ml of a dimethylformamide solution containing 17.6 g (0.1 mmole) of 1-chloromethylnaphthalene was added dropwise to this solution over 2 hours. This mixture was then stirred at room temperature overnight, after which the solvent was distilled off under reduced pressure. The residue was mixed with water and then extracted with ethyl acetate. The ethyl acetate layer was washed with a saturated aqueous solution of sodium chloride, dried over anhydrous sodium sulphate and concentrated by evaporation under reduced pressure. Evaporation of the residue under reduced pressure gave 21.3 g of the title compound as a colourless viscous liquid, boiling at 210°C (5 mmHg-667 Pa).

## PREPARATION 4

Diethyl benzyl(1-naphthylmethyl)malonate

1.05 g (24 mmole) of a 55% w/w suspension of sodium hydride in mineral oil was washed three times with anhydrous hexane to dry it, and then mixed with 100 ml of dimethylformamide. To this suspension was added 7.21 g (24 mmole) of diethyl (1-naphthylmethyl)malonate, whilst ice-cooling. After the generation of hydrogen had finished, the reaction mixture was stirred for 30 minutes, and then 4.10 g (24 mmole) of benzyl bromide were added, and the mixture was stirred for a further 2 hours at room temperature. At the end of this time, the reaction mixture was concentrated by evaporation under reduced pressure. The residue was mixed with water and extracted with ethyl acetate. The ethyl acetate layer was washed with a saturated aqueous solution of sodium chloride, dried over anhydrous sodium sulphate and concentrated by evaporation under reduced pressure. The residue was purified by silica gel column chromatography (eluted with a 15:1 by volume mixture of hexane and ethyl acetate) to give 7.3 g of the title compound as a colourless oily substance.

Mass spectrum (m/e) : 390 ($M^+$).

## PREPARATION 5

**Diethyl [2-(2-methoxyethoxy)ethyl](1-naphthylmethyl)-malonate**

The reaction described in Preparation 4 was repeated, but using 2.12 g (10 mmole) of 2-(2-methoxyethoxy)ethyl iodide and 2.0 g (6.7 mmole) of diethyl (1-naphthylmethyl)malonate, to give 1.93 g of the title compound as a colourless oily substance after purification by silica gel column chromatography (eluted with a 10:1 by volume mixture of hexane and ethyl acetate).

Mass spectrum (m/e) : 402 ($M^+$).

## PREPARATION 6

**Diethyl [2-(2-benzyloxyethoxy)ethyl](1-naphthylmethyl)-malonate**

The reaction described in Preparation 4 was repeated, but using 3.98 g (10 mmole) of 2-(2-benzyloxyethoxy)ethyl iodide and 3.0 g (10 mmole) of diethyl (1-naphthylmethyl)malonate, to give 3.62 g of the title compound as a colourless oily substance after purification by silica gel column chromatography (eluted

with an 8:1 by volume mixture of hexane and ethyl acetate).

Mass spectrum (m/e) : 478 ($M^+$).

PREPARATION 7

2-Benzyl-3-naphthylpropionic acid

A mixture of 7.3 g (18 mmole) of diethyl benzyl-(1-naphthylmethyl)malonate, 70 ml of butanol, 70 ml of water and 7 g of potassium hydroxide was heated on an oil bath (150°C) under reflux for 10 hours. The reaction mixture was cooled and then concentrated by evaporation under reduced pressure, after which it was acidified with concentrated hydrochloric acid and extracted with ethyl acetate. The ethyl acetate layer was washed with a saturated aqueous solution of sodium chloride, dried over anhydrous sodium sulphate and concentrated by evaporation under reduced pressure. The residue was purified by silica gel column chromatography (eluted with a 1:1 by volume mixture of hexane and ethyl acetate), and the resulting solid was recrystallized from a mixture of hexane and ethyl acetate, to give 3.29 g of the title compound as colourless crystals, melting at 119-120°C.

Mass spectrum (m/e): 290 ($M^+$).

## PREPARATION 8

2-[2-(2-Methoxyethoxy)ethyl]-3-(1-naphthyl)propionic acid

The reaction described in Preparation 7 was repeated, but using 1.87 g (4.6 mmole) of diethyl [2-(2-methoxyethoxy)ethyl](1-naphthylmethyl)malonate, to give 1.12 g of the title compound as a colourless oily substance after purification by silica gel column chromatography (eluted with ethyl acetate).

Mass spectrum (m/e): 302 ($M^+$).

## PREPARATION 9

2-[2-(2-Benzyloxyethoxy)ethyl]-3-(1-naphthyl)propionic acid

The reaction described in Preparation 7 was repeated, but using 2.50 g (5.2 mmole) of diethyl [2-(2-benzyloxyethoxy)ethyl](1-naphthylmethyl)malonate, to give 1.76 g of the title compound as a colourless oily substance after purification by silica gel column chromatography (eluted with ethyl acetate).

Mass spectrum (m/e): 378 ($M^+$).

CLAIMS:

1. Compounds of formula (I):

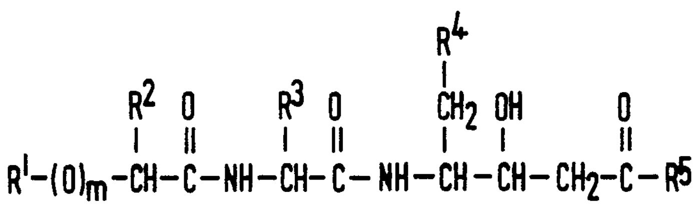

(I)

wherein:

m is 0 or 1;

$R^1$ and $R^2$ are the same or different and each represents a $C_1$-$C_{10}$ alkyl group, a group of formula $-(A)_p-R^6$, wherein:

p is 0 or 1;

A represents a $C_1$-$C_4$ alkylene group or a $C_2$-$C_4$ alkenylene group; and

$R^6$ represents an aryl group or a heterocyclic group;

or a group of formula $-B-R^7$, wherein:

B represents a $C_1$-$C_4$ alkylene group or an alkoxyalkyl group where the alkoxy and alkyl parts are both $C_1$-$C_3$; and

$R^7$ represents a $C_1$-$C_4$ alkoxy group, an aryloxy group, an aralkyloxy group where the alkyl part is $C_1$-$C_4$ or a heterocyclic group;

$R^3$ represents a $C_1$-$C_{10}$ alkyl group, a $C_1$-$C_{10}$ alkyl group having at least one of the substituents (a):

(a) hydroxy groups, $C_1$-$C_4$ alkoxy groups, aryloxy groups, aralkyloxy groups where the alkyl part is $C_1$-$C_4$, $C_1$-$C_7$ aliphatic carboxylic acyloxy groups, $C_1$-$C_4$ alkylthio groups, arylthio groups, aralkylthio groups where the alkyl part is $C_1$-$C_4$, $C_1$-$C_4$ alkylsulphinyl groups, $C_1$-$C_4$ alkylsulphonyl groups, arylsulphinyl groups, arylsulphonyl groups, $C_1$-$C_7$ aliphatic carboxylic acylamino groups, aromatic carboxylic acylamino groups, $C_2$-$C_7$ alkoxycarbonylamino groups, aralkyloxycarbonylamino groups where the

alkyl part is $C_1$-$C_4$, $C_2$-$C_7$ alkoxycarbonyl groups, aryloxycarbonyl groups, aralkyloxycarbonyl groups where the alkyl part is $C_1$-$C_4$, carbamoyl groups, alkylcarbamoyl groups where the alkyl part is $C_1$-$C_4$, dialkylcarbamoyl groups where each alkyl part is $C_1$-$C_4$, thiocarbamoyl groups, alkyl(thiocarbamoyl) groups where the alkyl part is $C_1$-$C_4$, dialkyl(thiocarbamoyl) groups where each alkyl part is $C_1$-$C_4$, ureido groups, alkylureido groups where the alkyl part is $C_1$-$C_4$, dialkylureido groups where each alkyl part is $C_1$-$C_4$, thioureido groups, alkyl(thioureido) groups where the alkyl part is $C_1$-$C_4$, dialkyl(thioureido) groups where each alkyl part is $C_1$-$C_4$, $C_3$-$C_8$ cycloalkyl groups, $C_5$-$C_8$ cycloalkenyl groups, aryl groups, heterocyclic groups, halogen atoms, mono-, di- and tri-halomethyl groups, mercapto groups, amino groups, $C_1$-$C_4$ alkylamino groups, dialkylamino groups where each alkyl part is $C_1$-$C_4$, carboxy groups and guanidino groups;

a $C_2$-$C_5$ alkenyl group, a $C_2$-$C_5$ alkenyl group having at least one halogen substituent, a $C_2$-$C_5$ alkynyl group, a hydrogen atom or a $C_3$-$C_8$ cycloalkyl group;

$R^4$ represents an isopropyl group, a $C_3$-$C_8$ cycloalkyl group or a phenyl group;

$R^5$ represents a hydroxy group, a $C_1$-$C_{10}$ alkoxy group, an aryloxy group, a group of formula $-NR^8R^9$ wherein

$R^8$ and $R^9$ are the same or different and each represents a hydrogen atom, a $C_1$-$C_{10}$ alkyl group, a $C_1$-$C_{10}$ alkyl group having at least one of the substituents (b):

(b) hydroxy groups, $C_1$-$C_4$ alkoxy groups, halogen atoms, aryl groups, $C_3$-$C_8$ cycloalkyl groups, $C_1$-$C_4$ alkylamino groups, dialkylamino groups where each alkyl part is $C_1$-$C_4$, ($C_1$-$C_4$ hydroxyalkyl)amino groups, di($C_1$-$C_4$ hydroxyalkyl)amino groups and heterocyclic groups;

a $C_3$ or $C_4$ alkenyl group, a $C_3$-$C_8$ cycloalkyl group, an aryl group or a heterocyclic group;

or a heterocyclic group;

said aryl groups and the aryl parts of said aryloxy,

aralkyloxy, arylthio, aralkylthio, arylsulphinyl, arylsulphonyl, aromatic carboxylic acylamino, aralkyloxycarbonylamino, aryloxycarbonyl and aralkyloxycarbonyl groups are $C_6$-$C_{14}$ carbocyclic aryl groups which are unsubstituted or have at least one of the substituents (a), (b) or (c):

(c) $C_1$-$C_4$ alkyl groups, nitro groups, cyano groups, $C_3$-$C_5$ alkenoyl groups, $C_3$-$C_5$ alkenoyl groups having at least one of said substituents (a) and $C_1$-$C_4$ alkyl groups having at least one of said substituents (b); and

said heterocyclic groups have from 5 to 14 ring atoms of which from 1 to 4 are nitrogen, oxygen or sulphur hetero-atoms and are unsubstituted or have at least one of said substituents (a), (b) or (c);

and pharmaceutically acceptable salts thereof.

2. Compounds as claimed in Claim 1, wherein:

<u>m</u> is 0;

$R^1$ and $R^2$ are the same or different and each represents a $C_1$-$C_{10}$ alkyl group, a group of formula $-(A)_p-R^6$, wherein:

p is 0 or 1;

A represents a $C_1-C_4$ alkylene group or a $C_2-C_4$ alkenylene group; and

$R^6$ represents an aryl group or an aromatic heterocyclic group;

or a group of formula $-B-R^7$, wherein:

B represents a $C_1-C_4$ alkylene group or an alkoxyalkyl group where the alkoxy and alkyl parts are both $C_1$ or $C_2$; and

$R^7$ represents a $C_1-C_4$ alkoxy group or an aralkyloxy group where the alkyl part is $C_1-C_4$;

$R^3$ represents a $C_1-C_{10}$ alkyl group, a $C_1-C_{10}$ alkyl group having at least one of the substituents (a'):

(a') hydroxy groups, $C_1-C_4$ alkoxy groups, $C_1-C_4$ alkylthio groups, $C_1-C_7$ aliphatic carboxylic acylamino groups, $C_2-C_5$ alkoxycarbonylamino groups, benzyloxycarbonylamino groups, $C_2-C_5$ alkoxycarbonyl groups, carbamoyl groups, $C_3-C_8$ cycloalkyl groups, phenyl groups, phenyl groups having at least one of the

substituents (a'), (b') and (c), aromatic heterocyclic groups and carboxy groups;

a $C_3$ or $C_4$ alkenyl group or a propargyl group;

$R^4$ represents an isopropyl group, a $C_5$ or $C_6$ cycloalkyl group or a phenyl group;

$R^5$ represents a hydroxy group, a group of formula $-NR^8R^9$ wherein

$R^8$ and $R^9$ are the same or different and each represents a hydrogen atom, a $C_1$-$C_{10}$ alkyl group, a $C_1$-$C_{10}$ alkyl group having at least one of the substituents (b'):

(b') hydroxy groups, $C_1$-$C_4$ alkoxy groups, pyridyl groups, dialkylamino groups where each alkyl part is $C_1$-$C_4$, di($C_1$-$C_4$ hydroxyalkyl)amino groups and non-aromatic heterocyclic groups;

a $C_3$ or $C_4$ alkenyl group or a heterocyclic group;

or a heterocyclic group;

said aryl groups and the aryl parts of said aralkyloxy

groups are $C_6$-$C_{10}$ carbocyclic aryl groups which are unsubstituted or have at least one of said substituents (a), (b) and (c) defined in Claim 1; and

said heterocyclic groups have from 5 to 10 ring atoms of which from 1 to 3 are nitrogen, oxygen or sulphur hetero-atoms and are unsubstituted or have at least one of said substituents (a), (b) and (c).

3. Compounds as claimed in Claim 1, wherein:

m is 0;

$R^1$ and $R^2$ are the same or different and each represents a group of formula $-A-R^6$ in which:

A represents a $C_1$-$C_4$ alkylene group; and
$R^6$ represents an aryl group or an aromatic heterocyclic group;

or of formula $-B-R^7$ in which:

B represents a $C_1$-$C_4$ alkylene group or an alkoxyalkyl group in which each of the alkoxy part and the alkyl part is $C_1$ or $C_2$; and
$R^7$ represents a $C_1$-$C_4$ alkoxy group or a benzyloxy group;

$R^3$ represents a $C_1$-$C_{10}$ alkyl group, a $C_1$-$C_{10}$ alkyl group having at least one of the substituents (a"):

(a") hydroxy groups, $C_1$-$C_4$ alkylthio groups, carbamoyl groups, phenyl groups, aromatic heterocyclic groups, $C_2$-$C_5$ alkoxycarbonylamino groups, carboxy groups and cyclopentyl groups;

a $C_3$ or $C_4$ alkenyl group or a propargyl group;

$R^4$ represents an isopropyl group, a $C_5$ or $C_6$ cycloalkyl group or a phenyl group; and

$R^5$ represents a group of formula $-NR^{8'}R^{9'}$, in which $R^{8'}$ and $R^{9'}$ are the same or different and each represents a hydrogen atom, a 1-benzyl-4-piperidyl group, a $C_1$-$C_{10}$ alkyl group or a $C_1$-$C_{10}$ alkyl group having at least one of the substituents (b"):

(b") hydroxy groups, $C_1$-$C_4$ alkoxy groups, di($C_1$-$C_4$ hydroxyalkyl)amino groups and non-aromatic heterocyclic groups.

4. Compounds as claimed in Claim 1, wherein:

__m__ is 0;

$R^2$ represents a 1-naphthylmethyl group; and

$R^4$ represents an isopropyl group.

5. Compounds as claimed in Claim 4, wherein:

$R^1$ represents a $C_1$-$C_4$ alkyl group having a single ω-substituent selected from phenyl, 3-pyridyl, 1-naphthyl, 2-methoxyethoxy and 2-benzyloxyethoxy groups;

$R^3$ represents an allyl group, a 2-propynyl group, a $C_1$-$C_{10}$ alkyl group or a $C_1$-$C_4$ alkyl group having a single ω-substituent selected from 5-imidazolyl, methylthio, t-butoxycarbonylamino, carbamoyl, carboxy, 1,3-thiazol-4-yl, phenyl and cyclopentyl substituents; and

$R^5$ represents an amino group, a (1-benzyl-4-piperidyl)amino group or a $C_2$-$C_6$ alkylamino group having one or two substituents selected from hydroxy, $C_1$ or $C_2$ alkoxy, di($C_1$ or $C_2$ alkyl)amino and morpholino substituents.

6. Compounds as claimed in Claim 1, wherein:

<u>m</u> is 0;

$R^1$ and $R^2$ both represent 1-naphthylmethyl groups; and

$R^4$ represents an isopropyl group.

7. Compounds as claimed in Claim 6, wherein:

$R^3$ represents an allyl group, a 2-propynyl group, a $C_1$-$C_5$ alkyl group or a $C_1$-$C_4$ alkyl group having a single ω-substituent selected from 5-imidazolyl, carbamoyl, 1,3-thiazol-4-yl and cyclopentyl substituents; and

$R^5$ represents a $C_2$-$C_6$ alkylamino group or a $C_2$-$C_6$ alkylamino group having a single substituent selected from hydroxy groups, di($C_1$ or $C_2$ alkyl)amino and morpholino groups.

8. N-[4(S)-{N-[Bis(1-naphthylmethyl)acetyl]-L-leucylamino}-3(S)-hydroxy-6-methylheptanoyl]-L-isoleucinol and pharmaceutically acceptable salts thereof.

9. 4(S)-{N-[Bis(1-naphthylmethyl)acetyl]-L-leucylamino}-3(S)-hydroxy-6-methyl-N-(2-methylbutyl)-heptanamide and pharmaceutically acceptable salts thereof.

10. 4(S)-{N-[Bis(1-naphthylmethyl)acetyl]-L-leucyl-

amino}-3(S)-hydroxy-6-methyl-N-(2-diethylaminoethyl)-heptanamide and pharmaceutically acceptable salts thereof.

11. 4(S)-{N-[Bis(1-naphthylmethyl)acetyl]-L-leucyl-amino}-3(S)-hydroxy-6-methyl-N-(2-morpholinoethyl)-heptanamide and pharmaceutically acceptable salts thereof.

12. 4-[4(S)-{N-[Bis(1-naphthylmethyl)acetyl]-L-leucylamino}-3(S)-hydroxy-6-methylheptanoyl]amino-1-benzylpiperidine and pharmaceutically acceptable salts thereof.

13. 4(S)-{N-[Bis(1-naphthylmethyl)acetyl]-L-histidyl-amino}-3(S)-hydroxy-6-methylheptanamide and pharmaceutically acceptable salts thereof.

14. N-[4(S)-{N-[Bis(1-naphthylmethyl)acetyl]-L-histidylamino}-3(S)-hydroxy-6-methylheptanoyl]-L-isoleucinol and pharmaceutically acceptable salts thereof.

15. 4(S)-{N-[Bis(1-naphthylmethyl)acetyl]-L-histidyl-amino}-3(S)-hydroxy-6-methyl-N-[2(S)-(-)-methylbutyl]-heptanamide and pharmaceutically acceptable salts thereof.

16. N-[4(S)-{N-[Bis(1-naphthylmethyl)acetyl]-L-isoleucylamino}-3(S)-hydroxy-6-methylheptanoyl]-L-isoleucinol and pharmaceutically acceptable salts thereof.

17. N-[4(S)-{N-[Bis(1-naphthylmethyl)acetyl]-L-norleucylamino}-3(S)-hydroxy-6-methylheptanoyl]-L-isoleucinol and pharmaceutically acceptable salts thereof.

18. N-[4(S)-{N-[Bis(1-naphthylmethyl)acetyl]-L-valylamino}-3(S)-hydroxy-6-methylheptanoyl]-L-isoleucinol and pharmaceutically acceptable salts thereof.

19. N-[4(S)-{N-[Bis(1-naphthylmethyl)acetyl]-L-glutaminylamino}-3(S)-hydroxy-6-methylheptanoyl]-L-isoleucinol and pharmaceutically acceptable salts thereof.

20. N-[4(S)-{N-[Bis(1-naphthylmethyl)acetyl]-3-(4-thiazolyl)-DL-alanylamino}-3(S)-hydroxy-6-methylheptanoyl]-L-isoleucinol and pharmaceutically acceptable salts thereof.

21. N-[4(S)-{N-[Bis(1-naphthylmethyl)acetyl]-3-(cyclopentyl)-DL-alanylamino}-3(S)-hydroxy-6-methylheptanoyl]-L-isoleucinol and pharmaceutically acceptable

salts thereof.

22. N-[4(S)-{2(RS)-[Bis(1-naphthylmethyl)acetyl]-aminoheptanoyl}amino-3(S)-hydroxy-6-methylheptanoyl]-L-isoleucinol and pharmaceutically acceptable salts thereof.

23. 4(S)-{2(RS)-[Bis(1-naphthylmethyl)acetyl]amino-heptanoyl}amino-3(S)-hydroxy-6-methyl-N-(2-morpholinoethyl)heptanamide and pharmaceutically acceptable salts thereof.

24. N-[4(S)-{N-[Bis(1-naphthylmethyl)acetyl]-DL-allylglycylamino}-3(S)-hydroxy-6-methylheptanoyl]-L-isoleucinol and pharmaceutically acceptable salts thereof.

25. N-[4(S)-{N-[Bis(1-naphthylmethyl)acetyl]-DL-propargylglycylamino}-3(S)-hydroxy-6-methylheptanoyl]-L-isoleucinol and pharmaceutically acceptable salts thereof.

26. A pharmaceutical composition for the treatment of angiotensin-induced hypertension in a mammal, which comprises an active compound in admixture with a pharmaceutically acceptable carrier or diluent, wherein the active compound is a compound as claimed in any one

of Claims 1 to 25.

27. A process for preparing compounds as claimed in any one of Claims 1 to 25, which comprises:

(i) reacting a compound of formula (II):

$$R^1-(O)_m-CH(R^2)-CO-(Y)_r-OH \quad (II)$$

[in which: $R^1$, _m_ and $R^2$ are as defined in Claim 1; Y represents a group of formula $-NH-CH(R^3)CO-$, where $R^3$ is as defined in Claim 1; and _r_ is 0 or 1] or a reactive derivative thereof with a compound of formula (III):

$$H(Y)_sNH-CH(CH_2R^4)-CH(OH)-CH_2COR^5 \quad (III)$$

(in which: Y is as defined above, and $R^4$ and $R^5$ are as defined in Claim 1; and _s_ is 1 if _r_ is 0 or 0 if _r_ is 1) or a reactive derivative thereof;

(ii) optionally subjecting the product to an acyl exchange reaction; and

(iii) optionally converting any group represented by $R^5$ to any other group represented by $R^5$.

28. The use for the manufacture of a medicament for the treatment of hypertension of a compound as claimed in any one of Claims 1 to 25.

CLAIMS for Austria
für Österreich
pour l'Autriche



1. A process for preparing compounds of formula (I):

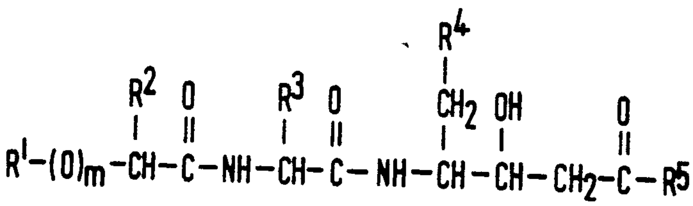

(I)

wherein:

m is 0 or 1;

$R^1$ and $R^2$ are the same or different and each represents a $C_1$-$C_{10}$ alkyl group, a group of formula $-(A)_p-R^6$, wherein:

p is 0 or 1;

A represents a $C_1$-$C_4$ alkylene group or a $C_2$-$C_4$ alkenylene group; and

$R^6$ represents an aryl group or a heterocyclic group;

or a group of formula $-B-R^7$, wherein:

B represents a $C_1-C_4$ alkylene group or an alkoxyalkyl group where the alkoxy and alkyl parts are both $C_1-C_3$; and

$R^7$ represents a $C_1-C_4$ alkoxy group, an aryloxy group, an aralkyloxy group where the alkyl part is $C_1-C_4$ or a heterocyclic group;

$R^3$ represents a $C_1-C_{10}$ alkyl group, a $C_1-C_{10}$ alkyl group having at least one of the substituents (a):

(a) hydroxy groups, $C_1-C_4$ alkoxy groups, aryloxy groups, aralkyloxy groups where the alkyl part is $C_1-C_4$, $C_1-C_7$ aliphatic carboxylic acyloxy groups, $C_1-C_4$ alkylthio groups, arylthio groups, aralkylthio groups where the alkyl part is $C_1-C_4$, $C_1-C_4$ alkylsulphinyl groups, $C_1-C_4$ alkylsulphonyl groups, arylsulphinyl groups, arylsulphonyl groups, $C_1-C_7$ aliphatic carboxylic acylamino groups, aromatic carboxylic acylamino groups, $C_2-C_7$ alkoxycarbonylamino groups, aralkyloxycarbonylamino groups where the

alkyl part is $C_1$-$C_4$, $C_2$-$C_7$ alkoxycarbonyl groups, aryloxycarbonyl groups, aralkyloxycarbonyl groups where the alkyl part is $C_1$-$C_4$, carbamoyl groups, alkylcarbamoyl groups where the alkyl part is $C_1$-$C_4$, dialkylcarbamoyl groups where each alkyl part is $C_1$-$C_4$, thiocarbamoyl groups, alkyl(thiocarbamoyl) groups where the alkyl part is $C_1$-$C_4$, dialkyl(thiocarbamoyl) groups where each alkyl part is $C_1$-$C_4$, ureido groups, alkylureido groups where the alkyl part is $C_1$-$C_4$, dialkylureido groups where each alkyl part is $C_1$-$C_4$, thioureido groups, alkyl(thioureido) groups where the alkyl part is $C_1$-$C_4$, dialkyl(thioureido) groups where each alkyl part is $C_1$-$C_4$, $C_3$-$C_8$ cycloalkyl groups, $C_5$-$C_8$ cycloalkenyl groups, aryl groups, heterocyclic groups, halogen atoms, mono-, di- and tri-halomethyl groups, mercapto groups, amino groups, $C_1$-$C_4$ alkylamino groups, dialkylamino groups where each alkyl part is $C_1$-$C_4$, carboxy groups and guanidino groups;

a $C_2$-$C_5$ alkenyl group, a $C_2$-$C_5$ alkenyl group having at least one halogen substituent, a $C_2$-$C_5$ alkynyl group, a hydrogen atom or a $C_3$-$C_8$ cycloalkyl group;

$R^4$ represents an isopropyl group, a $C_3$-$C_8$ cycloalkyl group or a phenyl group;

$R^5$ represents a hydroxy group, a $C_1$-$C_{10}$ alkoxy group, an aryloxy group, a group of formula $-NR^8R^9$ wherein

$R^8$ and $R^9$ are the same or different and each represents a hydrogen atom, a $C_1$-$C_{10}$ alkyl group, a $C_1$-$C_{10}$ alkyl group having at least one of the substituents (b):

(b) hydroxy groups, $C_1$-$C_4$ alkoxy groups, halogen atoms, aryl groups, $C_3$-$C_8$ cycloalkyl groups, $C_1$-$C_4$ alkylamino groups, dialkylamino groups where each alkyl part is $C_1$-$C_4$, ($C_1$-$C_4$ hydroxyalkyl)amino groups, di($C_1$-$C_4$ hydroxyalkyl)amino groups and heterocyclic groups;

a $C_3$ or $C_4$ alkenyl group, a $C_3$-$C_8$ cycloalkyl group, an aryl group or a heterocyclic group;

or a heterocyclic group;

said aryl groups and the aryl parts of said aryloxy,

aralkyloxy, arylthio, aralkylthio, arylsulphinyl, arylsulphonyl, aromatic carboxylic acylamino, aralkyloxycarbonylamino, aryloxycarbonyl and aralkyloxycarbonyl groups are $C_6$-$C_{14}$ carbocyclic aryl groups which are unsubstituted or have at least one of the substituents (a), (b) or (c):

(c) $C_1$-$C_4$ alkyl groups, nitro groups, cyano groups, $C_3$-$C_5$ alkenoyl groups, $C_3$-$C_5$ alkenoyl groups having at least one of said substituents (a) and $C_1$-$C_4$ alkyl groups having at least one of said substituents (b); and

said heterocyclic groups have from 5 to 14 ring atoms of which from 1 to 4 are nitrogen, oxygen or sulphur hetero-atoms and are unsubstituted or have at least one of said substituents (a), (b) or (c);

or a pharmaceutically acceptable salt thereof, which process comprises the steps:

(i) reacting a compound of formula (II):

$$R^1-(O)_m-CH(R^2)-CO-(Y)_r-OH \quad (II)$$

[in which: $R^1$, <u>m</u> and $R^2$ are as defined above; Y represents a group of formula $-NH-CH(R^3)CO-$, where

$R^3$ is as defined above; and r is 0 or 1] or a reactive derivative thereof with a compound of formula (III):

$$H(Y)_sNH-CH(CH_2R^4)-CH(OH)-CH_2COR^5 \quad (III)$$

(in which: Y is as defined above, and $R^4$ and $R^5$ are as defined above; and s is 1 if r is 0 or 0 if r is 1) or a reactive derivative thereof;

(ii) if necessary, converting any group comprised within the above definitions to any other such group; and

(iii) optionally salifying the compound.

2. A process as claimed in Claim 1, wherein:

m is 0;

$R^1$ and $R^2$ are the same or different and each represents a $C_1$-$C_{10}$ alkyl groups, groups of formula $-(A)_p-R^6$, wherein:

p is 0 or 1;

A represents a $C_1$-$C_4$ alkylene group or a $C_2$-$C_4$ alkenylene group; and

$R^6$ represents an aryl group or an aromatic heterocyclic group;

or a group of formula $-B-R^7$, wherein:

B represents a $C_1$-$C_4$ alkylene group or an alkoxyalkyl group where the alkoxy and alkyl parts are both $C_1$ or $C_2$; and

$R^7$ represents a $C_1$-$C_4$ alkoxy group or an aralkyloxy group where the alkyl part is $C_1$-$C_4$;

$R^3$ represents a $C_1$-$C_{10}$ alkyl group, a $C_1$-$C_{10}$ alkyl group having at least one of the substituents (a'):

(a') hydroxy groups, $C_1$-$C_4$ alkoxy groups, $C_1$-$C_4$ alkylthio groups, $C_1$-$C_7$ aliphatic carboxylic acylamino groups, $C_2$-$C_5$ alkoxycarbonylamino groups, benzyloxycarbonylamino groups, $C_2$-$C_5$ alkoxycarbonyl groups, carbamoyl groups, $C_3$-$C_8$ cycloalkyl groups, phenyl groups, phenyl groups having at least one of the substituents (a'), (b') and (c), aromatic heterocyclic groups and carboxy groups;

a $C_3$ or $C_4$ alkenyl group or a propargyl group;

$R^4$ represents an isopropyl group, a $C_5$ or $C_6$ cycloalkyl group or a phenyl group;

$R^5$ represents a hydroxy group, a group of formula $-NR^8R^9$ wherein

$R^8$ and $R^9$ are the same or different and each represents a hydrogen atom, a $C_1$-$C_{10}$ alkyl group, a $C_1$-$C_{10}$ alkyl group having at least one of the substituents (b'):

(b') hydroxy groups, $C_1$-$C_4$ alkoxy groups, pyridyl groups, dialkylamino groups where each alkyl part is $C_1$-$C_4$, di($C_1$-$C_4$ hydroxyalkyl)amino groups and non-aromatic heterocyclic groups;

a $C_3$ or $C_4$ alkenyl group or a heterocyclic group;

or a heterocyclic group;

said aryl groups and the aryl parts of said aralkyloxy groups are $C_6$-$C_{10}$ carbocyclic aryl groups which are unsubstituted or have at least one of the substituents (a), (b) and (c) defined in Claim 1; and

said heterocyclic groups have from 5 to 10 ring atoms of

which from 1 to 3 are nitrogen, oxygen or sulphur hetero-atoms and are unsubstituted or have at least one of said substituents (a), (b) and (c).

3. A process as claimed in Claim 1, wherein:

**m** is 0;

$R^1$ and $R^2$ are the same or different and each represents a group of formula $-A-R^6$ in which:

A represents a $C_1$-$C_4$ alkylene group; and
$R^6$ represents an aryl group or an aromatic heterocyclic group;

or of formula $-B-R^7$ in which:

B represents a $C_1$-$C_4$ alkylene group or an alkoxyalkyl group in which each of the alkoxy part and the alkyl part is $C_1$ or $C_2$; and
$R^7$ represents a $C_1$-$C_4$ alkoxy group or a benzyloxy group;

$R^3$ represents a $C_1$-$C_{10}$ alkyl group, a $C_1$-$C_{10}$ alkyl group having at least one of the substituents (a"):

(a") hydroxy groups, $C_1$-$C_4$ alkylthio groups, carbamoyl groups, phenyl groups, aromatic heterocyclic groups, $C_2$-$C_5$ alkoxycarbonylamino groups, carboxy groups and cyclopentyl groups;

a $C_3$ or $C_4$ alkenyl group or a propargyl group;

$R^4$ represents an isopropyl group, a $C_5$ or $C_6$ cycloalkyl group or a phenyl group; and

$R^5$ represents a group of formula $-NR^{8'}R^{9'}$, in which $R^{8'}$ and $R^{9'}$ are the same or different and each represents a hydrogen atom, a 1-benzyl-4-piperidyl group, a $C_1$-$C_{10}$ alkyl group or a $C_1$-$C_{10}$ alkyl group having at least one of the substituents (b"):

(b") hydroxy groups, $C_1$-$C_4$ alkoxy groups, di($C_1$-$C_4$ hydroxyalkyl)amino groups and non-aromatic heterocyclic groups.

4. A process as claimed in Claim 1, wherein:

<u>m</u> is 0;

$R^2$ represents a 1-naphthylmethyl group; and

$R^4$ represents an isopropyl group.

5. A process as claimed in Claim 4, wherein:

$R^1$ represents a $C_1$-$C_4$ alkyl group having a single ω-substituent selected from phenyl, 3-pyridyl, 1-naphthyl, 2-methoxyethoxy and 2-benzyloxyethoxy groups;

$R^3$ represents an allyl group, a 2-propynyl group, a $C_1$-$C_{10}$ alkyl group or a $C_1$-$C_4$ alkyl group having a single ω-substituent selected from 5-imidazolyl, methylthio, t-butoxycarbonylamino, carbamoyl, carboxy, 1,3-thiazol-4-yl, phenyl and cyclopentyl substituents; and

$R^5$ represents an amino group, a (1-benzyl-4-piperidyl)amino group or a $C_2$-$C_6$ alkylamino group having one or two substituents selected from hydroxy, $C_1$ or $C_2$ alkoxy, di($C_1$ or $C_2$ alkyl)amino and morpholino substituents.

6. A process as claimed in Claim 1, wherein:

**m** is 0;

$R^1$ and $R^2$ both represent 1-naphthylmethyl groups; and

$R^4$ represents an isopropyl group.

7. A process as claimed in Claim 6, wherein:

$R^3$ represents an allyl group, a 2-propynyl group, a $C_1$-$C_5$ alkyl group or a $C_1$-$C_4$ alkyl group having a single ω-substituent selected from 5-imidazolyl, carbamoyl, 1,3-thiazol-4-yl and cyclopentyl substituents; and

$R^5$ represents a $C_2$-$C_6$ alkylamino group or a $C_2$-$C_6$ alkylamino group having a single substituent selected from hydroxy groups, di($C_1$ or $C_2$ alkyl)amino and morpholino groups.

8. A process as claimed in Claim 1, wherein the reaction conditions and reagents are so chosen as to prepare:

N-[4(S)-{N-[Bis(1-naphthylmethyl)acetyl]-L-leucylamino}-3(S)-hydroxy-6-methylheptanoyl]-L-isoleucinol;

4(S)-{N-[Bis(1-naphthylmethyl)acetyl]-L-leucylamino}-3(S)-hydroxy-6-methyl-N-(2-methylbutyl)-heptanamide;

4(S)-{N-[Bis(1-naphthylmethyl)acetyl]-L-leucylamino}-3(S)-hydroxy-6-methyl-N-(2-diethylaminoethyl)-heptanamide;

4(S)-{N-[Bis(1-naphthylmethyl)acetyl]-L-leucylamino}-3(S)-hydroxy-6-methyl-N-(2-morpholinoethyl)-heptanamide;

4-[4(S)-{N-[Bis(1-naphthylmethyl)acetyl]-L-leucylamino}-3(S)-hydroxy-6-methylheptanoyl]amino-1-benzylpiperidine;

4(S)-{N-[Bis(1-naphthylmethyl)acetyl]-L-histidylamino}-3(S)-hydroxy-6-methylheptanamide;

N-[4(S)-{N-[Bis(1-naphthylmethyl)acetyl]-L-histidylamino}-3(S)-hydroxy-6-methylheptanoyl]-L-isoleucinol;

4(S)-{N-[Bis(1-naphthylmethyl)acetyl]-L-histidylamino}-3(S)-hydroxy-6-methyl-N-[2(S)-(-)-methylbutyl]-heptanamide;

N-[4(S)-{N-[Bis(1-naphthylmethyl)acetyl]-L-isoleucylamino}-3(S)-hydroxy-6-methylheptanoyl]-L-isoleucinol;

N-[4(S)-{N-[Bis(1-naphthylmethyl)acetyl]-L-norleucylamino}-3(S)-hydroxy-6-methylheptanoyl]-L-isoleucinol;

N-[4(S)-{N-[Bis(1-naphthylmethyl)acetyl]-L-valylamino}-3(S)-hydroxy-6-methylheptanoyl]-L-isoleucinol;

N-[4(S)-{N-[Bis(1-naphthylmethyl)acetyl]-L-glutaminylamino}-3(S)-hydroxy-6-methylheptanoyl]-L-isoleucinol;

N-[4(S)-{N-[Bis(1-naphthylmethyl)acetyl]-3-(4-thiazolyl)-DL-alanylamino}-3(S)-hydroxy-6-methylheptanoyl]-L-isoleucinol;

N-[4(S)-{N-[Bis(1-naphthylmethyl)acetyl]-3-(cyclopentyl)-DL-alanylamino}-3(S)-hydroxy-6-methylheptanoyl]-L-isoleucinol;

N-[4(S)-{2(RS)-[Bis(1-naphthylmethyl)acetyl]-aminoheptanoyl}amino-3(S)-hydroxy-6-methylheptanoyl]-L-isoleucinol;

4(S)-{2(RS)-[Bis(1-naphthylmethyl)acetyl]amino-heptanoyl}amino-3(S)-hydroxy-6-methyl-N-(2-morpholinoethyl)heptanamide;

N-[4(S)-{N-[Bis(1-naphthylmethyl)acetyl]-DL-allylglycylamino}-3(S)-hydroxy-6-methylheptanoyl]-L-isoleucinol;

N-[4(S)-{N-[Bis(1-naphthylmethyl)acetyl]-DL-propargylglycylamino}-3(S)-hydroxy-6-methylheptanoyl]-L-isoleucinol;

or a pharmaceutically acceptable salt thereof.

9. The use for the manufacture of a medicament for the treatment of hypertension of a compound of formula (I) or salt thereof as defined in any one of Claims 1 to 9.